# FASCICULE DE BREVET EUROPEEN

(11) **EP 0 772 615 B1**
(45) Date de publication et mention de la délivrance du brevet: **22.09.1999**
(21) Numéro de dépôt: 95925891.4
(22) Date de dépôt: 17.07.1995
(51) Int. Cl.: C07D 487/04, C07D 487/14, A61K 31/50

(54) **DERIVES D'ACIDE IMIDAZO[1,2-a]INDENO[1,2-e]PYRAZINE-2-CARBOXYLIQUE, LEUR PREPARATION ET LES MEDICAMENTS LES CONTENANT**
IMIDAZO[1,2-a]INDENO[1,2-e]PYRAZIN-2-CARBONSÄURE DERIVATE, IHRE HERSTELLUNG UND SIE ENTHALTENDE ARZNEIMITTEL
IMIDAZO[1,2-a]INDENO[1,2-e]PYRAZINE-2-CARBOXYLIC ACID DERIVATIVES, PREPARATION THEREOF AND DRUGS CONTAINING SAME

(30) Priorité: 20.07.1994 FR 9408997
(43) Date de publication de la demande: 14.05.1997
(73) Titulaire: RHONE-POULENC RORER S.A., 92160 Antony (FR)
(72) Inventeur: ALOUP, Jean-Claude, F-94290 Villeneuve-le-Roi (FR); AUDIAU, François, F-94220 Charenton-le-Pont (FR); BARREAU, Michel, F-92230 Montgeron (FR); DAMOUR, Dominique, F-75014 Paris (FR); GENEVOIS-BORELLA, Arielle, F-94320 Thiais (FR); JIMONET, Patrick, F-78450 Villepreux (FR); MIGNANI, Serge, F-92290 Chatenay-Malabry (FR); RIBEILL, Yves, F-91360 Villemoisson-sur-Orge (FR)
(74) Mandataire: Savina, Jacques
(86) Numéro de dépôt international: FR9500951
(87) Numéro de publication internationale: WO9602544

(56) Documents cités:
- EP-A- 0 013 914
- WO-A-91/07408
- WO-A-93/06103
- WO-A-93/20077
- WO-A-94/05665
- WO-A-94/07893
- WO-A-94/22865
- FR-A- 2 448 541

## Description

La présente invention concerne des composés de formule : 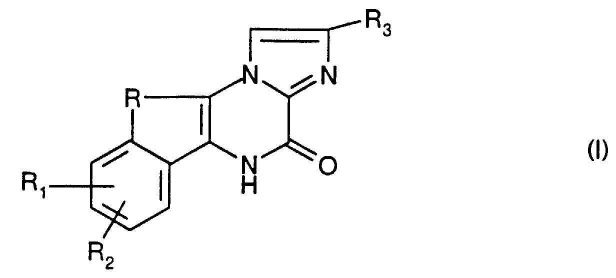 leurs sels, leur préparation et les médicaments les contenant.

WO-A-94/07893 divulgue des dérivés de 5H,10H-imidazo[1,2-a] indeno[1,2-e]pyrazine-4-one avec des affinités pour les récepteurs AMPA et NMDA.

Dans la formule (I),
- R représente un radical N-alk, C(R₄)R₅, CH-R₆ ou C=R₇,
- R₁ et R₂, identiques ou différents, représentent des atomes d'hydrogène ou d'halogène ou des radicaux alkyle, alcoxy, amino, -N=CH-N(alk)alk', nitro, cyano, phényle, imidazolyle, SO₃H, hydroxy, polyfluoroalcoxy, carboxy, alcoxycarbonyle, -NH-CO-NR₁₁R₁₂, -N(alk)-CO-NR₁₁R₁₂, -N(alk-Ar)-CO-NR₁₁R₁₂, -NH-CS-NR₁₁R₁₂, -N(alk)-CS-NR₁₁R₁₂, -NH-CO-R₁₁, -NH-CS-R₂₄, -NH-C(=NR₂₇)-NR₁₀R₁₂, -N(alk)-C(=NR₂₇)-NR₁₀R₁₂, -CO-NR₁₀R₁₂, -NH-SO₂-NR₁₀R₁₂, -N(alk)-SO₂-NR₁₀R₁₂, -NH-SO₂-CF₃, -NH-SO₂-alk, -NR₁₀R₁₃, -S(O)ₘ-alk-Ar, -SO₂-NR₁₀R₁₂, 2-oxo-1-imidazolidinyle dont la position -3 est éventuellement substituée par un radical alkyle ou 2-oxo-1-perhydropyrimidinyle dont la position -3 est éventuellement substituée par un radical alkyle,
- R₃ représente un radical carboxy, alcoxycarbonyle ou carboxamido,
- R₄ représente un radical alkyle, -alk-Het ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀,
- R₅ représente un radical alkyle (1-11C en chaîne droite ou ramifiée), -alk-Het, -NR₈R₉, -NH-CHO, -NH-COOR₁₇, -NH-SO₂R₂₄, -COOR₁₀, -alk-COOR₁₀, -alk-CONR₁₀R₁₈, -alk-NR₁₀R₁₈, -alk-OH, -alk-CN, phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, -NH-CO-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, -NH-CO-Het, -NH-CO-alk-Het, -NH-CO-alk-COOR₁₀, -NH-CO-alk-NR₁₀R₁₈, -NH-CO-alk-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, pyrrol-1-yle éventuellement substitué par un radical -COOR₁₀, -NH-CO-NH-alk-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, -NH-CO-NH-Het, -NH-CO-NH-alk-Het, -NH-CO-NH-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, -NH-COalk, -NH-COcycloalkyle, -NH-CO-NH-alk ou -NH-CO-NH₂,
ou bien R₄ et R₅ forment ensemble avec l'atome de carbone auquel ils sont rattachés un radical cycloalkyle,
- R₆ représente un atome d'hydrogène ou un radical hydroxy, alkyle (1-11C en chaîne droite ou ramifiée), -alk-OH, -NR₁₄R₁₅, -alk-NR₁₄R₁₅, -alk-Het, -NH-CHO, -COOalk, -alk-COOR₁₀, -alk-CO-NR₁₀R₂₁, phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, -R₁₆-COOR₁₀, -CO-COOR₁₀, pyrrol-1-yle éventuellement substitué par un radical -COOR₁₀ ou 2-oxo-2,5-dihydropyrrol-1-yle,
- R₇ représente un atome d'oxygène ou un radical NOH, NO-alk-COOR₁₀, NO-alk, CHR₁₉, NR₁₀, C(COOR₁₀)R₂₀ ou C(CONR₁₀R₂₁)R₂₀,
- R₈ représente un atome d'hydrogène ou un radical alkyle, -alk-COOR₁₀, -alk-NR₁₀R₂₁, -alk-Het ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀,
- R₉ représente un atome d'hydrogène ou un radical alkyle,
- R₁₀ représente un atome d'hydrogène ou un radical alkyle,
- R₁₁ représente un atome d'hydrogène ou un radical alkyle (1-9C en chaîne droite ou ramifiée), -alk-COOR₁₀, -alk-Het, -alk-NR₁₂R₁₀, phénylalkyle dont le noyau phényle est éventuellement substitué par un ou plusieurs substituants choisis parmi les atomes d'halogène et les radicaux alkyle, alcoxy, nitro, amino, hydroxy, -alk-NH₂, carboxy, alcoxycarbonyle, cyano et -alk-COOR₁₀, phényle éventuellement substitué par un ou plusieurs substituants choisis parmi les atomes d'halogène et les radicaux alkyle, alcoxy, nitro, amino, hydroxy, -alk-NH₂, carboxy, alcoxycarbonyle, cyano et -alk-COOR₁₀ ou -Het,
- R₁₂ représente un atome d'hydrogène ou un radical alkyle,
- R₁₃ représente un radical alkyle, Het ou alcoxycarbonyle,
- R₁₄ et R₁₅, identiques ou différents représentent chacun un radical alkyle ou bien R₁₄ représente un atome d'hydrogène et R₁₅ représente un atome d'hydrogène ou un radical alkyle, -COR₂₂, -CSR₂₃ ou -SO₂R₂₄,
- R₁₆ représente une chaîne -CHOH- ou -CH(OH)-alk(1-5C)-,
- R₁₇ représente un radical alkyle ou phénylalkyle,
- R₁₈ représente un atome d'hydrogène ou un radical alkyle,
- R₁₉ représente un radical hydroxy, alkyle, -alk-Het, -NR₂₅R₂₆, -alk-COOR₁₀, -Het, phényle éventuellement substitué par un ou plusieurs substituants choisis parmi les atomes d'halogène et les radicaux alkyle, alcoxy, nitro, amino, hydroxy, -alk-NH₂, -COOR₁₀, cyano et -alk-COOR₁₀ ou phénylalkyle dont le noyau phényle est éventuellement substitué par un ou plusieurs substituants choisis parmi les atomes d'halogène et les radicaux alkyle, alcoxy, nitro, amino, hydroxy, -alk-NH₂, -COOR₁₀, cyano et -alk-COOR₁₀,
- R₂₀ représente un atome d'hydrogène ou un radical alkyle,
- R₂₁ représente un atome d'hydrogène ou un radical alkyle,
- R₂₂ représente un radical alkyle, cycloalkyle, -COOalk, -alk-COOR₁₀, phényle éventuellement substitué par un ou plusieurs substituants choisis parmi les atomes d'halogène et les radicaux alkyle, alcoxy, nitro, amino, hydroxy, -alk-NH₂, -COOR₁₀, cyano et -alk-COOR₁₀, phénylalkyle dont le noyau phényle est éventuellement substitué par un ou plusieurs substituants choisis parmi les atomes d'halogène et les radicaux alkyle, alcoxy, nitro, amino, hydroxy, -alk-NH₂, -COOR₁₀, cyano et -alk-COOR₁₀, -alk-NR₁₀R₁₂, -NH-Ar dont Ar est éventuellement substitué par un ou plusieurs substituants choisis parmi les atomes d'halogène et les radicaux alkyle, alcoxy, nitro, amino, hydroxy, -alk-NH₂, -COOR₁₀, cyano et -alk-COOR₁₀, -Het, -alk-Het, -OR₁₇, -NH-alk-Ar dont Ar est éventuellement substitué par un ou plusieurs substituants choisis parmi les atomes d'halogène et les radicaux alkyle, alcoxy, nitro, amino, hydroxy, -alk-NH₂, -COOR₁₀, cyano et -alk-COOR₁₀, -NH-alk-Het, -NH-alk, -NH₂ ou -NH-Het,
- R₂₃ représente un radical -NH-alk, -NH-Ar, -NH-Het ou -NH₂,
- R₂₄ représente un radical alkyle ou phényle,
- R₂₅ et R₂₆, identiques ou différents, représentent chacun un radical alkyle ou cycloalkyle,
- R₂₇ représente un atome d'hydrogène ou un radical alkyle,
- alk représente un radical alkyle ou alkylène,
- alk' représente un radical alkyle,
- m est égal à 0, 1 ou 2,
- Ar représente un radical phényle,
- Het représente un hétérocycle mono ou polycyclique saturé ou insaturé contenant 1 à 9 atomes de carbone et un ou plusieurs hétéroatomes (O, S, N) éventuellement substitué par un ou plusieurs radicaux alkyle, phényle ou phénylalkyle.

Sauf mention contraire, dans les définitions qui précédent et celles qui suivent, les radicaux et portions alkyle, alkylène et alcoxy contiennent 1 à 6 atomes de carbone et sont en chaîne droite ou ramifiée, les radicaux et portions acyle contiennent 2 à 4 atomes de carbone, les radicaux cycloalkyle contiennent 3 à 6 atomes de carbone et les atomes d'halogène sont choisis parmi le fluor, le chlore, le brome et l'iode.

De préférence, Het est choisi parmi les cycles pyrrolyle éventuellement substitué par un ou plusieurs radicaux alkyle, phényle ou phénylalkyle, pyridyle éventuellement substitué par un ou plusieurs radicaux alkyle, phényle ou phénylalkyle, pyrimidinyle éventuellement substitué par un ou plusieurs radicaux alkyle, phényle ou phénylalkyle, imidazolyle éventuellement substitué par un ou plusieurs radicaux alkyle, phényle ou phénylalkyle, thiazolyle éventuellement substitué par un ou plusieurs radicaux alkyle, phényle ou phénylalkyle, oxazolinyle éventuellement substitué par un ou plusieurs radicaux alkyle, phényle ou phénylalkyle, thiazolinyle éventuellement substitué par un ou plusieurs radicaux alkyle, phényle ou phénylalkyle, pyrazinyle éventuellement substitué par un ou plusieurs radicaux alkyle, phényle ou phénylalkyle, tétrazolyle éventuellement substitué par un ou plusieurs radicaux alkyle, phényle ou phénylalkyle ou triazolyle éventuellement substitué par un ou plusieurs radicaux alkyle, phényle ou phénylalkyle. Les substituants préférés sont les radicaux méthyle, phényle et benzyle.

Les composés de formule (I) pour lesquels R₇ représente un radical NO-alk, C(COOR₁₀)R₂₀, C(CONR₁₀R₂₁)R₂₀ ou CHR₁₉ présentent des formes isomères (E et Z). Ces isomères et leurs mélanges font partie de l'invention.

Les composés de formule (I) pour lesquels R représente un radical CH-R₆ et R₆ représente un radical -CO-COOR₁₀ présentent des formes tautomères (E et Z). Ces formes tautomères font également partie de l'invention.

Les énantiomères et diastéréoisomères des composés de formule (I) pour lesquels R représente un radical C(R₄)R₅ ou CH-R₆ font également partie de l'invention.

Les composés de formule (I) pour lesquels R représente un radical N-alk ou CH-R₆, R₆ représente un atome d'hydrogène et R₃ représente un radical alcoxycarbonyle à l'exception de tert-butoxycarbonyle peuvent être préparés par cyclisation en présence d'acétate d'ammonium d'un dérivé de formule : 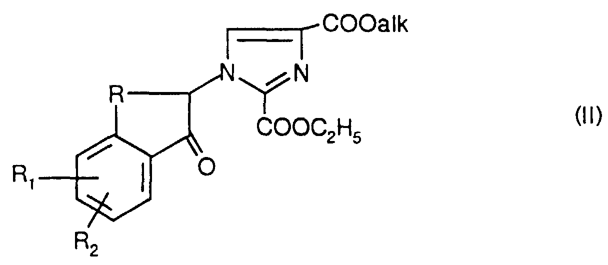 dans laquelle R représente un radical N-alk ou CH-R₆, R₆ représente un atome d'hydrogène, R₁ et R₂ ont les mêmes significations que dans la formule (I) et -COOalk représente un radical alcoxycarbonyle sauf tert-butoxycarbonyle.

Cette cyclisation s'effectue généralement au sein de l'acide acétique, à la température d'ébullition du milieu réactionnel.

Les dérivés de formule (Il) peuvent être obtenus par action d'un 4-alcoxycarbonyle-imidazole-2-carboxylate d'éthyle sur un dérivé de formule : 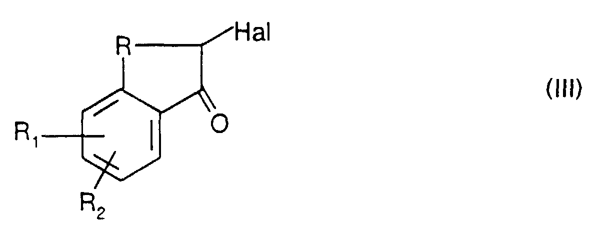 dans laquelle R représente un radical N-alk ou CH-R₆, R₆ représente un atome d'hydrogène, R₁ et R₂ ont les mêmes significations que dans la formule (I) et Hal représente un atome d'halogène.

Cette réaction s'effectue généralement au sein d'un solvant inerte tel qu'un alcool aliphatique inférieur (méthanol, éthanol par exemple), une cétone (acétone, méthyléthylcétone par exemple), un hydrocarbure aromatique (toluène par exemple), le diméthylformamide ou en absence de solvant, éventuellement en présence d'une base telle que l'hydrure de sodium ou le carbonate de potassium, à une température comprise entre 20°C et la température d'ébullition du milieu réactionnel ou la fusion du milieu réactionnel.

Les 4-alcoxycarbonyle-imidazole-2-carboxylate d'éthyle peuvent être obtenus par application ou adaptation de la méthode décrite par P.S. BRANCO et coll., Tetrahedron, 48 (30), 6335 (1992).

Les dérivés de formule (III) pour lesquels R représente un radical CH-R₆ et R₆ représente un atome d'hydrogène peuvent être obtenus par application ou adaptation de la méthode décrite par M. OLIVIER et coll., Bull. Soc. Chim. France, 3092 (1973).

Les dérivés de formule (III) pour lesquels R représente un radical N-alk peuvent être obtenus par alkylation d'un dérivé de formule (III) correspondant pour lequel R représente un radical NH, ce dernier étant obtenu par hydrolyse du dérivé acétylé correspondant préparé selon la méthode décrite par V.S. VELEZHEVA et coll., Khim. Farm. Zh., 24 (12), 46 (1990). L'alkylation s'effectue généralement au moyen d'un halogénure d'alkyle, en présence d'une base organique telle que la triéthylamine, un hydroxyde de métal alcalin (soude, potasse par exemple), éventuellement en présence de bromure de tétrabutylammonium, au sein d'un solvant inerte tel que le diméthylsulfoxyde, le diméthylformamide ou la pyridine, à une température comprise entre 20 et 50°C. L'hydrolyse s'effectue au sein d'un solvant inerte tel que le diméthylformamide, l'eau ou un mélange de ces solvants, à une température comprise entre 5°C et la température d'ébullition du milieu réactionnel.

Les composés de formule (I) pour lesquels R représente un radical N-alk ou CH-R₆, R₆ représente un atome d'hydrogène et R₃ représente un radical tert-butoxycarbonyle peuvent être préparés par action d'isobutène sur un composé de formule (I) correspondant pour lequel R représente un radical N-alk ou CH-R₆, R₆ représente un atome d'hydrogène et R₃ représente un radical carboxy.

Cette réaction s'effectue généralement au sein d'un solvant inerte tel que le dichlorométhane, le dioxanne, en présence d'acide sulfurique concentré, à une température comprise entre 20°C et la température d'ébullition du milieu réactionnel.

Les composés de formule (I) pour lesquels R représente un radical N-alk ou CH-R₆, R₆ représente un atome d'hydrogène et R₃ représente un radical carboxy peuvent être préparés par cyclisation en présence d'acétate d'ammonium d'un dérivé de formule (Il) dans laquelle R représente un radical N-alk ou CH-R₆, R₆ représente un atome d'hydrogène et COOalk représente un radical tert-butoxycarbonyle.

Cette cyclisation s'effectue généralement au sein de l'acide acétique, à la température d'ébullition du milieu réactionnel.

Les composés de formule (I) pour lesquels R représente un radical N-alk ou CH-R₆, R₆ représente un atome d'hydrogène et R₃ représente un radical carboxamido peuvent être préparés par cyclisation d'un dérivé de formule : 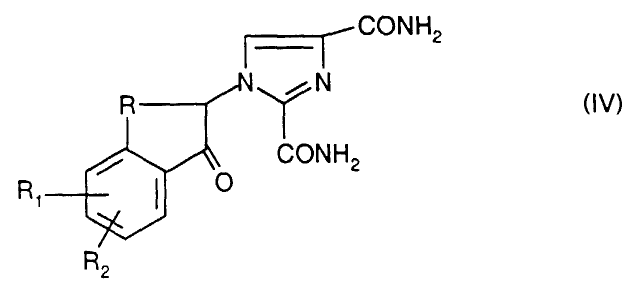 dans laquelle R représente un radical N-alk ou CH-R₆, R₆ représente un atome d'hydrogène, R₁ et R₂ ont les mêmes significations que dans la formule (I).

Cette cyclisation s'effectue généralement au moyen d'un acide tel que l'acide chlorhydrique ou l'acide acétique, en solution aqueuse, à une température comprise entre 20°C et la température d'ébullition du milieu réactionnel.

Les dérivés de formule (IV) peuvent être obtenus par action d'ammoniac sur un dérivé de formule (Il) correspondant.

Cette réaction s'effectue généralement au sein d'un solvant inerte tel qu'un alcool aliphatique inférieur, à une température comprise entre 20°C et la température d'ébullition du milieu réactionnel.

Les composés de formule (I) pour lesquels R représente un radical C=R₇ dans lequel R₇ représente un atome d'oxygène peuvent être préparés par hydrolyse d'un composé de formule (I) correspondant pour lequel R représente un radical C=R₇ et R₇ représente un radical NOH, suivie pour les composés pour lesquels R₃ représente un radical alcoxycarbonyle d'une estérification de l'acide correspondant.

Cette réaction s'effectue généralement au moyen d'un acide, en milieu aqueux, à la température d'ébullition du milieu réactionnel. Comme acide, on utilise de préférence l'acide chlorhydrique. L'estérification s'effectue au moyen d'un alcool aliphatique (C1-C6 en chaîne droite ou ramifiée), en milieu acide (acide chlorhydrique ou sulfurique par exemple), à la température d'ébullition du milieu réactionnel.

Les composés de formule (I) pour lesquels R représente un radical C=R₇ et R₇ représente un radical NOH peuvent être préparés par action d'un nitrite d'alkyle sur un composé de formule (I) correspondant pour lequel R représente un radical CH-R₆ et R₆ représente un atome d'hydrogène.

Cette réaction s'effectue de préférence au sein d'un solvant inerte tel que le diméthylsulfoxyde, en présence d'un hydrure de métal alcalin tel que l'hydrure de sodium, à une température voisine de 20°C. De préférence, on utilise le nitrite d'isoamyle.

Les composés de formule (I) pour lesquels R représente un radical C=R₇ et R₇ représente un radical NO-alk-COOR₁₀ ou NO-alk peuvent être préparés par action d'un composé de formule (I) correspondant pour lequel R représente un radical C=R₇ et R₇ représente un radical NOH sur un halogènure Hal-Ra pour lequel Hal représente un atome d'halogène et Ra représente un radical alkyle ou -alk-COOR₁₀, alk et R₁₀ ayant les mêmes significations que dans la formule (I).

Cette réaction s'effectue de préférence en présence d'une base telle qu'un hydrure de métal alcalin comme l'hydrure de sodium, au sein d'un solvant inerte tel que le diméthylsulfoxyde, à une température voisine de 20°C.

Les dérivés Hal-Ra pour lesquels Ra représente un radical -alk-COOR₁₀ sont commercialisés ou peuvent être obtenus par action de Hal-alk-Hal dans lequel Hal représente un atome d'halogène et alk représente un radical alkyle sur un cyanure alcalin (cyanure de sodium ou de potassium), au sein d'un mélange eau-alcool aliphatique inférieur, à une température comprise entre 0°C et la température d'ébullition du milieu réactionnel, suivie de l'action d'un acide fort tel que l'acide chlorhydrique, en présence d'un alcool aliphatique inférieur (méthanol, éthanol par exemple), à une température comprise entre 0°C et la température d'ébullition du milieu réactionnel.

Les composés de formule (I) pour lesquels R représente un radical C=R₇ et R₇ représente un radical CH-R₁₉ dans lequel R₁₉ représente un radical hydroxy peuvent être préparés par hydrolyse d'un composé de formule (I) correspondant pour lequel R₁₉ représente un radical -NR₂₅R₂₆, suivie pour les composés pour lesquels R₃ représente un radical alcoxycarbonyle d'une estérification de l'acide correspondant.

Cette réaction s'effectue de préférence au moyen d'un acide tel que l'acide chlorhydrique, en milieu aqueux, à une température comprise entre 20 et 40°C. L'estérification s'effectue au moyen d'un alcool aliphatique (C1-C6 en chaîne droite ou ramifiée), en milieu acide (acide chlorhydrique ou sulfurique par exemple), à la température d'ébullition du milieu réactionnel.

Les composés de formule (I) pour lesquels R représente un radical C=R₇ et R₇ représente un radical CH-R₁₉ dans lequel R₁₉ représente un radical -NR₂₅R₂₆ peuvent être préparés par action d'un composé de formule (I) correspondant pour lequel R représente un radical -CH-R₆ et R₆ représente un atome d'hydrogène sur un dérivé HC(Rb)(Rc)Rd dans laquelle soit Rb et Rd, identiques ou différents, représentent chacun un radical -NR₂₅R₂₆ dans lequel R₂₅ et R₂₆ ont les mêmes significations que dans la formule (I) et Rc représente un radical alcoxy tel que tert-butoxy, soit Rb, Rc et Rd, identiques, représentent chacun un radical -NR₂₅R₂₆ dans lequel R₂₅ et R₂₆ ont les mêmes significations que dans la formule (I).

Cette réaction s'effectue généralement au sein d'un solvant inerte tel que le diméthylformamide, à une température comprise entre 20 et 40°C.

Les dérivés HC(Rb)(Rc)Rd peuvent être obtenus par application ou adaptation de la méthode décrite par H. BREDERECK, Liebigs Ann. Chem., 762, 62 (1972).

Les composés de formule (I) pour lesquels R représente un radical C=R₇, R₇ représente un radical CHR₁₉ et R₁₉ représente un radical alkyle, phényle éventuellement substitué, -alk-Het, phénylalkyle dont le noyau phényle est éventuellement substitué, -Het ou -alk-COOR₁₀ peuvent être préparés par action d'un composé de formule (I) correspondant pour lequel R représente un radical CH-R₆ et R₆ représente un atome d'hydrogène sur un aldéhyde de formule OHC-Re dans laquelle Re représente un radical alkyle, phényle éventuellement substitué par un ou plusieurs substituants choisis parmi les atomes d'halogène et les radicaux alkyle, alcoxy, nitro, amino, hydroxy, -alk-NH₂, -COOR₁₀, cyano et -alk-COOR₁₀, -alk-Het, phénylalkyle dont le noyau phényle est éventuellement substitué par un ou plusieurs substituants choisis parmi les atomes d'halogène et les radicaux alkyle, alcoxy, nitro, amino, hydroxy, -alk-NH₂, -COOR₁₀, cyano et -alk-COOR₁₀, -Het ou -alk-COOR₁₀ dans lesquels alk, Het et R₁₀ ont les mêmes significations que dans la formule (I).

Cette réaction s'effectue généralement soit au sein d'un solvant inerte tel que le diméthylformamide, le 1,2-diméthoxyéthane, un alcool aliphatique inférieur (méthanol, éthanol par exemple) ou un mélange de ces solvants, en présence d'une base telle que la soude, la potasse, une base organique forte telle que le 1,8-diazabicyclo[5,4,0]undec-7-ène, à une température comprise entre 20 et 100°C, soit au sein du diméthylsulfoxyde, en présence d'un hydrure alcalin tel que l'hydrure de sodium, à une température voisine de 20°C, soit en présence de bromure de tétrabutylammonium et d'une base telle qu'un hydroxyde de métal alcalin (soude, potasse par exemple), au sein du diméthylsulfoxyde, à une température comprise entre 20°C et la température d'ébullition du milieu réactionnel.

Les dérivés OHC-Re sont commercialisés ou peuvent être obtenus (a) par oxydation des alcools correspondants (à l'aide de K₂Cr₂O₇, en milieu sulfurique; de CrO₃ au sein de la pyridine ou de MnO₂ au sein d'un solvant chloré (dichlorométhane par exemple), à une température voisine de 20°C ou à l'aide du diméthylsulfoxyde et de CICO-COCI par adaptation ou application de la méthode décrite par D. SWERN et coll., J. Org. Chem., 44, 4148 (1979)); (b) par réduction des acides carboxyliques correspondants (à l'aide de l'hydrure de lithium-aluminium ou de AlH₃, dans un solvant inerte tel que le tétrahydrofuranne, à une température comprise entre 0 et 25°C); (c) par réduction des esters correspondants (à l'aide d'hydrure de diisobutylaluminium, au sein d'un solvant inerte tel que le toluène, à une température comprise entre -70°C et 25°C ou d'hydrure de lithium-aluminium, au sein d'un solvant inerte tel que le tétrahydrofuranne, à une température comprise entre 0 et 25°C).

Les alcools correspondants HOH₂C-alk-Het ou HOH₂C-alk-Ar dont Ar est éventuellement substitué sont commercialisés ou peuvent être obtenus à partir des composés organométalliques correspondants par application ou adaptation des méthodes décrites par N.S. NARASIMHAN et coll., Tetrahedron Lett., 22 (29), 2797 (1981); L. ESTEL et coll., J. Het. Chem., 26, 105 (1989); N.S. NARASIMHAN et coll., Synthesis, 957 (1983); H.W. GSCHWEND et coll., Organic Reactions, 26, I (1979); V. SNIECKUS, Chem. Rev., 90, 879 (1990) et F. MARSAIS et coll., J. Heterocyclic Chem., 25, 81 (1988). De préférence, on fait réagir l'organolithien ou l'organomagnésien de l'hétérocycle ou du benzène éventuellement substitué sur le formol, l'oxyde d'éthylène ou un dérivé Hal-alk-CH₂OP où P est un groupe protecteur (méthyléther, tétrahydropyranyléther, benzyléther ou triéthylsilyléther par exemple), Hal représente un halogène et alk représente un alkyle puis on libère la fonction alcool par application ou adaptation des méthodes décrites par W. GREENE et coll., Protecting Groups in Organic Synthesis, second edition, 1991, John Wiley and Sons.

Les alcools correspondants HOH₂C-alk-Het ou HOH₂C-alk-Ar dont Ar est éventuellement substitué peuvent également être obtenus par réduction des acides carboxyliques ou des esters correspondants, au moyen d'hydrure de lithium-aluminium, au sein d'un solvant inerte tel que le tétrahydrofuranne ou le diéthyléther, à la température d'ébullition du milieu réactionnel.

Les alcools HOH₂C-alk-Het peuvent aussi être obtenus par application ou adaptation de la méthode décrite par J.Th. MEYER et coll., Helv. Chem. Acta, 65, 1868 (1982) à partir des dérivés Hal-alk(0-5C)-Het pour lesquels Hal représente un halogène, alk représente un alkyle et Het a les mêmes significations que dans la formule (I) qui sont eux-mêmes obtenus par action d'un agent d'halogénation (dérivé halogéné du phosphore ou chlorure de thionyle) sur un dérivé HOH₂C-alk(0-5C)-Het correspondant, éventuellement au sein d'un solvant inerte tel que le dichlorométhane, à une température comprise entre 20 et 40°C.

Les acides carboxyliques correspondants HOOC-Het, HOOC-alk-Het, HOOC-alk-Ar dont Ar est éventuellement substitué sont commercialisés ou peuvent être obtenus à partir des hétérocycles ou du benzène éventuellement substitué correspondants par application ou adaptation des méthodes décrites par L. ESTEL et coll., J. Heterocyclic Chem., 26, 105 (1989); N.S. NARASIMHAN et coll., Synthesis, 957 (1983); A. TURCK et coll., Synthesis, 881 (1988); A.J. CLARKE et coll., Tetrahedron Lett, 27, 2373 (1974); A.R. KATRITZKY et coll., Org. Perp. Procedure Int., 20 (6), 585 (1988); N. FURUKAWA et coll., Tetrahedron Lett., 28 (47), 5845 (1987); H.W. GSCHWEND et coll., Organic Reactions, 26, 1 (1979) et V. SNIECKUS, Chem. Rev., 90, 879 (1990). De préférence, on prépare le dérivé organométallique correspondant de l'hétérocycle ou du benzène éventuellement substitué correspondant (organolithien, organomagnésien par exemple) et le fait réagir soit sur du CO₂ soit sur un dérivé Hal-alk-COOalk dans lequel Hal représente un atome d'halogène et alk un radical alkyle. Les esters correspondants sont commercialisés ou peuvent être obtenus à partir des acides par action d'un acide tel que l'acide chlorhydrique ou l'acide sulfurique, au sein de l'alcool servant également d'agent d'estérification, à la température d'ébullition du milieu réactionnel. Les dérivés Hal-alk-COOalk sont commercialisés ou préparés par action de Hal-alk-Hal dans lequel Hal représente un atome d'halogène et alk est un alkyle sur un cyanure alcalin tel que le cyanure de sodium ou de potassium au sein d'un mélange eau-alcool aliphatique inférieur, à une température comprise entre 0°C et la température d'ébullition du milieu réactionnel, suivie de l'action d'un acide tel que l'acide chlorhydrique, en présence d'un alcool aliphatique inférieur, à une température comprise entre 0°C et la température d'ébullition du milieu réactionnel.

Les dérivés HOC-Re pour lesquels Re représente un radical -alk-COOR₁₀ sont commercialisés ou peuvent être obtenus par réduction des acides carboxyliques correspondants par application ou adaptation des méthodes décrites par H.C. BROWN et coll., J. Am. Chem. Soc., 106, 8001 (1984) et J. Org. Chem., 52, 5400 (1987). Les acides correspondants sont commercialisés ou peuvent être obtenus par application ou adaptation des méthodes décrites par H. HUNSDIECKER et coll., Chem. Ber., 75, 256 (1942) et R.F. NAYLOR, J. Chem. Soc., 1108 (1947).

Les composés de formule (I) pour lesquels R représente un radical C=R₇, R₇ représente un radical NR₁₀ peuvent être préparés par action de trifluoroacétate d'éthyle sur un dérivé de formule : 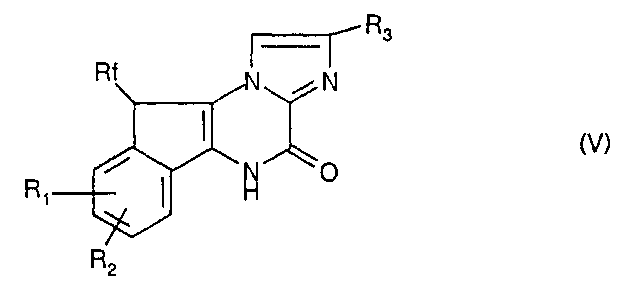 dans laquelle R₁, R₂ et R₃ ont les mêmes significations que dans la formule (I) et Rf représente un radical -NH₂ ou -NH-alk, alk ayant les mêmes significations que dans la formule (I).

Cette réaction s'effectue généralement au sein d'un solvant inerte tel que le diméthylformamide, à une température voisine de 60°C.

Les dérivés de formule (V) pour lesquels Rf représente un radical -NH₂ ou -NH-alk peuvent être obtenus par analogie avec les procédés décrits ci-après pour la préparation des composés de formule (I) pour lesquels R représente un radical CH-R₆ et R₆ représente un radical -NR₁₄R₁₅.

Les composés de formule (I) pour lesquels R représente un radical C=R₇ et R₇ représente un radical C(COOR₁₀)R₂₀ peuvent être préparés par déshydratation d'un composé de formule (V) pour lequel R₁, R₂ et R₃ ont les mêmes significations que dans la formule (I) et Rf représente un radical -C(R₂₀)(OH)-COOR₁₀ dans lequel R₂₀ et R₁₀ ont les mêmes significations que dans la formule (I).

Cette réaction s'effectue généralement au sein de l'anhydride acétique, à la température d'ébullition du milieu réactionnel.

Les composés de formule (V) pour lesquels R₁, R₂ et R₃ ont les mêmes significations que dans la formule (I) et Rf représente un radical -C(R₂₀)(OH)-COOR₁₀ dans lequel R₂₀ et R₁₀ ont les mêmes significations que dans la formule (I) peuvent être obtenus par action d'un composé de formule (I) correspondant pour lequel R représente un radical CH-R₆ et R₆ représente un atome d'hydrogène sur un dérivé R₂₀-CO-COOR₁₀ pour lequel R₁₀ et R₂₀ ont les mêmes significations que dans la formule (I), suivie d'un traitement à l'acide acétique.

Cette réaction s'effectue au sein du diméthylsulfoxyde, en présence d'un hydrure de métal alcalin tel que l'hydrure de sodium, à une température voisine de 20°C ou en présence de bromure de tétrabutylammonium et d'une base telle qu'un hydroxyde de métal alcalin (soude, potasse par exemple), au sein du diméthylsulfoxyde, à une température comprise entre 20 et 100°C. Le traitement à l'acide acétique s'effectue à une température inférieure à 20°C.

Les dérivés de formule R₂₀-CO-COOR₁₀ dans laquelle R₁₀ et R₂₀ ont les mêmes significations que dans la formule (I) sont commercialisés ou peuvent être obtenus par application ou adaptation des méthodes décrites par L.A. CARPINO, J. Org. Chem., 29, 2820 (1964) et H.H. WASSERMAN, J. Org. Chem., 50, 3573 (1985).

Les composés de formule (I) pour lesquels R représente un radical C=R₇ et R₇ représente un radical C(CONR₁₀R₂₁)R₂₀ peuvent être préparés par action d'un composé de formule (I) correspondant pour lequel R représente un radical C=R₇ et R₇ représente un radical C(COOR₁₀)R₂₀ sur une amine HNR₁₀R₂₁ dans laquelle R₁₀ et R₂₁ ont les mêmes significations que dans la formule (I).

Lorsque l'on met en oeuvre l'acide, on opère en présence d'un agent de condensation utilisé en chimie peptidique tel qu'un carbodiimide (par exemple le N,N'-dicyclohexylcarbodiimide) ou le N,N'-diimidazole carbonyle, dans un solvant inerte tel qu'un éther (tétrahydrofuranne, dioxanne par exemple), un amide (diméthylformamide) ou un solvant chloré (chlorure de méthylène, dichloro-1,2 éthane, chloroforme par exemple) à une température comprise entre 0°C et la température de reflux du mélange réactionnel. Lorsque l'on met en oeuvre un ester, on opère alors soit en milieu organique, éventuellement en présence d'un accepteur d'acide tel qu'une base organique azotée (trialkylamine, pyridine, diaza-1,8 bicyclo [5.4.0] undécène-7 ou diaza-1,5 bicyclo [4.3.0] nonène-5 par exemple), dans un solvant tel que cité ci-dessus, ou un mélange de ces solvants, à une température comprise entre 0°C et la température de reflux du mélange réactionnel, soit en milieu hydroorganique biphasique en présence d'une base alcaline ou alcalino-terreuse (soude, potasse) ou d'un carbonate ou bicarbonate d'un métal alcalin ou alcalino-terreux à une température comprise entre 0 et 40°C.

Les composés de formule (I) pour lesquels R représente un radical C(R₄)R₅, R₄ représente un radical alkyle, -alk-Het ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀ et R₅ est identique à R₄ peuvent être préparés par action d'un composé de formule (I) correspondant pour lequel R représente un radical CH-R₆ et R₆ représente un atome d'hydrogène sur un halogénure de formule Hal-Rg dans laquelle Rg représente un radical alkyle, -alk-Het ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, alk, Het et R₁₀ ayant les mêmes significations que dans la formule (I).

Cette réaction s'effectue de préférence au sein d'un solvant inerte tel que le diméthylsulfoxyde, le diméthylformamide, le tétrahydrofuranne, le dioxanne, en présence d'une base tel qu'un hydroxyde de métal alcalin (soude, potasse par exemple), éventuellement en présence de bromure de tétrabutylammonium, soit au sein du diméthylsulfoxyde ou du diméthylformamide en présence d'un hydrure de métal alcalin (hydrure de sodium par exemple), à une température comprise entre 20°C et la température d'ébullition du milieu réactionnel.

Les dérivés Hal-Rg sont commercialisés ou peuvent être obtenus à partir des alcools correspondants par application ou adaptation des méthodes décrites par R.C. LAROCK, "Comprehensive Organic Transformations", Ed. VCH, page 353 (1989).

Les composés de formule (I) pour lesquels R représente un radical C(R₄)R₅, R₄ représente un radical alkyle, -alk-Het ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀ et R₅ représente un radical alkyle (1-11C en chaîne droite ou ramifiée), -alk-CN, -alk-Het, -alk-NR₁₀R₁₈, -alk-CO-NR₁₀R₁₈ ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, peuvent être préparés par action d'un composé de formule (I) correspondant pour lequel R représente un radical CH-R₆ et R₆ représente un radical alkyle, -alk-Het ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀ sur un halogénure Hal-Rh pour lequel Rh représente un radical alkyle (1-11C en chaîne droite ou ramifiée), -alk-Het, -alk-CN, -alk-NR₁₀R₁₈, -alk-CO-NR₁₀R₁₈ ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, alk, Het, R₁₀ et R₁₈ ayant les mêmes significations que dans la formule (I).

Cette réaction s'effectue de préférence au sein d'un solvant inerte tel que le diméthylsulfoxyde, le diméthylformamide, le tétrahydrofuranne, le dioxanne, en présence d'une base tel qu'un hydroxyde de métal alcalin (soude, potasse par exemple), éventuellement en présence de bromure de tétrabutylammonium, soit au sein du diméthylsulfoxyde ou du diméthyformamide en présence d'un hydrure de métal alcalin (hydrure de sodium par exemple), à une température comprise entre 20°C et la température d'ébullition du milieu réactionnel.

Les dérivés Hal-Rh sont commercialisés ou ceux pour lesquels Rh représente un radical -alk-CO-NR₁₀R₁₈ peuvent être préparés par action d'une amine HNR₁₀R₁₈ sur un dérivé Hal-alk-CO-Hal dans laquelle Hal représente un atome d'halogène et alk représente un radical alkyle, au sein d'un solvant inerte tel que le diméthylformamide, le tétrahydrofuranne, un solvant chloré, en présence d'une base organique telle qu'une trialkylamine ou la pyridine, à une température comprise entre 0°C et la température d'ébullition du milieu réactionnel. Les dérivés Hal-alk-CO-Hal sont commercialisés ou peuvent être obtenus par halogénation des acides carboxyliques correspondants au moyen d'un agent d'halogénation tel que le chlorure de thionyle, au sein d'un solvant inerte tel que le 1,2-dichloroéthane, à une température voisine de 60°C. Les acides Hal-alk-COOH sont commercialisés ou peuvent être obtenus par action d'un cyanure de métal alcalin sur un dérivé Hal-alk-Hal dans lequel alk représente un radical alkyle et Hal représente un atome d'halogène, au sein d'un mélange eau-alcool aliphatique inférieur, à une température comprise entre 0°C et la température d'ébullition du milieu réactionnel suivie de l'action d'un acide fort tel que l'acide chlorhydrique, en milieu aqueux, à une température comprise entre 0°C et la température d'ébullition du milieu réactionnel.

Les composés de formule (I) pour lesquels R représente un radical C(R₄)R₅ et R₄ et R₅ forment avec l'atome de carbone auquel ils sont rattachés un radical cycloalkyle peuvent être préparés par action d'un composé de formule (I) correspondant pour lequel R représente un radical CH-R₆ et R₆ représente un atome d'hydrogène sur un dérivé de formule Hal-alk-Hal dans laquelle Hal représente un atome d'halogène et alk représente un radical alkyle (2-5C).

Cette réaction s'effectue de préférence au sein d'un solvant inerte tel que le diméthylsulfoxyde, le diméthylformamide, le tétrahydrofuranne, le dioxanne, en présence d'une base tel qu'un hydroxyde de métal alcalin (soude, potasse par exemple), éventuellement en présence de bromure de tétrabutylammonium, soit au sein du diméthylsulfoxyde ou du diméthyformamide en présence d'un hydrure de métal alcalin (hydrure de sodium par exemple), à une température comprise entre 20°C et la température d'ébullition du milieu réactionnel.

Les dérivés Hal-alk-Hal sont commercialisés ou peuvent être obtenus à partir des dialcools correspondants par application ou adaptation des méthodes décrites par C. LAROCK, "Comprehensive Organic Transformations", Ed. VHC, page 353 (1989).

Les composés de formule (I) pour lesquels R représente un radical C(R₄)R₅ dans lequel R₅ représente un radical -NR₈R₉, R₈ et R₉ représentent des atomes d'hydrogène peuvent être préparés par action d'un halogènure Hal-R₄ dans lequel Hal représente un atome d'halogène et R₄ a les mêmes significations que dans la formule (I), sur un composé de formule (I) correspondant pour lequel R représente un radical CH-R₆, R₆ représente un radical -NR₁₄R₁₅, R₁₄ représente un atome d'hydrogène, R₁₅ représente un radical -COR₂₂ et R₂₂ représente un radical alkyle (1C), suivie d'une hydrolyse.

Cette réaction s'effectue généralement au sein d'un solvant inerte tel que le diméthylsulfoxyde, en présence d'un hydrure de métal alcalin tel que l'hydrure de sodium, à une température voisine de 20°C. L'hydrolyse s'effectue généralement au moyen d'un acide minéral tel que l'acide chlorhydrique, en milieu aqueux, à la température d'ébullition du milieu réactionnel.

Les composés de formule (I) pour lesquels R représente un radical C(R₄)R₅ dans lequel R₅ représente un radical -NR₈R₉, R₉ représente un atome d'hydrogène et R₈ représente un radical alkyle, -alk-COOR₁₀, -alk-NR₁₀R₂₁, -alk-Het ou phénylalkyle dont le noyau phényle est éventuellement substitué peuvent être préparés par action d'un composé de formule (I) correspondant pour lequel R représente un radical C(R₄)R₅, R₅ représente un radical -NR₈R₉, R₈ et R₉ représentent des atomes d'hydrogène sur un halogénure Hal-R₈ dans lequel R₈ a les mêmes significations que précédemment.

Cette réaction s'effectue généralement au sein d'un solvant inerte tel que le diméthylformamide, en présence d'un carbonate de métal alcalin tel que le carbonate de sodium ou de potassium ou une trialkylamine telle que la triéthylamine ou la pyridine, à une température comprise entre 0°C et la température d'ébullition du milieu réactionnel.

Les halogènures Hal-R₈ sont commercialisés ou ceux pour lesquels R₈ représente un radical -alk-NR₁₀R₂₁ peuvent être obtenus par action de l'amine HNR₁₀R₂₁ dans laquelle R₁₀ et R₂₁ ont les mêmes significations que dans la formule (I) sur un halogénure Hal-alk-Hal dans lequel Hal représente un atome d'halogène et alk représente un radical alkyle, au sein d'un solvant inerte tel que le diméthylformamide, en présence d'un accepteur d'acide tel qu'un base azotée, à une température comprise entre 0 et 25°C. Ceux pour lesquels R₈ représente un radical -alk-COOR₁₀ peuvent être obtenus par action d'un dérivé Hal-alk-Hal dans laquelle Hal représente un atome d'halogène et alk représente un radical alkyle sur un cyanure alcalin (cyanure de sodium ou de potassium), au sein d'un mélange eau-alcool aliphatique inférieur, à une température comprise entre 0°C et la température d'ébullition du milieu réactionnel, suivie de l'action d'un acide fort tel que HCI, en présence d'un alcool aliphatique inférieur, à une température comprise entre 0°C et la température d'ébullition du milieu réactionnel.

Les composés de formule (I) pour lesquels R représente un radical C(R₄)R₅ dans lequel R₅ représente un radical -NR₈R₉, R₉ représente un atome d'hydrogène ou un radical alkyle et R₈ représente un radical alkyle (2-6C) peuvent être préparés par action d'un composé de formule (I) correspondant pour lequel R représente un radical C(R₄)R₅, R₅ représente un radical -NR₈R₉, R₉ représente un atome d'hydrogène ou un radical alkyle et R₈ représente un atome d'hydrogène sur un halogènure d'acyle (2-6C) puis réduction du produit obtenu.

Cette réaction s'effectue généralement au sein d'un solvant inerte tel que le diméthylformamide, en présence d'un accepteur d'acide tel qu'une base organique azotée (trialkylamine telle que la triéthylamine ou la pyridine), à une température comprise entre 0°C et la température d'ébullition du milieu réactionnel. La réduction s'effectue au sein d'un solvant inerte tel que le tétrahydrofuranne, en présence de B₂H₆, à la température d'ébullition du milieu réactionnel.

Les composés de formule (I) pour lesquels R représente un radical C(R₄)R₅ dans lequel R₅ représente un radical -NR₈R₉ et R₉ représente un radical alkyle peuvent également être préparés par action d'un composé de formule (I) correspondant pour lequel R représente un radical C(R₄)R₅, R₅ représente un radical -NR₈R₉, R₈ a les mêmes significations que dans la formule (I) et R₉ représente un atome d'hydrogène sur un dérivé de formule Hal-alk dans laquelle Hal représente un atome d'halogène et alk représente un radical alkyle.

Cette réaction s'effectue au sein d'un solvant inerte tel que le diméthylformamide, en présence d'un accepteur d'acide tel qu'une base organique azotée (pyridine ou trialkylamine comme la triéthylamine), à une température comprise entre 0°C et la température d'ébullition du milieu réactionnel.

Les dérivés Hal-R₉ peuvent être obtenus par application ou adaptation des méthodes décrites par C. LAROCK, "Comprehensive Organic Transformations", Ed. VCH, pages 345 et 353 (1989).

Les composés de formule (I) pour lesquels R représente un radical C(R₄)R₅ dans lequel R₅ représente un radical -NR₈R₉, R₉ représente un atome d'hydrogène ou un radical alkyle et R₈ représente un radical -alk(2-6C)-NR₁₀R₂₁ peuvent être préparés par réduction d'un dérivé de formule : 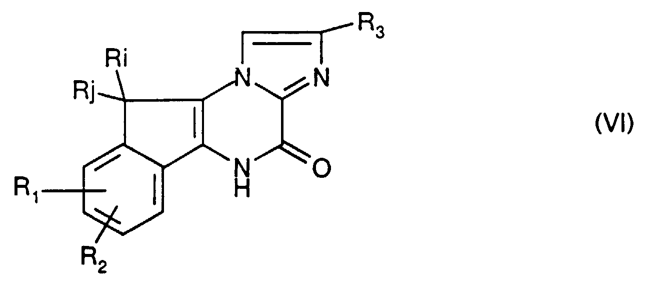 dans laquelle R₁, R₂ et R₃ ont les mêmes significations que dans la formule (I), Ri représente un radical alkyle, phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀ ou -alk-Het et Rj représente un radical -NH-CO-alk(1-5C)-NR₁₀R₂₁ ou -N(alk)-CO-alk(1-5C)-NR₁₀R₂₁ dans lesquels alk, Het, R₁₀ et R₂₁ ont les mêmes significations que dans la formule (I).

Cette réduction s'effectue au sein d'un solvant inerte tel que le tétrahydrofuranne, au moyen de B₂H₆, à la température d'ébullition du milieu réactionnel.

Les dérivés de formule (VI) dans laquelle Ri représente un radical alkyle, phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, ou -alk-Het et Rj représente un radical -NHCO-alk(1-5C)-NR₁₀R₂₁ ou -N(alk)-CO-alk(1-5C)-NR₁₀R₂₁ dans lesquels R₁₀ et R₂₁ sont des atomes d'hydrogène peuvent être obtenus par action d'un composé de formule (I) correspondant pour lequel R représente un radical C(R₄)R₅, R₅ représente un radical -NR₈R₉, R₈ représente un atome d'hydrogène et R₉ représente un atome d'hydrogène ou un radical alkyle sur un dérivé de formule : 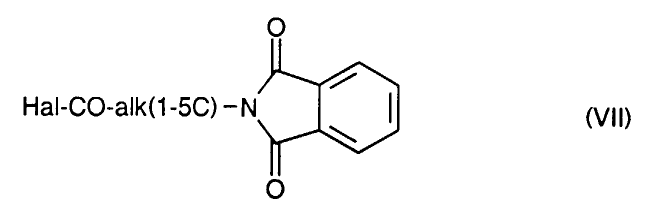 dans laquelle Hal représente un atome d'halogène et alk représente un radical alkyle puis déprotection de la fonction amino.

Cette réaction s'effectue au sein d'un solvant inerte tel que le diméthylformamide, en présence d'un accepteur d'acide tel qu'un base organique azotée (pyridine ou trialkylamine comme la triéthylamine), à une température comprise entre 0°C et la température d'ébullition du milieu réactionnel. La déprotection s'effectue au sein d'un alcool aliphatique inférieur (éthanol par exemple), en présence d'hydrazine, à la température d'ébullition du milieu réactionnel.

Les dérivés de formule (VII) peuvent être obtenus par application ou adaptation de la méthode décrite par K. BALENOVIC et coll., J. Org. Chem., 17, 1459 (1952).

Les dérivés de formule (VI) pour lesquels Ri représente un radical alkyle, phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀ ou -alk-Het et Rj représente un radical -NH-CO-alk(1-5C)-NR₁₀R₂₁ ou -N(alk)-CO-alk(1-5C)-NR₁₀R₂₁ dans lesquels R₁₀ et R₂₁ ont les mêmes significations que dans la formule (I) peuvent aussi être obtenus par action d'un composé de formule (I) correspondant pour lequel R représente un radical C(R₄)R₅, R₅ représente un radical -NR₈R₉, R₈ représente un atome d'hydrogène et R₉ représente un atome d'hydrogène ou un radical alkyle sur un dérivé de formule Hal-CO-alk(1-5C)-NR₁₀R₂₁ dans laquelle alk représente un radical alkyle, R₁₀ et R₂₁ ont les mêmes significations que dans la formule (I), Hal représente un atome d'halogène et, de préférence un atome de chlore ou de brome.

Cette réaction s'effectue au sein d'un solvant inerte tel que le diméthylformamide, en présence d'un accepteur d'acide tel qu'une base organique azotée (pyridine ou trialkylamine comme la triéthylamine), à une température comprise entre 0°C et la température d'ébullition du milieu réactionnel.

Les dérivés de formule Hal-CO-alk(1-5C)-NR₁₀R₂₁ peuvent être obtenus par chauffage de l'acide carboxylique correspondant et d'un réactif d'halogénation tel que le chlorure de thionyle, au sein d'un solvant inerte tel que le 1,2-dichloroéthane, à une température voisine de 60°C. Les acides correspondants sont commercialisés ou peuvent être obtenus par action des halogénures alkOOC-alk(1-5C)-Hal dans lesquels Hal représente un atome d'halogène et alk un radical alkyle, sur une amine HNR₁₀R₂₁ dans laquelle R₁₀ et R₂₁ ont les mêmes significations que dans la formule (I), en présence d'un carbonate alcalin (carbonate de sodium) ou une trialkyamine, à une température comprise entre 20°C et la température d'ébullition du milieu réactionnel, suivie d'une hydrolyse en milieu basique (soude-alcool aliphatique inférieur), à une température comprise entre 0 et 60°C et d'une acidification au moyen d'un acide tel que l'acide chlorhydrique, à une température comprise entre 20 et 60°C.

Les composés de formule (I) pour lesquels R représente un radical C(R₄)R₅ dans lequel R₅ représente un radical -COOR₁₀ et R₁₀ représente un atome d'hydrogène peuvent être préparés par hydrolyse d'un composé de formule (I) correspondant pour lequel R représente un radical C(R₄)R₅ dans lequel R₅ représente un radical -COOR₁₀ et R₁₀ représente un radical alkyle.

Cette hydrolyse s'effectue de préférence au moyen d'une base telle qu'un hydroxyde de métal alcalin (soude ou potasse par exemple), au sein d'un mélange eau-alcool aliphatique inférieur (éthanol par exemple), à une température d'environ 20 à 30°C.

Les composés de formule (I) pour lesquels R représente un radical C(R₄)R₅ dans lequel R₅ représente un radical -COOR₁₀ et R₁₀ représente un radical alkyle peuvent être préparés par action d'un composé de formule (I) correspondant pour lequel R représente un radical CH-R₆ et R₆ représente un radical alkyle, -alk-Het ou phénylalkyle éventuellement substitué sur un halogènure de formule Hal-COOR₁₀ dans lequel R₁₀ représente un radical alkyle et Hal représente un atome d'halogène.

Cette réaction s'effectue généralement au sein d'un solvant inerte tel que le dioxanne, en présence d'un hydrure de métal alcalin tel que l'hydrure de sodium ou de potassium, à une température voisine de 20°C.

Les dérivés Hal-COOR₁₀ sont commercialisés ou peuvent être obtenus par application ou adaptation des méthodes décries dans HOUBEN-WEYL, band 8, page 102 (1952).

Les composés de formule (I) pour lesquels R représente un radical C(R₄)R₅ dans lequel R₅ représente un radical -alk-COOR₁₀ peuvent être préparés par action d'un composé de formule (I) correspondant pour lequel R représente un radical CH-R₆, R₆ représente un radical -alk-COOR₁₀ et R₁₀ a les mêmes significations que dans la formule (I) sur un halogènure Hal-R₄ dans lequel R₄ a les mêmes significations que dans la formule (I).

Cette réaction s'effectue au sein d'un solvant inerte tel que le diméthylformamide, en présence d'un hydrure de métal alcalin tel que l'hydrure de sodium ou de potassium, à une température voisine de 20°C.

Les composés de formule (I) pour lesquels R représente un radical C(R₄)R₅ dans lequel R₅ représente un radical -alk-CN dans lequel alk contient 1 atome de carbone peuvent être préparés par action de cyanure de sodium sur un dérivé de formule (VI) pour lequel R₁, R₂ et R₃ ont les mêmes significations que dans la formule (I), Ri représente un radical -CH₂OTs dans lequel Ts représente un reste tosylate et Rj représente un radical alkyle, -alk-Het ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, alk, Het et R₁₀ ayant les mêmes significations que dans la formule (I).

Cette réaction s'effectue de préférence au sein du diméthylformamide, en présence d'iodure de sodium, à une température comprise entre 20 et 100°C.

Les dérivés de formule (VI) pour lequel R₁, R₂ et R₃ ont les mêmes significations que dans la formule (I), Ri représente un radical -CH₂OTs dans lequel Ts représente un reste tosylate et Rj représente un radical alkyle, -alk-Het ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀ peuvent être obtenus par action de chlorure de tosyle sur un composé de formule (I) correspondant pour lequel R₅ représente un radical -alk(1C)OH au sein de la pyridine, à une température comprise entre -10 et 20°C.

Les composés de formule (I) pour lesquels R représente un radical C(R₄)R₅ dans lequel R₅ représente un radical -alk(2-6C)OH peuvent être préparés par action de (COCl)₂ sur un composé de formule (I) correspondant pour lequel R représente un radical C(R₄)R₅ dans lequel R₅ représente un radical -alk-COOR₁₀ et R₁₀ représente un atome d'hydrogène suivie d'une réduction.

Cette réaction s'effectue au sein d'un solvant inerte tel que le dioxanne. La réduction s'effectue de préférence au moyen de borohydrure de sodium, au sein d'un solvant inerte tel que le diméthylformamide, à une température comprise entre 10 et 20°C.

Les composés de formule (I) pour lesquels R représente un radical C(R₄)R₅ dans lequel R₅ représente un radical -alk(1C)OH peuvent être préparés par action de chlorure de triméthylsilane sur un dérivé de formule (VI) pour lequel R₁, R₂ et R₃ ont les mêmes significations que dans la formule (I), Ri représente un atome d'hydrogène et Rj représente un radical alkyle, -alk-Het ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, alk, Het et R₁₀ ayant les mêmes significations que dans la formule (I), puis du trioxane.

Cette réaction s'effectue généralement au sein d'un solvant inerte tel que le diméthylformamide, en présence d'hydrure de sodium puis réaction avec le trioxane à une température comprise entre 0 et 25°C.

Les composés de formule (I) pour lesquels R représente un radical C(R₄)R₅ dans lequel R₅ représente un radical -NH-CHO peuvent être préparés par action d'un dérivé de formule (VI) dans laquelle R₁, R₂ et R₃ ont les mêmes significations que dans la formule (I), Ri représente un radical alkyle, -alk-Het ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, alk, Het et R₁₀ ont les mêmes significations que dans la formule (I) et Rj représente un radical amino sur CH₃COOCHO.

Cette réaction s'effectue de préférence au sein d'un solvant inerte tel que l'acide formique, en présence d'acétate de sodium, à une température voisine de 20°C.

Les composés de formule (I) pour lesquels R représente un radical C(R₄)R₅ dans lequel R₅ représente un radical -NH-COOR₁₇, -NH-CO-Het, -NH-CO-alk-COOR₁₀, -NH-CO-alk-NR₁₀R₁₈, -NH-CO-Ar dont Ar est éventuellement substitué, -NH-CO-alk-Ar dont Ar est éventuellement substitué, -NH-SO₂-R₂₄, -NH-CO-alk-Het, -NH-CO-alk ou -NH-CO-cycloalkyle peuvent être préparés par action d'un composé de formule (I) correspondant pour lequel R représente un radical C(R₄)R₅, R₅ représente un radical -NR₈R₉, R₈ et R₉ représentent des atomes d'hydrogène sur un dérivé Hal-Rk dans lequel Hal représente un atome d'halogène et Rk représente un radical -COOR₁₇, -CO-Het, -CO-alk-COOR₁₀, -CO-alk-NR₁₀R₁₈, -CO-alk-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, -SO₂-R₂₄, -CO-alk-Het, -CO-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, -CO-alk ou -CO-cycloalkyle, R₇, R₁₀, R₁₇, R₁₈, R₂₄, Het, Ar et alk ayant les mêmes significations que dans la formule (I).

Cette réaction s'effectue généralement au sein d'un solvant inerte tel que le diméthylformamide, le diméthylsulfoxyde, en présence d'un accepteur d'acide tel qu'une trialkylamine (triéthylamine par exemple) ou un hydrure de métal alcalin (hydrure de sodium par exemple), à une température voisine de 20°C.

Les dérivés Hal-Rk sont commercialisés ou ceux pour lesquels Rk représente un radical -CO-Het, -CO-alk-Het, -CO-cycloalkyle, -CO-alk-COOR₇, -CO-alk-NR₁₀R₁₈, -CO-alk-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀ peuvent être obtenus à partir des acides carboxyliques correspondants par action d'un halogénure de phosphore (PCl₅ ou POCl₃ par exemple), de préférence au sein de l'halogénure de phosphore, éventuellement en présence d'un solvant inerte tel que le dichlorométhane, à une température comprise entre 20°C et la température d'ébullition du milieu réactionnel.

Les composés de formule (I) pour lesquels R représente un radical C(R₄)R₅ dans lequel R₅ représente un radical -NH-CO-Het, -NH-CO-alk-COOR₁₀, -NH-CO-alk-NR₁₀R₁₈, -NH-CO-Ar dont Ar est éventuellement substitué, -NH-CO-alk-Ar dont Ar est éventuellement substitué, -NH-CO-alk-Het, -NH-CO-alk ou -NH-CO-cycloalkyle peuvent également être préparés par action d'un composé de formule (I) correspondant pour lequel R représente un radical C(R₄)R₅, R₅ représente un radical -NR₈R₉, R₈ et R₉ représentent des atomes d'hydrogène sur un dérivé HO-RI dans lequel RI représente un radical -CO-Het, -CO-alk-COOR₁₀, -CO-alk-NR₁₀R₁₈, -CO-alk-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, -CO-alk-Het, -CO-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, -CO-alk ou -CO-cycloalkyle, R₁₀, R₁₈, Het, Ar et alk ayant les mêmes significations que dans la formule (I).

Cette réaction s'effectue généralement au sein d'un solvant inerte tel que le diméthylformamide, en présence d'hydroxybenzotriazole, de 1-(3-diméthylaminopropyl)-3 éthylcarbodiimide et d'une base organique telle qu'une trialkylamine (triéthylamine par exemple), à une température comprise entre 0°C et 5°C.

Les composés de formule (I) pour lesquels R représente un radical C(R₄)R₅ dans lequel R₅ représente un radical pyrrol-1-yle éventuellement substitué par un radical -COOR₁₀ peuvent être préparés par action d'un dérivé de formule (VI) pour lequel R₁, R₂ et R₃ ont les mêmes significations que dans la formule (I), Ri représente un radical alkyle, -alk-Het ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, alk, Het et R₁₀ ayant les mêmes significations que dans la formule (I) et Rj représente un radical amino, sur un dérivé de formule : 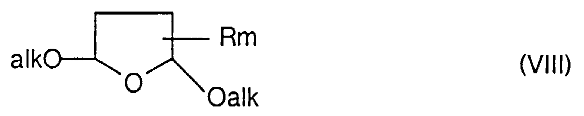 dans laquelle Rm représente un atome d'hydrogène ou un radical -COOR₁₀, alk et R₁₀ ont les mêmes significations que dans la formule (I).

Cette réaction s'effectue généralement au sein d'un solvant inerte tel que l'acide acétique, à la température d'ébullition du milieu réactionnel, éventuellement en présence d'un accepteur d'acide tel que l'acétate de sodium.

Les dérivés de formule (VIII) peuvent être obtenus par application ou adaptation des méthodes décrites par J. FAKSTORP et coll., J. Am. Chem. Soc., 72, 869 (1950), N. CLAUSON-KAAS et coll., Acta Chem. Scan., 6, 551 (1952) et STIBOR et coll., Collect. Czech. Chem. Commun., 47 (12), 3261 (1992).

Les composés de formule (I) pour lesquels R représente un radical C(R₄)R₅ dans lequel R₅ représente un radical -NH-CO-NH-alk-Ar dont Ar est éventuellement substitué, -NH-CO-NH-Het, -NH-CO-NH-alk-Het, -NH-CO-NH-Ar dont Ar est éventuellement substitué, -NH-CO-NH-alk ou -NH-CO-NH₂ peuvent être préparés par action d'un composé de formule (I) correspondant pour lequel R représente un radical C(R₄)R₅, R₅ représente un radical -NR₈R₉, R₈ et R₉ représentent des atomes d'hydrogène sur un dérivé O=C=N-Rn dans lequel Rn représente un radical triméthylsilyle, alkyle, -Het, -alk-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, -alk-Het, phényle éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀ dans lesquels R₁₀, alk, Ar et Het ont les mêmes significations que dans la formule (I).

Cette réaction s'effectue généralement au sein d'un solvant inerte tel que le diméthylformamide, le tétrahydrofuranne ou le dioxanne, à une température comprise entre 20°C et la température d'ébullition du milieu réactionnel. Pour les composés pour lesquels R₅ représente un radical -NH-CO-NH₂, cette réaction est suivie d'une hydrolyse du dérivé silylé précédemment obtenu au moyen d'une solution aqueuse, à une température comprise entre 20 et 50°C.

Les dérivés O=C=N-Rn sont commercialisés ou peuvent être obtenus par action de phosgène sur l'amine primaire correspondante, par adaptation des méthodes décrites par R.L. SHRINER et coll., Organic Synth., II, 453; G.M. DYON, Organic Synth., I, 165; R.J. SLOCOMPIE et coll., J. Am. Chem. Soc., 72, 1888 (1950) et S. PATAI, "The chemistry of cyanates and their thio derivatives", Ed. John Wiley and Sons, page 619 (1977). Les amines primaires correspondantes sont commercialisées ou celles pour lesquelles Rn représente un radical Het ou Ar éventuellement substitué peuvent être obtenues par application ou adaptation des méthodes décrites par B.A. TERTOV et coll., Khim. Geterotsikl. Soedin, Il, 1552 (1972) et R.C. LAROCK, "Comprehensive Organic Transformations", Ed. VCH, page 399, qui consiste à faire réagir l'organolithien ou l'organomagnésien de l'hétérocycle ou du benzène éventuellement substitué sur PhN₃, en présence d'acide acétique, de NH₂OCH₃, (PHO)₂PON₃ ou de N₃CH₂Si(CH₃)₃. Les organolithiens ou organomagnésiens peuvent être obtenus par application ou adaptation des méthodes décrites par D.L. COMINS et coll., J. Org. Chem., 52, 104 (1987); N. FURUKAWA et coll., Tetrahedron Lett., 28 (47), 5845 (1987); A.R. KATRITZKY et coll., Org. Prep. Procedure Int., 20 (6), 585 (1988); A.J. CLARKE et coll., Tetrahedron Lett., 27, 2373 (1974) et A.W. GSCHWEND et coll., Organic Reaction, 26, 1 (1979). Les amines pour lesquelles Rn représente un radical -alk-Het ou -alk-Ar dont Ar est éventuellement substitué sont commercialisées ou sont obtenues à partir des halogénures correspondants par action de NaN(SiCH₃)₃ ou du sel de potassium du phtalimide, au sein d'un solvant inerte tel que le diméthylformamide, en présence d'une base organique telle qu'une trialkylamine ou la pyridine, à une température comprise entre 0°C et la température d'ébullition du milieu réactionnel suivie d'une hydrolyse en milieu acide (HCI par exemple), à une température comprise entre 20°C et la température d'ébullition du milieu réactionnel. Les dérivés H₂N-alk-Ar dont Ar est éventuellement substitué peuvent également être obtenus par application ou adaptation des méthodes décrites par J.F. KING et coll., J. Am. Chem. Soc., 114, 3028 (1992); B.M. ADGER et coll., Tetrahedron Lett., 25 (45), 5219 (1984); R. SCARPATI et coll., Gazz. Chim. Ital., 97 (5), 654 (1967).

Les composés de formule (I) pour lesquels R représente un radical CH-R₆ dans lequel R₆ représente un radical hydroxy peuvent être préparés par réduction d'un composé de formule (I) correspondant pour lequel R représente un radical C=R₇ et R₇ représente un atome d'oxygène.

Cette réaction s'effectue de préférence au sein d'un solvant inerte tel qu'un alcool aliphatique inférieur (méthanol, éthanol par exemple), en présence de borohydrure de sodium, à une température comprise entre 15 et 40°C.

Les composés de formule (I) pour lesquels R représente un radical CH-R₆ dans lequel R₆ représente un radical alkyle (2-11C), phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀ ou -alk-Het peuvent être préparés par hydrogénation d'un dérivé de formule : 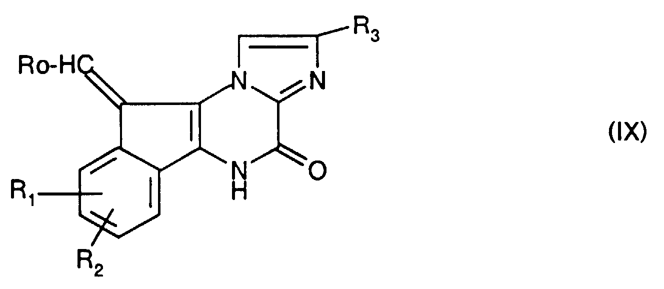 dans laquelle R₁, R₂ et R₃ ont les mêmes significations que dans la formule (I), Ro représente un radical alkyle en chaîne droite ou ramifiée contenant 1 à 10 atomes de carbone, phényle, phénylalkyle(1-5C) dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, -Het, -alk(1-5C)-Het dans lesquels alk, Het et R₁₀ ont les mêmes significations que dans la formule (I).

Cette réduction s'effectue au moyen d'hydrogène sous une pression de 1 à 20 bar, en présence d'un catalyseur d'hydrogénation tel que le charbon palladié, l'hydroxyde de palladium ou le palladium (N. RICO et coll., Nouveau Journal de Chimie, 10, 1, 25 (1986)), au sein d'un solvant inerte tel que le diméthylformamide, l'acide acétique, l'acétate d'éthyle, un alcool aliphatique inférieur (méthanol ou éthanol par exemple) ou un mélange de ces solvants, à une température comprise entre 20 et 60°C ou par adaptation de la méthode de L.M. STRAWN et coll., J. Med. Chem., 32, 2104 (1989) qui consiste à faire réagir le dérivé éthylénique sur le sulfate d'hydroxylamine et H₂NOSO₃H, en milieu aqueux, à un pH compris entre 6 et 7, à une température de 10°C.

Les dérivés de formule (IX) pour lesquels Ro représente un radical alkyle en chaîne droite ou ramifiée, contenant 5 à 10 atomes de carbone peuvent être préparés comme décrit précédemment pour leurs homologues (composés de formule (I) pour lesquels R représente un radical C=R₇, R₇ représente un radical -CH-R₁₉ et R₁₉ représente un radical alkyle).

Les composés de formule (I) pour lesquels R représente un radical CH-R₆ dans lequel R₆ représente un radical phénylalkyle éventuellement substitué ou -alk-Het peuvent également être préparés par action d'un composé de formule (I) correspondant pour lequel R représente un radical CH-R₆ et R₆ représente un atome d'hydrogène sur un halogènure de formule Hal-Rp dans laquelle Rp représente un radical phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀ ou -alk-Het dans lesquels alk, Het et R₁₀ ont les mêmes significations que dans la formule (I).

Cette réaction s'effectue de préférence au sein d'un solvant inerte tel que le diméthylsulfoxyde, le diméthylformamide, le tétrahydrofuranne, le dioxanne, l'éther diéthylique, en présence d'une base telle qu'un hydroxyde de métal alcalin (soude, potasse par exemple), éventuellement en présence de bromure de tétrabutylammonium, ou en présence d'un hydrure de métal alcalin (hydrure de sodium par exemple), à une température voisine de 20°C.

Les composés de formule (I) pour lesquels R représente un radical CH-R₆ dans lequel R₆ représente un radical alkyle (1C) peuvent être préparés par réduction d'un composé de formule (I) correspondant pour lequel R représente un radical C=R₇, R₇ représente un radical CH-R₁₉ et R₁₉ représente un radical hydroxy ou -NR₂₅R₂₆.

Cette réduction s'effectue généralement au moyen d'hydrogène, sous une pression de 1 à 50 bar, au sein d'un solvant inerte tel que le diméthylformamide, l'acide acétique, l'acétate d'éthyle, un alcool aliphatique inférieur (méthanol, éthanol par exemple) ou un mélange de ces solvants, en présence d'un catalyseur d'hydrogénation tel que le charbon palladié, l'hydroxyde de palladium, à une température comprise entre 20°C et 60°C.

Les composés de formule (I) pour lesquels R représente un radical CHR₆ et R₆ représente un radical -alk(1C)-OH peuvent être préparés par réduction d'un composé de formule (I) correspondant pour lequel R représente un radical C=R₇, R₇ représente un radical CH-R₁₉ et R₁₉ représente un radical hydroxy.

Cette réduction s'effectue généralement à l'aide d'un agent réducteur tel que le borohydrure de sodium, au sein d'un solvant inerte tel qu'un alcool aliphatique inférieur (méthanol, éthanol par exemple), à une température voisine de 20°C.

Les composés de formule (I) pour lesquels R représente un radical CHR₆ et R₆ représente un radical -alk(2-6C)-OH peuvent être préparés par réduction d'un dérivé de formule (IX) dans laquelle R₁, R₂ et R₃ ont les mêmes significations que dans la formule (I) et Ro représente un radical -alk(1-5C)-O-CH₂-Ar, alk et Ar ayant les mêmes significations que dans la formule (I).

Cette réduction s'effectue de préférence au moyen d'hydrogène, sous une pression de 1 à 50 bar, en présence d'un catalyseur tel que le charbon palladié, l'hydroxyde de palladium, au sein d'un solvant inerte tel que le diméthylformamide, l'acide acétique, un alcool aliphatique inférieur, l'acétate d'éthyle, à une température comprise entre 20 et 60°C.

Les dérivés de formule (IX) pour lesquels Ro représente un radical -alk(1-5C)-O-CH₂-Ar peuvent être obtenus par action d'un composé de formule (I) correspondant pour lequel R représente un radical CH-R₆ et R₆ représente un atome d'hydrogène sur un aldéhyde OHC-alk(1-5C)-O-CH₂-Ar.

Cette réaction s'effectue dans les mêmes conditions que celles mentionnées précédemment pour la préparation des composés de formule (I) pour lesquels R représente un radical C=R₇, R₇ représente un radical CH-R₁₉ et R₁₉ représente un radical alkyle.

Les dérivés OHC-alk(1-5C)-O-CH₂-Ar peuvent être obtenus par application ou adaptation des méthodes décrites par P. SCHORIGIN et coll., Chem. Ber., 68, 838 (1935) et A. GAIFFE et coll., C. R. Acad. Sc. Paris, Ser. C, 266, 1379 (1968).

Les composés de formule (I) pour lesquels R représente un radical CH-R₆ dans lequel R₆ représente un radical -NR₁₄R₁₅, R₁₄ et R₁₅ représentent chacun un atome d'hydrogène peuvent être préparés par hydrolyse d'un composé de formule (I) correspondant pour lequel R représente un radical CH-R₆ dans lequel R₆ représente un radical -NR₁₄R₁₅, R₁₄ représente un atome d'hydrogène, R₁₅ représente un radical -COR₂₂ et R₂₂ représente un radical alkyle, suivie pour les composés pour lesquels R₃ représente un radical alcoxycarbonyle d'une estérification de l'acide correspondant.

Cette hydrolyse s'effectue généralement au moyen d'un acide tel que l'acide chlorhydrique, en milieu aqueux, à la température d'ébullition du milieu réactionnel. L'estérification s'effectue au moyen d'un alcool aliphatique (C1-C6 en chaîne droite ou ramifiée), en milieu acide (acide chlorhydrique ou sulfurique par exemple), à la température d'ébullition du milieu réactionnel.

Les composés de formule (I) pour lesquels R représente un radical CH-R₆ dans lequel R₆ représente un radical -NR₁₄R₁₅, R₁₄ représente un atome d'hydrogène, R₁₅ représente un radical -COR₂₂ et R₂₂ représente un radical alkyle peuvent être préparés par action d'un agent réducteur sur un composé de formule (I) correspondant pour lequel R représente un radical C=R₇ et R₇ représente un radical NOH suivi de l'action d'un anhydride d'acide (alkCO)₂O dans lequel alk représente un radical alkyle.

Cette réaction s'effectue généralement au sein d'un solvant inerte, tel que l'acide acétique, à une température comprise entre 50 et 100°C. Comme agent réducteur on utilise de préférence le zinc.

Les composés de formule (I) pour lesquels R représente un radical CH-R₆ et R₆ représente un radical -NR₁₄R₁₅ ou -alk-NR₁₄R₁₅, dans lesquels R₁₄ et R₁₅, identiques ou différents, représentent chacun un radical alkyle ou bien R₁₄ représente un atome d'hydrogène et R₁₅ représente un radical alkyle, -COR₂₂ ou -SO₂R₂₄, R₂₂ représente un radical alkyle, cycloalkyle, -COOalk, -alk-COOR₁₀, phényle éventuellement substitué par un ou plusieurs substituants choisis parmi les atomes d'halogène et les radicaux alkyle, alcoxy, nitro, amino, hydroxy, -alk-NH₂, -COOR₁₀, cyano et -alk-COOR₁₀, phénylalkyle dont le noyau phényle est éventuellement substitué par un ou plusieurs substituants choisis parmi les atomes d'halogène et les radicaux alkyle, alcoxy, nitro, amino, hydroxy, -alk-NH₂, -COOR₁₀, cyano et -alk-COOR₁₀, -OR₁₇, -Het, -alk-Het, -alk-NR₁₀R₁₂ et R₂₄ représente un radical alkyle ou phényle peuvent être préparés par action d'un composé de formule (I) correspondant pour lequel R₁₄ et R₁₅ représentent chacun un atome d'hydrogène sur un halogènure de formule Hal-Rr dans laquelle Rr représente un radical alkyle, -COR₂₂ ou -SO₂R₂₄, R₂₂ représente un radical alkyle, cycloalkyle, -COOalk, -alk-COOR₁₀, phényle éventuellement substitué par un ou plusieurs substituants choisis parmi les atomes d'halogène et les radicaux alkyle, alcoxy, nitro, amino, hydroxy, -alk-NH₂, -COOR₁₀, cyano et -alk-COOR₁₀, phénylalkyle dont le noyau phényle est éventuellement substitué par un ou plusieurs substituants choisis parmi les atomes d'halogène et les radicaux alkyle, alcoxy, nitro, amino, hydroxy, -alk-NH₂, -COOR₁₀, cyano et -alk-COOR₁₀, -OR₁₇, -Het, -alk-Het, -alk-NR₁₀R₁₂ et R₂₄ représente un radical alkyle ou phényle, alk, Het, R₁₂, R₁₇ et R₁₀ ayant les mêmes significations que dans la formule (I).

Cette réaction s'effectue de préférence au sein d'un solvant inerte tel que le diméthylformamide, le tétrahydrofuranne ou le diméthylsulfoxyde, en présence d'une base telle qu'une amine tertiaire (la triéthylamine par exemple) ou aromatique (pyridine par exemple) ou une base minérale telle qu'un hydroxyde de métal alcalin (soude, potasse par exemple), à une température comprise entre 20°C et la température d'ébullition du milieu réactionnel.

Les dérivés Hal-Rr sont commercialisés ou ceux pour lesquels Rr représente un radical alkyle ou -COR₂₂ peuvent être obtenus à partir des acides carboxyliques correspondants par application ou adaptation des méthodes décrites par B. HELFERICH et coll., Organic Synth., I, 147; R. ADAMS et coll., Organic Synth., I, 304 et J. GASON, Organic Synth., III, 169 et ceux pour lesquels Rr représente un radical -SO₂R₂₄ peuvent être obtenus à partir des acides sulfoniques correspondants par réaction d'un dérivé halogéné du phosphore (PCl₅, POCl₃ par exemple) ou de chlorure de thionyle, en phase aqueuse ou au sein d'un solvant inerte (dichlorométhane par exemple, à une température comprise entre 20 et 40°C.

Les composés de formule (I) pour lesquels R représente un radical CH-R₆ et R₆ représente un radical -NR₁₄R₁₅ ou -alk-NR₁₄R₁₅ dans lesquels R₁₄ représente un atome d'hydrogène, R₁₅ représente un radical -COR₂₂ ou -CSR₂₃, R₂₂ représente un radical -NH-alk, -NH₂, -NH-Ar dont Ar est éventuellement substitué, -NH-alk-Ar dont Ar est éventuellement substitué, -NH-alk-Het ou -NH-Het et R₂₃ représente un radical -NH-alk, -NH₂, -NH-Ar ou -NH-Het peuvent être préparés par action d'un composé de formule (I) correspondant pour lequel R représente un radical CH-R₆, R₆ représente un radical -NR₁₄R₁₅, R₁₄ et R₁₅ représentent chacun un atome d'hydrogène sur un dérivé Rs-N=C=Rt pour lequel Rs représente un radical triméthylsilyle, alkyle, phényle éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, -alk-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, -alk-Het ou Het dans lesquels Het, alk, R₁₀ et Ar ont les mêmes significations que dans la formule (I) et Rt représente un atome d'oxygène ou de soufre, suivie éventuellement par une hydrolyse.

Cette réaction s'effectue de préférence au sein d'un solvant inerte tel que le diméthylformamide, le tétrahydrofuranne ou le dioxanne, à une température comprise entre 20°C et la température d'ébullition du milieu réactionnel. Pour les composés pour lesquels R₂₂ et R₂₃ sont des radicaux NH₂, cette réaction est suivie d'une hydrolyse du dérivé silylé précédemment obtenu au moyen d'une solution aqueuse, à une température comprise entre 20 et 50°C.

Les dérivés Rs-N=C=Rt peuvent être obtenus à partir des amines primaires correspondantes par action du phosgène ou de thiophosgène par application ou adaptation des méthodes décrites par R.L. SHRINER et coll., Organic Synth., II, 453 et G.M. DYSON, Organic Synth., I, 165; R.J. SLOCOMPIE et col., J. Am. Chem. Soc., 72, 1888 (1950) et S. PATAI, "The chemistry of cyanates and their thio derivatives", Ed. John Wiley and Sons, pages 619 et 819 (1977).

Les composés de formule (I) pour lesquels R représente un radical CH-R₆ et R₆ représente un radical -NR₁₄R₁₅ ou -alk-NR₁₄R₁₅ dans lesquels R₁₄ représente un atome d'hydrogène, R₁₅ représente un radical -COR₂₂ et R₂₂ représente un radical -alk (1C)-NR₁₀R₁₂ dans lequel R₁₀ et R₁₂ sont des atomes d'hydrogène peuvent être préparés par action d'un composé de formule (I) correspondant pour lequel R₁₄ et R₁₅ représentent chacun un atome d'hydrogène sur un acide HOOC-CH₂-NH-Ru dans lequel Ru représente un groupe protecteur de la fonction amine tel que tert-butoxycarbonyle suivie d'une hydrolyse.

Cette réaction s'effectue, de préférence, au sein d'un solvant inerte tel que le diméthylformamide, en présence d'hydroxybenzotriazole, de 1-(3-diméthylaminopropyl)-3 éthylcarbodiimide et d'une base organique telle qu'une trialkyamine (triéthylamine par exemple), à une température comprise entre 0 et 5°C. L'hydrolyse s'effectue généralement au moyen d'acide trifluoroacétique, à une température voisine de 20°C.

Les composés de formule (I) pour lesquels R représente un radical CH-R₆, R₆ représente un radical -alk-NR₁₄R₁₅, R₁₄ et R₁₅ représentent chacun un atome d'hydrogène à l'exception de ceux pour lesquels R₃ représente un radical carboxamido peuvent être préparés par action de brome et de soude sur un composé de formule (I) correspondant pour lequel R représente un radical CH-R₆, R₆ représente un radical -alk-CONR₁₀R₂₁ et R₁₀ et R₂₁ représentent des atomes d'hydrogène.

Cette réaction s'effectue généralement en milieu aqueux, à une température comprise entre 20 et 70°C.

Les composés de formule (I) pour lesquels R représente un radical CH-R₆, R₆ représente un radical -alk-NR₁₄R₁₅, R₁₄ et R₁₅ représentent chacun un atome d'hydrogène et R₃ représente un radical carboxamido peuvent être préparés par action d'ammoniac sur un composé de formule (I) correspondant pour lequel R représente un radical CH-R₆, R₆ représente un radical -alk-NR₁₄R₁₅, R₁₄ et R₁₅ représentent chacun un atome d'hydrogène et R₃ représente un radical alcoxycarbonyle.

Cette réaction s'effectue au sein d'un solvant inerte tel qu'un alcool aliphatique inférieur (méthanol, éthanol par exemple), à une température voisine de 20°C.

Les composés de formule (I) pour lesquels R représente un radical CH-R₆, R₆ représente un radical -NR₁₄R₁₅ ou -alkNR₁₄R₁₅ dans lesquels R₁₄ représente un atome d'hydrogène et R₁₅ représente un radical -COR₂₂, R₂₂ représente un radical alkyle, cycloalkyle, -alkCOOR₁₀, phényle éventuellement substitué, phénylalkyle dont le phényle est éventuellement substitué, Het, -alk-Het, -alk-NR₁₀R₁₂ peuvent être préparés par action d'un composé de formule (I) correspondant pour lequel R représente un radical CH-R₆, R₆ représente un radical -NR₁₄R₁₅ ou -alk-NR₁₄R₁₅ dans lesquels R₁₄ et R₁₅ représentent chacun un atome d'hydrogène sur un acide HOOC-R₂₂, R₂₂ représente un radical alkyle, cycloalkyle, -Het, -alk-COOR₁₀, -alk-NR₁₀R₁₂, phénylalkyle dont le phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, -alk-Het, phényle éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, alk, Het, R₁₀, R₁₂ ayant les mêmes significations que dans la formule (I) ou l'anhydride correspondant.

Cette réaction s'effectue généralement au sein d'un solvant inerte tel que le diméthylformamide, en présence d'hydroxybenzotriazole, de 1-(3-diméthylaminopropyl)-3 éthylcabodiimide et d'une base organique telle qu'une trialkylamine (triéthylamine par exemple), à une température comprise entre 0°C et 5°C. Lorsque l'on utilise l'anhydride, on opère de préférence au sein de l'acide acétique, en présence d'acétate de sodium, à une température voisine de 50°C.

Les composés de formule (I) pour lesquels R représente un radical CH-R₆ et R₆ représente un radical -alk-COOR₁₀ peuvent être préparés par hydrogénation d'un dérivé de formule (IX) correspondant pour lequel R₁, R₂ et R₃ ont les mêmes significations que dans la formule (I) et Ro représente un radical -alk(1-5C)-COOR₁₀ ou -COOR₁₀ dans lesquels alk a les mêmes significations que dans la formule (I), R₁₀ représente un radical alkyle ou phénylalkyle, suivie éventuellement par une saponification de l'ester ainsi obtenu.

Cette réduction s'effectue au moyen d'hydrogène sous une pression de 1 à 20 bar, en présence d'un catalyseur d'hydrogénation tel que le charbon palladié, l'hydroxyde de palladium ou le palladium (N. RICO et coll., Nouveau Journal de Chimie, 10, 1, 25 (1986)), au sein d'un solvant inerte tel que le diméthylformamide, l'acide acétique, l'acétate d'éthyle, un alcool aliphatique inférieur (méthanol ou éthanol par exemple) ou un mélange de ces solvants, à une température comprise entre 20 et 60°C ou par adaptation de la méthode de L.M. STRAWN et coll., J. Med. Chem., 32, 2104 (1989) qui consiste à faire réagir le dérivé éthylénique sur le sulfate d'hydroxylamine et H₂NOSO₃H, en milieu aqueux, à un pH compris entre 6 et 7, à une température de 10°C. La saponification s'effectue par toute méthode connue et, de préférence, au moyen d'un acide tel que l'acide chlorhydrique, au sein d'un alcool aliphatique inférieur tel que l'éthanol, à une température de 20 à 60°C ou au moyen d'acide trifluoroacétique à une température voisine de 20 à 60°C.

Les dérivés de formule (IX) pour lesquels R₁, R₂ et R₃ ont les mêmes significations que dans la formule (I) et Ro représente un radical -COOR₁₀ dans lequel R₁₀ représente un radical alkyle peuvent être obtenus par action d'un composé de formule (I) correspondant pour lequel R représente un radical CH-R₆ et R₆ représente un atome d'hydrogène sur un glyoxalate d'alkyle.

Cette réaction s'effectue au sein d'un solvant inerte tel que le diméthylsulfoxyde, en présence d'un hydrure de métal alcalin (hydrure de sodium ou de potassium par exemple), à une température voisine de 20°C.

Les composés de formule (I) pour lesquels R représente un radical CH-R₆, R₆ représente un radical -alk-CO-NR₁₀R₂₁ peuvent être préparés par action d'un composé de formule (I) correspondant pour lequel R représente un radical CH-R₆ et R₆ représente un radical -alk-COOR₁₀, alk ayant les mêmes significations que dans la formule (I) et R₁₀ représente un atome d'hydrogène ou un radical alkyle sur une amine HNR₁₀R₂₁ dans laquelle R₁₀ et R₂₁ ont les mêmes significations que dans la formule (I).

Lorsque l'on met en oeuvre l'acide, on opère en présence d'un agent de condensation utilisé en chimie peptidique tel qu'un carbodiimide (par exemple le N,N'-dicyclohexylcarbodiimide) ou le N,N'-diimidazole carbonyle, dans un solvant inerte tel qu'un éther (tétrahydrofuranne, dioxanne par exemple), un amide (diméthylformamide) ou un solvant chloré (chlorure de méthylène, dichloro-1,2 éthane, chloroforme par exemple) à une température comprise entre 0°C et la température de reflux du mélange réactionnel. Lorsque l'on met en oeuvre un ester, on opère alors soit en milieu organique, éventuellement en présence d'un accepteur d'acide tel qu'une base organique azotée (trialkylamine, pyridine, 1,8-diazabicyclo[5.4.0]undec-7-ène ou 1,5-diazabicyclo[4.3.0]non-5-ène par exemple), dans un solvant tel que cité ci-dessus, ou un mélange de ces solvants, à une température comprise entre 0°C et la température de reflux du mélange réactionnel, soit en milieu hydroorganique biphasique en présence d'une base alcaline ou alcalino-terreuse (soude, potasse) ou d'un carbonate ou bicarbonate d'un métal alcalin ou alcalino-terreux à une température comprise entre 0 et 40°C.

Les amines HNR₁₀R₂₁ peuvent être obtenus à partir des halogénures correspondants par application ou adaptation des méthodes décrites par R.C. LAROCK, "Comprehensive Organic Transformations", Ed. VCH, page 397 (1989).

Les composés de formule (I) pour lesquels R représente un radical CH-R₆, R₆ représente un radical -alk(C1)-CO-NR₁₀R₂₁ et R₁₀ et R₂₁ représentent des atomes d'hydrogène peuvent également être préparés par hydrogénation d'un dérivé de formule (IX) dans laquelle R₁, R₂ et R₃ ont les mêmes significations que dans la formule (I) et Ro représente un radical -CONH₂.

Cette réaction s'effectue généralement soit au moyen d'hydrogène, au sein d'un solvant inerte tel que le diméthylformamide, en présence d'un catalyseur d'hydrogènation tel que le charbon palladié ou le palladium, à une température voisine de 20 à 30°C, soit par application ou adaptation de la méthode de L.M. STRAWN et coll., J. Med. Chem., 32, 2104 (1989) qui consiste à faire réagir le dérivé éthylénique sur le sulfate d'hydroxylamine et H₂NOSO₃H, en milieu aqueux, à un pH compris entre 6 et 7, à une température de 10°C

Les dérivés de formule (IX) dans laquelle R₁, R₂ et R₃ ont les mêmes significations que dans la formule (I) et Ro représente un radical -CONH₂ peuvent être obtenus par action d'ammoniac sur un composé de formule (IX) correspondant pour lequel Ro représente un radical -COOR₁₀ dans lequel R₁₀ représente un radical alkyle dans les conditions décrites par D.I. MOWRY et coll., Organic Synth., IV, 486 et J. KLEINBERG et coll., Organic Synth., IV, 516.

Les composés de formule (I) pour lesquels R représente un radical CH-R₆ et R₆ représente un radical -R₁₆-COOR₁₀ peuvent être préparés par action d'un composé de formule (I) correspondant pour lequel R représente un radical CH-R₆ et R₆ représente un atome d'hydrogène sur un dérivé de formule OHC-alk(0-5C)-COOR₁₀ dans laquelle alk et R₁₀ ont les mêmes significations que dans la formule (I).

Cette réaction s'effectue de préférence au sein d'un solvant inerte tel que le diméthylsulfoxyde, en présence d'un hydrure de métal alcalin tel que l'hydrure de sodium, à une température voisine de 20°C.

Les dérivés de formule OHC-alk(0-5C)-COOR₁₀ peuvent être obtenus par application ou adaptation de la méthode décrite par L.A. CARPINO, J. Org. Chem., 29, 2820 (1964).

Les composés de formule (I) pour lesquels R représente un radical CH-R₆ et R₆ représente un radical -NH-CHO peuvent être préparés par action d'un composé de formule (I) correspondant pour lequel R représente un radical CH-R₆, R₆ représente un radical -NR₁₄R₁₅, R₁₄ et R₁₅ représentent des atomes d'hydrogène sur CH₃COOCHO.

Cette réaction s'effectue de préférence au sein d'un solvant inerte tel que l'acide formique, en présence d'acétate de sodium, à une température voisine de 20°C

Les composés de formule (I) pour lesquels R représente un radical CH-R₆ et R₆ représente un radical -CO-COOR₁₀ peuvent être préparés par oxydation d'un composé de formule (I) correspondant pour lequel R représente un radical CH-R₆, R₆ représente un radical -R₁₆-COOR₁₀ et R₁₆ représente un radical -CHOH- suivie éventuellement d'une estérification.

Cette oxydation s'effectue de préférence au moyen de permanganate de potassium, au sein d'une solution de soude 3N, à une température voisine de -3°C ou au moyen de platine sur charbon, au sein d'une solution de soude 2N, à une température de 70°C. L'estérification s'effectue de préférence au moyen d'un alcool aliphatique inférieur, en présence d'un acide tel que l'acide chlorhydrique ou sulfurique, à la température d'ébullition du milieu réactionnel.

Les composés de formule (I) pour lesquels R représente un radical CH-R₆, R₆ représente un radical pyrrol-1-yle éventuellement substitué par un radical -COOR₁₀ peuvent être préparés par action d'un composé de formule (I) correspondant pour lequel R représente un radical CH-R₆ et R₆ représente un radical -NR₁₄R₁₅, R₁₄ et R₁₅ représentent des atomes d'hydrogène sur un dérivé de formule (VIII) dans laquelle Rm représente un atome d'hydrogène ou un radical -COOR₁₀ dans lequel R₁₀ a les mêmes significations que dans la formule (I).

Cette réaction s'effectue généralement au sein d'un solvant inerte tel que l'acide acétique, en présente éventuellement d'un accepteur d'acide tel que l'acétate de sodium, à la température d'ébullition du milieu réactionnel.

Les composés de formule (I) pour lesquels R représente un radical CH-R₆ et R₆ représente un radical -COOalk peuvent être préparés par action d'un acide minéral sur un dérivé de formule : 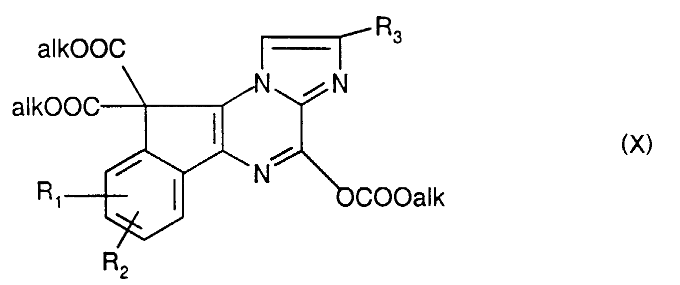 dans laquelle R₁, R₂ et R₃ ont les mêmes significations que dans la formule (I) et alk représente un radical alkyle.

Cette réaction s'effectue généralement au sein d'un solvant inerte tel que le tétrahydrofuranne, à une température de 0°C. Comme acide minéral, on utilise de préférence l'acide chlorhydrique, en solution aqueuse 1N.

Les dérivés de formule (X) peuvent être obtenus par action d'un composé de formule (I) correspondant pour lequel R représente un radical CH-R₆ et R₆ représente un atome d'hydrogène sur un halogénure Hal-COOalk dans lequel alk représente un radical alkyle.

Cette réaction s'effectue généralement au sein d'un solvant inerte tel que le dioxanne, en présence d'un hydrure de métal alcalin (sodium par exemple), à une température comprise entre 20°C et la température d'ébullition du milieu réactionnel.

Les dérivés Hal-COOalk sont commercialisés ou peuvent être obtenus par application ou adaptation des méthodes décrites dans HOUBEN-WEYL, band 8, page 102 (1952).

Les composés de formule (I) pour lesquels R₃ représente un radical carboxy peuvent également être préparés par hydrolyse d'un composé de formule (I) correspondant pour lequel R₃ représente un radical alcoxycarbonyle.

Cette hydrolyse s'effectue soit au moyen d'un acide (acide chlorhydrique ou sulfurique par exemple), en solution aqueuse, à une température comprise entre 20°C et la température d'ébullition du milieu réactionnel; soit au moyen d'une base (soude, potasse, carbonate de potassium par exemple), en solution aqueuse ou en milieu hydroorganique (eau-tétrahydrofuranne, eau-dioxanne par exemple), à une température comprise entre 0°C et la température d'ébullition du milieu réactionnel.

Les composés de formule (I) pour lesquels R₁ et/ou R₂ représentent un radical -NH-CO-NR₁₁R₁₂ dans lequel R₁₁ représente un atome d'hydrogène ou un radical alkyle (1-9C en chaîne droite ou ramifiée), -alk-COOR₁₀, -alk-Het, -alk-NR₁₂R₁₀, phénylalkyle dont le noyau phényle est éventuellement substitué par un ou plusieurs substituants choisis parmi les atomes d'halogène et les radicaux alkyle, alcoxy, nitro, amino, hydroxy, -alk-NH₂, carboxy, alcoxycarbonyle, cyano et -alk-COOR₁₀, phényle éventuellement substitué par un ou plusieurs substituants choisis parmi les atomes d'halogène et les radicaux alkyle, alcoxy, nitro, amino, hydroxy, -alk-NH₂, carboxy, alcoxycarbonyle, cyano et -alk-COOR₁₀ ou -Het et R₁₂ représente un atome d'hydrogène peuvent également être préparés par action d'un composé de formule (I) correspondant pour lequel R₁ et/ou R₂ représente un radical amino avec un isocyanate O=C=NR₁₁ dans lequel R₁₁ a les mêmes significations que précédemment ou représente un radical triméthylsilyle.

Cette réaction s'effectue au sein d'un solvant inerte tel que le tétrahydrofuranne, le diméthylformamide ou le dioxanne, à une température comprise entre 20°C et la température d'ébullition du milieu réactionnel. Pour les composés pour lesquels R₁₁ représente un atome d'hydrogène, cette réaction est suivie d'une hydrolyse du dérivé silylé précédemment obtenu au moyen d'une solution aqueuse, à une température comprise entre 20 et 50°C.

Les isocyanates O=C=NR₁₁ sont commercialisés ou peuvent être préparés comme précédemment décrit pour les isocyanates de formule O=C=N-Rn.

Les composés de formule (I) pour lesquels R₁ et/ou R₂ représentent un radical amino peuvent également être préparés par réduction d'un composé de formule (I) correspondant pour lequel R₁ et/ou R₂ représentent un radical nitro.

Cette réduction s'effectue généralement au moyen d'hydrogène, sous une pression de 1 à 2 bar, en présence d'un catalyseur tel que le palladium sur charbon, à une température voisine de 20°C.

Les composés de formule (I) pour lesquels R représente un radical CH-R₆, R₆ représente un radical -NR₁₄R₁₅, R₁₄ et R₁₅ représentent des atomes d'hydrogène peuvent également être préparés par réduction en présence d'un agent réducteur d'un composé de formule (I) correspondant pour lequel R représente un radical C=R₇ et R₇ représente un radical NOH.

Cette réaction s'effectue de préférence au sein d'un alcool aliphatique inférieur (éthanol par exemple), en présence d'ammoniaque et d'acétate d'ammonium, à la température d'ébullition du milieu réactionnel. Comme agent réducteur on utilise de préférence le zinc.

Les composés de formule (I) pour lesquels R représente un radical C=R₇, R₇ représente un radical CH-R₁₉, R₁₉ représente un radical phényle substitué par un radical COOR₁₀ et R₁₀ représente un atome d'hydrogène peuvent également être préparés par hydrolyse d'un composé de formule (I) correspondant pour lequel R représente un radical C=R₇, R₇ représente un radical CH-R₁₉, R₁₉ représente un radical phényle substitué par un radical cyano.

Cette réaction s'effectue au moyen d'un acide tel que l'acide sulfurique, à la température d'ébullition du milieu réactionnel.

Les composés de formule (I) pour lesquels R représente un radical CH-R₆ et R₆ représente un radical 2-oxo-2,5-dihydro-pyrrol-1-yle peuvent être préparés par action d'un composé de formule (I) correspondant pour lequel R représente un radical CH-R₆, R₆ représente un radical -NR₁₄R₁₅, R₁₄ et R₁₅ représentent des atomes d'hydrogène sur le 2,5-diméthoxy-2,5-dihydrofuranne.

Cette réaction s'effectue généralement au sein de l'acide acétique, en présence d'acétate de sodium, à une température comprise entre 40 et 70°C.

Il est entendu pour l'homme du métier que, pour la mise en oeuvre des procédés selon l'invention décrits précédemment, il peut être nécessaire d'introduire des groupes protecteurs des fonctions amino, hydroxy et carboxy afin d'éviter des réactions secondaires. Ces groupes sont ceux qui permettent d'être éliminés sans toucher au reste de la molécule. Comme exemples de groupes protecteurs de la fonction amino on peut citer les carbamates de tert-butyle ou de méthyle qui peuvent être régénérés au moyen d'iodotriméthylsilane. Comme exemples de groupes protecteurs de la fonction hydroxy, on peut citer les triéthylsilyle, benzyle. Comme groupes protecteurs des fonctions carboxy, on peut citer les esters (méthoxyméthylester, tétrahydropyranylester, benzylester par exemple, les oxazoles et les 2-alkyl-1,3-oxazolines. D'autres groupes protecteurs utilisables sont décrits par W. GREENE et coll., Protecting Groups in Organic Synthesis, second edition, 1991, John Wiley & Sons.

Les composés de formule (I) peuvent être purifiés par les méthodes connues habituelles, par exemple par cristallisation, chromatographie ou extraction.

Les énantiomères des composés de formule (I) pour lesquels R représente un radical C(R₄)R₅ ou CH-R₆ peuvent être obtenus par dédoublement des racémiques par exemple par chromatographie sur colonne chirale selon W.H. PIRCKLE et coll., asymetric synthesis, vol. 1, Academic Press (1983) ou par synthèse à partir des précurseurs chiraux.

Les diastéréoisomères des composés de formule (I) pour lesquels R représente un radical C(R₄)R₅ ou CH-R₆ comportant un ou plusieurs carbones chiraux et les différents isomères E et Z des composés de formule (I) peuvent être séparés par les méthodes connues habituelles, par exemple par cristallisation ou chromatographie.

Les composés de formule (I) comportant un reste basique peuvent être éventuellement transformés en sels d'addition avec un acide minéral ou organique par action d'un tel acide au sein d'un solvant organique tel qu'un alcool aliphatique inférieur, une cétone, un éther ou un solvant chloré.

Les composés de formule (I) comportant un reste acide peuvent éventuellement être transformés en sels métalliques ou en sels d'addition avec les bases azotées selon des méthodes connues en soi. Ces sels peuvent être obtenus par action d'une base métallique (alcaline ou alcalinoterreuse par exemple), de l'ammoniac, d'une amine ou d'un sel d'une amine sur un composé de formule (I), dans un solvant. Le sel formé est séparé par les méthodes habituelles.

Ces sels font également partie de l'invention.

Comme exemples de sels pharmaceutiquement acceptables, peuvent être cités les sels d'addition avec les acides minéraux ou organiques (tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate), les sels avec les métaux alcalins (sodium, potassium, lithium) ou avec les métaux alcalinoterreux (calcium, magnésium), le sel d'ammonium, les sels de bases azotées (éthanolamine, triméthylamine, méthylamine, benzylamine, N-benzyl-β-phénéthylamine, choline, arginine, leucine, lysine, N-méthyl glucamine).

Les composés de formule (I) présentent des propriétés pharmacologiques intéressantes. Ces composés sont des antagonistes du récepteur de l'acide α-amino-3-hydroxy-5-méthyl-4-isoxazolepropionique (AMPA), connu aussi sous le nom de récepteur du quisqualate.

Par ailleurs, les composés de formule (I) sont des antagonistes non compétitifs du récepteur N-méthyl-D-aspartate (NMDA) et, plus particulièrement, ce sont des ligands pour les sites modulateurs de la glycine du récepteur NMDA.

Ces composés sont donc utiles pour traiter ou prévenir toutes les ischémies (telles l'ischémie focale ou globale) consécutives à des accidents vasculaires cérébraux, un arrêt cardiaque, une hypotension artérielle, une intervention chirurgicale cardiaque ou pulmonaire ou une hypoglycémie sévère. Ils sont également utiles dans le traitement des effets dus à une anoxie, qu'elle soit périnatale ou consécutive à une noyade ou à des lésions cérébro-spinales. Ces composés peuvent également être utilisés pour traiter ou prévenir l'évolution de maladies neurodégénératives, de la chorée d'HUNTINGTON, de la maladie d'ALZHEIMER, de la sclérose latérale amyotrophique, de l'atrophie olivo-pontocérébelleuse et de la maladie de PARKINSON. Ces composés peuvent aussi être utilisés vis-à-vis des manifestations épileptogènes et/ou convulsives, pour le traitement des traumatismes cérébraux ou spinaux, des traumatismes liés à la dégénérescence de l'oreille interne (R. PUJOL et coll., Neuroreport, 3, 299-302 (1992) ou de la rétine (J.L. MONSINGER et coll., Exp. Neurol., 113, 10-17 (1991), de l'anxiété (KEHNE et coll., Eur. J. Pharmacol., 193, 283 (1991)), de la dépression (TRULLAS et coll.,Eur. J. Pharmacol., 185, 1 (1990)), de la schizophrénie (REYNOLDS, TIPS, 13, 116 (1992)), du syndrome de TOURETTE, des encéphalopathies hépatiques, en tant qu'analgésiques (DICKENSON et coll., Neurosc. Letters, 121, 263 (1991)), antiinflammatoire (SLUTA et coll., Neurosci. Letters, 149, 99-102 (1993)) antianorexiques (SORRELS et coll., Brain Res., 572, 265 (1992)), antimigraineux, antiémétiques et pour traiter les empoisonnements par des neurotoxines ou d'autres substances agonistes du récepteur NMDA, ainsi que les troubles neurologiques associés aux maladies virales telles que le sida (LIPTON et coll., Neuron, 7, 111 (1991)), la rage, la rougeole et le tétanos (BAGETTA et coll., Br. J. Pharmacol., 101, 776 (1990)). Ces composés sont aussi utiles pour la prévention des symptômes d'abstinence aux drogues et à l'alcool et de l'inhibition de l'accoutumance et de la dépendance aux opiacés. Ils peuvent également être utilisés dans le traitement des déficits liés à des anomalies mitochondriales telles que la myopathie mitochondriale, le syndrome de LEBER, l'encéphalopathie de WERNICKE, le syndrome de RETT, l'homocystéinémie, l'hyperprolinémie, l'hydroxybutirique-aminoacidurie, l'encéphalopathie saturnine (intoxication au plomb) et la déficience en sulfite oxydase.

L'affinité des composés de formule (I) vis-à-vis du récepteur AMPA a été déterminée en étudiant l'antagonisme de la fixation spécifique du [³H]-AMPA sur des membranes de cortex cérébral de rat (HONORE et coll., Neuroscience letters, 54, 27 (1985)). Le [³H]-AMPA est mis à incuber en présence de 0,2 mg de protéines à 4°C pendant 30 minutes dans du tampon KH₂PO₄ 10mM, KSCN 100mM, pH7,5. La fixation non spécifique est déterminée en présence de L-glutamate 1mM. La radioactivité liée est séparée par filtration sur filtres PHARMACIA (Printed Filtermate A). L'activité inhibitrice de ces produits est inférieure ou égale à 100 µM.

L'affinité des composés de formule (I) pour le site glycine lié au récepteur NMDA a été déterminée en étudiant l'antagonisme de la fixation spécifique du [³H]-DCKA sur des membranes de cortex cérébral de rat selon la méthode décrite par T. CANTON et coll., J. Pharm. Pharmacol., 44, 812 (1992). Le [³H]-DCKA (20nM) est mis à incuber en présence de 0,1 mg de protéines à 4°C pendant 30 minutes dans du tampon HEPES 50 mM, pH7,5. La fixation non spécifique est déterminée en présence de glycine 1mM. La radioactivité liée est séparée par filtration sur filtres Whatman GF/B. L'activité inhibitrice de ces produits est inférieure ou égale à 100 µM.

Les composés de formule (I) présentent une toxicité faible. Leur DL50 est supérieure à 50 mg/kg par voie IP chez la souris.

Les composés de formule (I) préférés sont ceux pour lesquels R représente un radical CH-R₆, C=R₇ ou C(R₄)R₅, R₁ représente un atome d'hydrogène ou d'halogène ou un radical -NH-CO-NR₁₁R₁₂ dans lequel R₁₁ est un atome d'hydrogène et R₁₂ est un radical alkyle, R₂ représente un atome d'hydrogène, R₃ représente un radical carboxy, alcoxycarbonyle ou carboxamido, R₄ représente un radical alkyle, R₅ représente un radical -alk-COOR₁₀, R₆ représente un atome d'hydrogène ou un radical -alk-Het, 2-oxo-2,5-dihydropyrrol-1-yle, pyrrol-1-yle substitué par -COOR₁₀ ou -NR₁₄R₁₅, R₇ représente un radical CH-R₁₉, NO-alk-COOR₁₀ ou NOH, R₁₉ représente un radical Het ou phényle substitué par -COOR₁₀, soit R₁₄ et R₁₅ sont des atomes d'hydrogène soit R₁₄ est un atome d'hydrogène et R₁₅ est un radical -COR₂₂ dans lequel R₂₂ est un radical -alk-COOR₁₀ ou -NH-Ar.

Les exemples suivants illustrent l'invention.

### EXEMPLE 1

A une solution de 2 g de 1-(1-oxoindan-2-yl)imidazole-2,4-dicarboxylate de diéthyle dans 80 ml d'acide acétique on ajoute peu à peu 51,2 g d'acétate d'ammonium et on chauffe à reflux pendant 2 heures. Après refroidissement à une température voisine de 20°C le précipité formé est filtré et rincé à l'eau distillée, puis avec de la méthyléthylcétone (2x10 ml) pour donner 0,65 g de 4,5-dihydro-4-oxo-10H-imidazo[1,2-a]indéno[1,2-e]pyrazine-2-carboxylate d'éthyle sous forme de solide beige fondant au-dessus de 260°C (Analyse % calculé C : 65,08, H : 4,44, N : 14,23, % trouvé C : 65,2, H : 4,3, N : 13,8).

Le 1-(1-oxoindan-2-yl)imidazole-2,4-dicarboxylate de diéthyle peut être préparé de la façon suivante : à une suspension de 3,5 g d'hydrure de sodium à 60% dans 50 ml de diméthylformamide on ajoute goutte à goutte sous agitation à une température voisine de 15°C une solution de 17,2 g d'imidazole-2,4-dicarboxylate de diéthyle dans 200 ml de diméthylformamide et on maintient l'agitation pendant 45 minutes. On ajoute ensuite en 40 minutes à une température voisine de 15°C une solution de 17,1 g de 2-bromoindanone dans 250 ml de diméthylformamide et on continue d'agiter en laissant remonter la température du milieu réactionnel vers 20°C; l'agitation est maintenue dans cet état pendant 48 heures. Le mélange réactionnel est concentré à l'évaporateur rotatif et traité à l'eau, puis extrait au dichlorométhane (4x150 ml). La phase organique est lavée à l'eau distillée, séchée sur sulfate de magnésium, filtrée et évaporée à l'évaporateur rotatif. On obtient une huile brune (29 g) qui est chromatographiée sur colonne de silice en éluant avec du dichlorométhane pour donner 11,6 g de 1-(1-oxoindan-2-yl)imidazole-2,4-dicarboxylate de diéthyle sous forme de solide meringué utilisé tel quel dans les synthèses ultérieures.

L'imidazole-2,4-dicarboxylate de diéthyle peut être préparé de la manière suivante : à une suspension de 46 g d'α-amino-oximinoacétate d'éthyle dans 800 ml de xylène maintenue à une température voisine de 0°C on ajoute goutte à goutte sous agitation 59 ml de triéthylamine, puis 41,6 g de propiolate d'éthyle. On laisse la température du milieu réactionnel revenir vers 20°C et on continue l'agitation pendant 48 heures. Le mélange réactionnel est traité avec 220 ml d'eau et la phase organique est lavée avec 50 ml d'eau distillée, séchée sur sulfate de magnésium, filtrée et concentrée au demi à l'évaporateur rotatif: le produit insoluble qui s'est formé est éliminé par filtration et le filtrat est séché par azéotropie pendant 24 heures, puis évaporé à l'évaporateur rotatif. Le résidu d'évaporation est trituré dans du dichlorométhane et le solide cristallisé qui se forme est filtré pour donner 7 g d'imidazole-2,4-dicarboxylate de diéthyle sous forme de solide beige fondant à 170°C. Par chromatographie sur colonne de silice, en éluant avec un mélange de cyclohexane et d'acétate d'éthyle (50-50 en volmes), on récupère, à partir du filtrat de cristallisation, d'abord 7,2 g d'imidazole-2,4-dicarboxylate de diéthyle, puis en éluant avec de l'acétate d'éthyle seul 3 g d'imidazole-2,4-dicarboxylate de diéthyle.

L'α-amino-oximinoacétate d'éthyle peut être préparé comme décrit par P. S. BRANCO et coll., Tetrahedron, 48(30), 6335 (1992).

### EXEMPLE 2

On chauffe à reflux pendant 7 heures une solution de 150 mg de 1-(1-oxoindan-2-yl)imidazole-2,4-dicarboxamide dans 8 ml d'acide acétique. Après refroidissement à une température voisine de 20°C le précipité formé est filtré, rincé à l'eau distillée, puis avec de l'oxyde d'isopropyle pour donner 70 mg de 4,5-dihydro-4-oxo-10H-imidazo[1,2-a]indéno[1,2-e]pyrazine-2-carboxamide sous forme de solide beige fondant au-dessus de 260°C (Analyse % calculé C : 63,15, H : 3,79, N : 21,04, % trouvé C : 62,8, H : 3,1, N : 21,3).

Le 1-(1-oxoindan-2-yl)imidazole-2,4-dicarboxamide peut être préparé de la façon suivante : dans une solution de 125 mg de 1-(1-oxoindan-2-yl)imidazole-2,4-dicarboxylate de diéthyle dans 20 ml de méthanol refroidie à une température voisine de -30°C on fait passer un courant d'ammoniac pendant 30 minutes, puis on laisse remonter la température du milieu réactionnel vers 20°C et on agite ce milieu pendant 48 heures. Après évaporation à l'évaporateur rotatif on obtient une huile brune (27 mg) qui, après trituration, donne le 1-(1-oxoindan-2-yl)imidazole-2,4-dicarboxamide sous forme de solide beige fondant au-dessus de 260°C utilisé tel quel dans les synthèses ultérieures.

### EXEMPLE 3

A une suspension de 580 mg de 8-amino-4,5-dihydro-4-oxo-10H-imidazo[1,2-a]indéno[1,2-e]pyrazine-2-carboxylate d'éthyle dans 10 ml de diméthylformamide on ajoute goutte à goutte sous agitation 0,44 ml d'isocyanate de méthyle et on maintient l'agitation pendant 12 heures à une température voisine de 20°C. Le mélange réactionnel est alors filtré, rincé avec du diméthylformamide et séché à 35°C sous vide (1 mmHg; 0,13 kPa). On obtient 320 mg de 8-(3-méthyluréido)-4,5-dihydro-4-oxo-10H-imidazo[1,2-a]indéno[1,2-e]pyrazine-2-carboxylate d'éthyle à l'état de dihydrate sous forme de solide beige fondant au-dessus de 260°C (Analyse % calculé C : 53,59, H : 5,25, N : 17,36, % trouvé C : 53,2, H : 4,7, N : 17,7).

Le 8-amino-4,5-dihydro-4-oxo-10H-imidazo[1,2-a]indéno[1,2-e]pyrazine-2-carboxylate d'éthyle peut être préparé de la façon suivante : un mélange de 1,1 g de 8-nitro-4,5-dihydro-4-oxo-,10H-imidazo[1,2-a]indéno[1,2-e]pyrazine-2-carboxylate d'éthyle et 40 ml de diméthylformamide est hydrogéné à une température voisine de 20°C sous une pression de 1,9 bar pendant 24 heures en présence de 0,14 g de charbon palladié à 10%. On filtre le catalyseur sous atmosphère inerte, écarte le filtrat, remet le charbon palladié en suspension dans du diméthylsulfoxyde, filtre et évapore ce nouveau filtrat à l'évaporateur rotatif. On obtient une huile noire (2 g) que l'on triture dans l'éthanol. Le solide obtenu est filtré, rincé avec de l'éthanol et séché pour donner 0,58 g de 8-amino-4,5-dihydro-4-oxo-10H-imidazo[1,2-a]indéno[1,2-e]pyrazine-2-carboxylate d'éthyle sous forme de solide beige fondant au-dessus de 260°C utilisé tel quel dans les synthèses ultérieures.

Le 8-nitro-4,5-dihydro-4-oxo-10H-imidazo[1,2-a]indéno[1,2-e]pyrazine-2-carboxylate d'éthyle peut être préparé de la manière suivante : à 11 ml d'acide sulfurique concentré refroidi à une température voisine de -5°C on ajoute peu à peu sous agitation 1 g de 4-oxo-5H,10H-imidazo[1,2-a]indéno[1,2-e]pyrazine-2-carboxylate d'éthyle en maintenant le mélange réactionnel à une température voisine de 0°C. Après 30 minutes d'agitation à cette température, on ajoute progressivement 0,34 g de nitrate de potassium et on poursuit l'agitation 30 minutes à 0°C. On laisse alors remonter la température vers 20°C et on maintient l'agitation pendant 12 heures. Le mélange réactionnel est versé sur de la glace et le précipité formé est isolé par filtration, rincé à l'eau distillée, puis avec de l'oxyde d'isopropyle pour donner 1,1 g de 8-nitro-4,5-dihydro-4-oxo-10H-imidazo[1,2-a]indéno[1,2-e]pyrazine-2-carboxylate d'éthyle sous forme de solide jaune fondant vers 180 °C utilisé tel quel dans les synthèses ultérieures.

### EXEMPLE 4

On agite pendant 12 heures à une température voisine de 20°C un mélange de 200 mg de 8-(3-méthyluréido)-4,5-dihydro-4-oxo-10H-imidazo[1,2-a]indéno[1,2-e]pyrazine-2-carboxylate d'éthyle, 20 ml de dioxane, 0,5 ml d'eau et 1,6 ml de soude 1N. Après concentration du mélange réactionnel à l'évaporateur rotatif on acidifie à pH 1 avec de l'acide chlorhydrique 1N. Le précipité obtenu est filtré, rincé à l'eau distillée, puis avec de l'oxyde d'isopropyle pour fournir 120 mg d'acide 8-(3-méthyluréido)-4,5-dihydro-4-oxo-10H-imidazo[1,2-a]indéno[1,2-e]pyrazine-2-carboxylique sous forme de solide marron fondant au-dessus de 260°C (Spectre de R.M.N.: ¹ H (200 MHz, (CD₃)₂SO d₆ , δ en ppm): 2,65 (s, 3H : CH₃); 3,90 (s, 2H : CH₂); 7,30 (d, J=9Hz, 1H : CH arom); 7,70 (d, J=9Hz, 1H : CH arom); 7,80 (s, 1H : CH arom); 8,40 (s, 1H : CH arom)).

### EXEMPLE 5

On opère comme à l'exemple 1 mais à partir de 5 g de 1-(6-chloro-1-oxoindan-2-yl)imidazole-2,4-dicarboxylate de diéthyle, 118 g d'acétate d'ammonium et 190 ml d'acide acétique. Après lavage du précipité à l'eau, puis avec de la méthyléthylcétone, on triture le solide obtenu d'abord dans l'acétonitrile, puis dans le méthanol et enfin dans l'acétone pour obtenir 2,7 g de 7-chloro-4,5-dihydro-4-oxo-10H-imidazo[1,2-a]indéno[1,2-e]pyrazine-2-carboxylate d'éthyle sous forme de solide brun fondant au-dessus de 260°C (Analyse % calculé C : 58,28, H : 3,67, Cl : 10,75, N : 12,74, % trouvé C : 58,6, H : 3,4, Cl : 10,4, N : 13,1).

Le 1-(6-chloro-1-oxoindan-2-yl)imidazole-2,4-dicarboxylate de diéthyle peut être préparé de la façon suivante: on opère comme à l'exemple 1 mais à partir de 17 g d'imidazole-2,4-dicarboxylate de diéthyle, 3,5 g d'hydrure de sodium à 60%, 500 ml de diméthylformamide et 19,6 g de 2-bromo-6-chloroindanone. On obtient 14 g de 1-(6-chloro-1-oxoindan-2-yl)imidazole-2,4-dicarboxylate de diéthyle utilisé tel quel dans les synthèses ultérieures.

La 2-bromo-6-chloroindanone peut être préparée comme décrit par P. STREHLKE, G.A. HOYER et E. SCHROEDER dans Arch. Pharm., 308(2), 94 (1975).

### EXEMPLE 6

On opère comme à l'exemple 2 mais à partir de 2,7 g de 2-carbamoyl-1-(1-oxoindan-2-yl)imidazole-4-carboxylate de tert-butyle et de 35 ml d'acide acétique. On obtient, après séchage à 50°C sous vide (1 mmHg; 0,13 kPa), 1,75 g d'acide 4,5-dihydro-4-oxo-10H-imidazo[1,2-a]indéno[1,2-e]pyrazine-2-carboxylique à l'état d'hydrate sous forme de de solide blanc fondant au-dessus de 260°C (Analyse % calculé C : 58,95, H : 3,89, N : 14,73, % trouvé C : 58,8, H : 4,0, N : 14,5).

Le 2-carbamoyl-1-(1-oxoindan-2-yl)imidazole-4-carboxylate de tert-butyle peut être préparé de la manière suivante: on opère comme à l'exemple 2 mais à partir de 3,7 g de 2-éthoxycarbonyl-1-(1-oxoindan-2-yl)imidazole-4-carboxylate de tert-butyle, 45 ml de méthanol et d'ammoniac gazeux. Au bout de 20 heures de contact le précipité apparu dans le milieu réactionnel est isolé par filtration, rincé avec du méthanol et séché sous vide (15 mmHg; 2 kPa). On obtient 2,7 g de 2-carbamoyl-1-(1-oxoindan-2-yl)imidazole-4-carboxylate de tert-butyle sous forme de solide blanc fondant à 240°C.

Le 2-éthoxycarbonyl-1-(1-oxoindan-2-yl)imidazole-4-carboxylate de tert-butyle peut être préparé comme à l'exemple 1 mais à partir de 5,5 g de 2-éthoxycarbonyl-imidazole-4-carboxylate de tert-butyle, 1g d'hydrure de sodium à 60%, 140 ml de diméthylformamide et 4,8 g de 2-bromoindanone. Le produit brut (9 g) est chromatographié sur colonne de silice en éluant avec un mélange de cyclohexane et d'acétate d'éthyle (50-50 en volumes). On obtient 4,1 g de 2-éthoxycarbonyl-1-(1-oxoindan-2-yl)imidazole-4-carboxylate de tert-butyle sous forme de meringue beige utilisée telle quelle dans les synthèses ultérieures.

Le 2-éthoxycarbonyl-imidazole-4-carboxylate de tert-butyle peut être préparé selon la méthode suivante: on opère comme à l'exemple 1 mais à partir de 15 g d'α-amino-oximinoacétate d'éthyle, 500 ml de xylène, 19,1 g de triéthylamine et 14,3 ml de propiolate de tert-butyle. Le produit brut (19 g) est chromatographié sur colonne de silice en éluant avec un mélange de cyclohexane et d'acétate d'éthyle (50-50 en volumes) pour fournir 5 g de 2-éthoxycarbonyl-imidazole-4-carboxylate de tert-butyle utilisé tel quel dans les synthèses ultérieures.

### EXEMPLE 7

Une suspension de 1,4 g de chlorhydrate de l'acide 10-amino-4,5-dihydro-4-oxo-10H-imidazo[1,2-a]indéno[1,2-e]pyrazin-2-carboxylique, 0,57 ml de 2,5-diméthoxytétrahydrofuranne et 0,36 g d'acétate de sodium dans 20 ml d'acide acétique est chauffée au reflux pendant 1 heure et 30 minutes. Après refroidissement à une température voisine de 20°C, l'insoluble est filtré, lavé à l'eau, à l'acétone puis séché pour conduire à 0,65 g de chlorhydrate et d'hydrate de l'acide 10-(1-pyrrolyle)-4,5-dihydro-4-oxo-10H-imidazo[1,2-a]indéno[1,2-e]pyrazin-2-carboxylique sous forme de poudre blanche dont le point de fusion est supérieur à 260°C (Analyse C21H15ClN4O4; 0,2 HCl; % calculé C : 55,89; H : 3,91; N : 14,48; O : 16,55; % trouvé C : 55,9; H : 3,8; N : 13,9; O : 16,4); Spectre ¹H dans DMSO, T=300K, δ en ppm (200 MHz) : 6,12 (2H, s, =CH), 6,62 (1H, s, CH), 6,93 (2H, s, =CH), entre 7,30 et 7,60 (4H, m, CH arom.), 7,94 (1H, d, J=7Hz, CH arom.), 12,75 (1H, s large, NH)).

### EXEMPLE 8

A une solution constituée de 0,77 g de l'acide 10-hydroxyimino-4,5-dihydro-4-oxo-imidazo[1,2-a]indéno[1,2-e]pyrazin-2-carboxylique, 0,46 g d'acétate d'ammonium, 50 ml d'ammoniaque à 28% et 50 ml d'éthanol absolu, on ajoute par petites portions 0,76 g de zinc en poudre. Le mélange réactionnel est porté au reflux pendant 5 heures puis refroidi à une température voisine de 20°C et traité goutte à goutte avec 40 ml d'acide chlorhydrique 6N. Le précipité formé est filtré, lavé à l'eau et séché sous pression réduite (1 mmHg; 0,13 kPa). 0,3 g de chlorhydrate et d'hydrate de l'acide 10-amino-4,5-dihydro-4-oxo-10H-imidazo[1,2-a]indéno[1,2-e]pyrazin-2-carboxylique sont ainsi obtenus sous forme de poudre blanche dont le point de fusion est supérieur à 260°C (Analyse C14H13ClN4O4; 0,55 HCI; 0,83 H2O; % calculé C : 49,94; H : 3,89; N : 16,64; O : 19,01; % trouvé C : 50,0; H : 3,9; N : 15,5; O : 19,0); Spectre ¹H dans DMSO, T=300K, δ en ppm (200 MHz): 5,42 (1H, s, CH), 7,45 (1H, t, J=7Hz, CH arom.),7,52 (1H, t, J=7Hz, CH arom.), 7,86 (1H, d, J=7Hz, CH arom.), 7,92 (1H, d, J=7Hz, CH arom.), 8,88 (1H, s, CH)).

### EXEMPLE 9

A 1 g de 4,5-dihydro-4-oxo-10H-imidazo[1,2-a]indéno[1,2-e]pyrazin-2-carboxylate d'éthyle en solution dans 20 ml de diméthylsulfoxyde anhydre, on ajoute 0,34 g d'hydrure de sodium en maintenant la température du milieu réactionnel inférieure à 20°C. On ajoute ensuite 0,44 ml de nitrite d'isoamyle et on poursuit l'agitation 18 heures à la même température. Le mélange réactionnel est alors versé sur de l'eau glacée et la solution acidifiée à l'aide de 15 ml d'acide acétique. Le précipité formé est filtré, lavé à l'eau, à l'acétone puis au méthanol et enfin séché sous pression réduite (1 mm Hg; 0,13 kPa) pour conduire à 0,25 g d'acide 10-hydroxyimino-4,5-dihydro-4-oxo-imidazo[1,2-a]indéno[1,2-e]pyrazin-2-carboxylique sous forme de poudre brune dont le point de fusion est supérieur à 260°C (Spectre ¹H dans DMSO, T=300K, δ en ppm (200 MHz), mélange des isomères SYN et ANTI :
Isomère majoritaire : entre 7,30 et 7,50 (2H, m, CH arom.), 7,62 (1H, s, CH arom.), 7,82 (1H, d, J=7Hz, CH arom.), 8,00 (1H, s, CH arom.), 8,20 (1H, d, J=7Hz, CH arom.), 12,7 (1H, s , NH), 13,0 (1H, s , OH),
Isomère minoritaire : entre 7,30 et 7,50 (2H, m, CH arom.), 7,51 (1H, s, CH arom.), 7,70 (1H, d, J=7Hz, CH arom.), 7,88 (1H, d, J=7Hz, CH arom.), 8,58 (1H, s, CH arom.), 12,7 (1H, s, NH), 13,2 (1H, s, OH)).

### EXEMPLE 10

0,58 g de 7-chloro-4,5-dihydro-4-oxo-10H-imidazo[1,2-a]indéno[1,2-e]pyrazin-2-carboxylate d'éthyle en suspension dans 10 ml d'acide chlorhydrique 6N sont portés au reflux pendant 24 heures. Après refroidissement à une température voisine de 20°C, l'insoluble est filtré, lavé à l'eau et séché sous pression réduite (1 mm Hg; 0,13 kPa) à 45°C. On obtient ainsi 0,38 g d'hydrate de l'acide 7-chloro-4,5-dihydro-4-oxo-10H-imidazo[1,2-a]indéno[1,2-e]pyrazin-2-carboxylique sous forme de poudre beige dont le point de fusion est supérieur à 260°C (Analyse C14H10ClN3O4; % calculé C : 52,60; H : 3,15; Cl : 11,09; N : 13,14; % trouvé C : 52,9; H : 3,3; Cl : 10,8; N : 12,9); Spectre ¹H dans DMSO, T=300K, δ en ppm (300 MHz): 4,02 (2H, s, CH₂), 7,37 (1H, d, J=7Hz, CH arom.), 7,60 (1H, d, J=7Hz, CH arom.), 7,92 (1H, s, CH arom.), 8,52 (1H, s, CH arom.), 12,45 (1H, s, NH)).

### EXEMPLE 11

Une suspension de 0,7 g de 8-amino-4,5-dihydro-4-oxo-10H-imidazo[1,2-a]indéno[1,2-e]pyrazin-2-carboxylate d'éthyle dans 14 ml d'acide chlorhydrique 6N est chauffée au reflux pendant 24 heures. Après refroidissement à une température voisine de 20°C, l'insoluble est filtré, lavé à l'eau puis avec de l'éther isopropylique pour conduire à 0,38 g de chlorhydrate et d'hydrate de l'acide 8-amino-4,5-dihydro-4-oxo-10H-imidazo[1,2-a]indéno[1,2-e]pyrazin-2-carboxylique sous forme de poudre brune dont le point de fusion est supérieur à 260°C (Spectre ¹H dans DMSO, T=300K, δ en ppm (300 MHz): 4,08 (2H, s, CH₂), 7,42 (1H, d, J=7Hz, CH arom.), 7,60 (1H, s, CH arom.), 7,97 (1H, d, J=7Hz, CH arom.), 8,51 (1H, s, CH arom.), 12,5 (1H, s, NH)).

### EXEMPLE 12

A une suspension de 0,6 g d'acide 5-(2-éthoxycarbonyl-10-méthyl-4,5-dihydro-4-oxo-10H-imidazo[1,2-a]indéno[1,2-e]pyrazin-10-yl)valérique dans 30 ml de dioxanne, on ajoute 4,3 ml de soude 1N et on poursuit l'agitation 15 heures à une température voisine de 20°C. Le milieu réactionnel est alors acidifié à l'aide d'acide chlorydrique 1N et la phase organique extraite par du dichlorométhane, séchée sur sulfate de magnésium, filtrée et concentrée à sec sous pression réduite à l'évaporateur rotatif. On obtient 0,11 g d'hydrate de l'acide 5- (2-carboxy-10-méthyl-4, 5-dihydro-4-oxo-10H-imidazo[1,2-a]indéno[1,2-e]pyrazin-10-yl)valérique sous forme d'un solide beige se décomposant à 230°C (Analyse C20H21N306; 0,55H2O; % calculé C 60,14; H : 5,30; N : 10,52; % trouvé C 60,3; H : 5,3; N : 9,4); Spectre ¹H dans DMSO, T=300K, δ en ppm (200 MHz): 1,25 (2H, t, J=6Hz, CH₂), 1,57 (3H, s, CH₃), 1,98 (2H, t, J=6Hz, COCH₂), entre 2,05 et 2,55 (4H, m, (CH₂)₂), 7,40 (2H, m, CH arom.), 7,36 (2H, t, J=7Hz, CH arom.), 7,44 (1H, d, J=7Hz, CH arom.), 7,50 (2H, d, J=7Hz, CH arom.), 7,58 (1H, d, J=7Hz, CH arom.), 7,88 (1H, d, J=7Hz, CH arom.), 8,60 (1H, s, CH), 12,5 (1H, s, NH)).

L'acide 5-(2-éthoxycarbonyl-10-méthyl-4,5-dihydro-4-oxo-10H-imidazo[1,2-a]indéno[1,2-e]pyrazin-10-yl)valérique peut être obtenu de la façon suivante: à une suspension maintenue à 20°C et sous azote de 1 g de 10-méthyl-4,5-dihydro-4-oxo-10H-imidazo[1,2-a]indéno[1,2-e]pyrazin-2-carboxylate d'éthyle dans 40 ml de diméthylformamide anhydre, on ajoute progressivement 0,71 g d'hydrure de sodium à 60%. Après arrêt du dégagement gazeux (environ 30 minutes), on ajoute goutte à goutte 0,45 ml de triméthylchlorosilane et on poursuit l'agitation 30 minutes à 20°C. 0,57 ml d'ester éthylique de l'acide 5-bromovalérique sont ensuite ajoutés goutte à goutte au milieu réactionnel et la réaction poursuivie 15 heures à la même température. Après addition de 25 ml d'eau distillée, le milieu réactionnel est acidifié à l'aide d'acide chlorhydrique 1N. Le précipité formé est filtré et lavé avec de l'éther isopropylique pour conduire à 0,84 g de produit attendu sous forme de solide ocre utilisé sans purification supplémentaire dans les étapes ultérieures.

Le 10-méthyl-4,5-dihydro-4-oxo-10H-imidazo[1,2-a]indéno[1,2-e]pyrazin-2-carboxylate d'éthyle peut être préparé selon la procédure suivante : une suspension de 8 g de 10-diméthylaminométhylène-4,5-dihydro-4-oxo-imidazo[1,2-a]indéno[1,2-e]pyrazin-2-carboxylate d'éthyle dans 200 ml de diméthylformamide en présence de 0,65 g de palladium sur charbon à 10% est hydrogénée à pression atmosphérique et à température ambiante pendant 15 heures. Le milieu réactionnel est filtré sur célite et l'insoluble lavé avec du diméthylformamide. Le filtrat est concentré à sec à l'évaporateur rotatif pour conduire à 7,6 g de produit attendu sous forme de solide ocre dont le point de fusion est supérieur à 260°C (Spectre ¹H dans DMSO, T=300K, δ en ppm (300 MHz): 1,36 (3H, t, J=6Hz, CH₃), 1,54 (3H, d, J=6Hz, CH₃), 4,19 (1H, q, J=6Hz, CH), 4,37 (2H, q, J=6Hz, OCH₂), entre 7,30 et 7,45 (2H, m, CH arom.), 7,62 (1H, d, J=7Hz, CH arom.), 7,88 (1H, d, J=7Hz, CH arom.), 8,58 (1H, s, CH), 12,5 (1H, s, NH)).

Le 10-diméthylaminométhylène-4,5-dihydro-4-oxo-imidazo[1,2-a]indéno[1,2-e]pyrazin-2-carboxylate d'éthyle peut être obtenu de la manière suivante : à une suspension de 9 g de 4,5-dihydro-4-oxo-10H-imidazo[1,2-a]indéno[1,2-e]pyrazine-2-carboxylate d'éthyle dans 100 ml de diméthylformamide anhydre, on ajoute, goutte à goutte à une température voisine de 20°C, 30 ml de tert-butoxy-bis(diméthylamino)méthane. Après 5 heures d'agitation à la même température, le milieu réactionnel est filtré et l'insoluble lavé au méthanol et à l'acétone. On obtient ainsi 5,7 g de produit attendu sous forme de solide jaune dont le point de fusion est supérieur à 260°C (Spectre ¹H dans DMSO, T=300K, δ en ppm (200 MHz): 1,38 (3H, t, J=6Hz, CH₃), 3,40 (6H, s, NCH₃), 4,40 (2H, q, J=6Hz, OCH₂), 7,19 (1H, t, J=7Hz, CH arom.), 7,28 (1H, t, J=7Hz, CH arom.), 7,45 (1H, d, J=7Hz, CH arom.), 7,95 (1H, d, J=7Hz, CH arom.), 8,21 (1H, s, NCH), 8,97 (1H, s, CH arom.), 12,3 (1H, s, NH)).

### EXEMPLE 13

A une suspension de 0,2 g de 4,5-dihydro-4-oxo-10H-imidazo[1,2-a]indéno[1,2-e]pyrazine-2-carboxylate d'éthyle dans 15 ml de dioxanne, on ajoute 5 ml de soude 1N et on agite fortement le mélange réactionnel tout en faisant buller de l'air comprimé pendant 15 heures. Après addition de 10 ml d'eau distillée, le milieu est acidifié à l'aide d'acide chlorhydrique concentré. Le précipité formé est filtré, lavé à l'eau et séché pour conduire à 0,13 g d'acide 4,5-dihydro-4,10-dioxo-imidazo[1,2-a]indéno[1,2-e]pyrazine-2-carboxylique sous forme de poudre brune dont le point de fusion est supérieur à 260°C (Spectre ¹H dans DMSO, T=300K, δ en ppm (200 MHz): entre 7,35 et 7,65 (3H, m, CH arom.), 7,75 (1H, d, J=7Hz, CH arom.), 8,38 (1H, s, CH arom.), 12,5 (1H, s, NH)).

### EXEMPLE 14

Une solution de 1,26 g d'acide (E)-4,5-dihydro-4-oxo-10-(3-cyanobenzylidène)-imidazo[1,2-a]indéno[1,2-e]pyrazine-2-carboxylique dans 15 ml d'acide sulfurique à 60% est chauffée au reflux pendant 3 heures et 30 minutes. Après refroidissement à une température voisine de 20°C, le milieu réactionnel est coulé sur de l'eau glacée et le précipité formé filtré, puis repris dans la soude 1N, l'insoluble écarté et le filtrat acidifié à l'aide d'acide chlorhydrique concentré. Le précipité est repris dans un mélange acétone-eau (50/50 en volumes), chauffé au reflux, filtré à chaud et séché. On obtient ainsi 0,27 g de sulfate de l'acide (E)-4,5-dihydro-4-oxo-10-(3-carboxybenzylidène)-imidazo[1,2-a]indéno[1,2-e]pyrazine-2-carboxylique sous forme de poudre jaune dont le point de fusion est supérieur à 260°C (Spectre ¹H dans DMSO + CD₃CO₂D, T=300K, δ en ppm (200 MHz): entre 7,50 et 7,75 (3H, m, CH arom.), 7,78 (1H, d, J=7Hz, CH arom.), 7,84 (1H, s, CH ethyl.), 7,88 (1H, d, J=7Hz, CH arom.), 7,94 (1H, d, J=7Hz, CH arom.), 8,13 (1H, s, CH arom.), 8,23 (1H, d, J=7Hz, CH arom.), 9,16 (1H, s, CH arom.)).

L'acide (E)-4,5-dihydro-4-oxo-10-(3-cyanobenzylidène)-imidazo[1,2-a]indéno[1,2-e]pyrazine-2-carboxylique peut être préparé de la manière suivante : à une solution constituée de 2 g de 4,5-dihydro-4-oxo-10H-imidazo[1,2-a]indéno[1,2-e]pyrazine-2-carboxylate d'éthyle et de 0,9 g de 3-cyanobenzaldéhyde dans 30 ml de diméthylsulfoxyde, on ajoute 0,5 g d'hydrure de sodium en évitant que la température dépasse 20°C. La réaction est poursuivie 15 heures à une température voisine de 20°C. 30 ml d'eau distillée puis 10 ml d'acide acétique sont alors ajoutés au milieu réactionnel.Le précipité formé est filtré puis repris et agité 1 heure dans un mélange de 50 ml de méthanol et de 5 ml d'éther chlorhydrique 2,5N. L'insoluble est filtré puis recristallisé dans un mélange diméthylformamide-eau distillée (50/50 en volumes). On obtient ainsi 1,26 g de produit attendu sous forme de poudre verte dont le point de fusion est supérieur à 260°C (Spectre ¹H dans DMSO, T=300K, δ en ppm (250 MHz): 7,2 (1H, s, CH ethyl.), entre 7,2 et 8,3 (7H, m larges, CH arom.), 9,1 (1H, s, CH arom.), 12,8 (1H, s, NH), 13,0 (1H, s large, OH)).

### EXEMPLE15

A une suspension constituée de 0,5g de chlorhydrate de l'acide 10-amino-4,5-dihydro-4-oxo-10H-imidazo[1,2-a]indéno[1,2-e]pyrazine-2-carboxylique et de 0,26 g d'acétate de sodium dans 10 ml d'acide acétique chauffés à 50°C, on ajoute 0,4 g d'anhydride succinique et on poursuit la réaction 5 heures à 50°C. Après refroidissement à une tempéarature voisine de 20°C, le milieu réactionnel est filtré et l'insoluble lavé à l'eau distillée et séché sous pression réduite (1 mm Hg; 0,13 kPa) à 50°C. On obtient 0,39 g d'hydrate de l'acide 10-(3-carboxypropionylamino)-4,5-dihydro-4-oxo-10H-imidazo[1,2-a]indéno[1,2-e]pyrazine-2-carboxylique sous forme de poudre beige dont le point de fusion est supérieur à 260°C (Analyse C18H16N407; 0,66H2O; % calculé C : 54,01; H : 4,03; N : 13,99; % trouvé C : 54,0; H : 3,8; N : 13,9; Spectre ¹H dans DMSO, T=300K, δ en ppm (300 MHz): 2,55 (4H, m, (CH₂)₂), 6,15 (1H, d, J=6Hz, CH), 7,41 (1H, d, J=7Hz, CH arom.), 7,49 (1H, t, J=7Hz, CH arom.), 7,51 (1H, t, J=7Hz, CH arom.), 7,88 (1H, d, J=7Hz, CH arom.), 8,07 (1H, s, CH), 8,64 (1H, d, J=6Hz, NH), 12,65 (1H, s, NH)).

### EXEMPLE 16

A une solution contenant 0,5 g de chlorhydrate de l'acide 10-amino-4,5-dihydro-4-oxo-10H-imidazo[1,2-a]indéno[1,2-e]pyrazine-2-carboxylique et 0,66 ml de triéthylamine dans 8 ml de diméthylformamide anhydre, on ajoute, goutte à goutte à une température inférieure à 20°C, une solution de 0,5 ml d'isocyanate de phényle dans 2 ml de diméthylformamide anhydre. La réaction est poursuivie 18 heures à une température voisine de 20°C, puis l'insoluble est filtré et séché pour conduire à 0,16 g de dihydrate du sel de triéthylamine de l'acide 10-(3-phényluréido)-4,5-dihydro-4-oxo-10H-imidazo[1,2-a]indéno[1,2-e]pyrazine-2-carboxylique sous forme de solide crême dont le point de fusion est supérieur à 260°C (Analyse C27H34N6O6; 0,46H2O; % calculé C : 60,22; H : 6,36; N : 15,6; % trouvé C : 60,2; H : 6,2; N : 15,8; Spectre ¹H dans DMSO, T=300K, δ en ppm (300 MHz): 6,04 (1H, d, J=6Hz, CH), 6,96 (1H, t, J=7Hz, CH arom.), 7,26 (2H, t, J=7Hz, CH arom.), 7,36 (2H, t, J=7Hz, CH arom.), 7,44 (1H, d, J=7Hz, CH arom.), 7,50 (2H, d, J=7Hz, CH arom.), 7,58 (1H, d, J=7Hz, CH arom.), 7,86 (1H, d, J=6Hz, NH), 8,10 (1H, s, CH), 9,15 (1H, s, NH)).

### EXEMPLE 17

Sous couverture d'argon à une solution agitée de 1,63 g de 4,5-dihydro-4-oxo-10H-imidazo[1,2-a]indéno[1,2-e]pyrazine-2-carboxylate d'éthyle et de 0,63 g de 1-méthylimidazole-2-carboxaldéhyde dans 20 ml de diméthylsulfoxyde, on ajoute par portions, à une température voisine de 19°C, 0,41 g d'hydrure de sodium à 80%. L'agitation est maintenue pendant 5 heures à environ 20°C. Le mélange réactionnel est refroidi à une température voisine de 12°C et traité avec 30 ml d'eau distillée en réglant l'addition de l'eau pour maintenir la température du milieu réactionnel entre 22 et 25°C. On ajoute pour finir 6 ml d'acide acétique. La suspension est alors filtrée et le solide ainsi isolé est lavé à l'eau, puis au méthanol et essoré à fond pendant la nuit. On obtient l'acide 10-[(1-méthylimidazol-2-yl)méthylène]-4,5-dihydro-4-oxo-imidazo[1,2-a]indéno[1,2-e]pyrazine-2-carboxylique brut sous forme de solide pâteux noir dont on a purifié le tiers par mise en suspension dans 15 ml de méthanol et addition de 10 ml d'acide chlorhydrique 0,45 N dans l'éther éthylique. L'agitation est maintenue pendant 90 minutes, puis la suspension est filtrée et le solide est lavé avec un mélange de méthanol et d'éther éthylique (10 ml). Après séchage à 60°C sous vide (1 mm Hg; 0,13 kPa), on obtient 0,42 g d'acide 10-[(1-méthylimidazol-2-yl)méthylène]-4,5-dihydro-4-oxo-imidazo[1,2-a]indéno[1,2-e]pyrazine-2-carboxylique, chlorhydrate sous forme de solide rouge brique fondant au-dessus de 260°C (Analyse % calculé C: 57,65, H: 3,57, CI: 8,96, N: 17,69, O: 12,13, % trouvé C: 58,1, H: 3,4,N: 17,1).

Le 1-méthylimidazole-2-carboxaldéhyde peut être préparé selon le procédé décrit par P. FOURNARI et coll., Bull. Soc. Chim. Fr., (6), 2438 (1968).

### EXEMPLE 18

On opère comme à l'exemple 17 mais à partir de 1,48 g de 4,5-dihydro-4-oxo-10H-imidazo[1,2-a]indéno[1,2-e]pyrazine-2-carboxylate d'éthyle, 0,55 g de 1-méthylimidazole-5-carboxaldéhyde, 20 ml de diméthylsulfoxyde et 0,37 g d'hydrure de sodium à 80%. L'acide 10-[(1-méthylimidazol-5-yl) méthylène]-4,5-dihydro-4-oxo-im idazo[1,2-a]indéno[1,2-e]pyrazine-2-carboxylique brut se présente comme un solide noir dont un tiers est purifié de la même façon que dans l'exemple 17. On obtient 0,54 g d'acide 10-[(1-méthylimidazol-5-yl)méthylène]-4,5-dihydro-4-oxo-imidazo[1,2-a]indéno[1,2-e]pyrazine-2-carboxylique, chlorhydrate sous forme de solide vert foncé fondant au-dessus de 260°C (Analyse % calculé C: 57,66, H: 3,57, CI: 8,96, N: 17,69, O: 12,13, % trouvé C: 58,1, H: 3,0, N: 16,8).

Le 1-méthylimidazole-5-carboxaldéhyde peut être préparé selon le procédé décrit par R. KIRCHLECHNER et coll., Synthesis, 247, (1994).

### EXEMPLE 19

Un mélange d'acide 10-[(1-méthylimidazol-2-yl)méthylène]-4,5-dihydro-4-oxo-imidazo[1,2-a]indéno[1,2-e]pyrazine-2-carboxylique brut (le reste de l'acide brut décrit dans l'exemple 17), 50 ml d'eau distillée et 3 ml de soude N est hydrogéné à une température voisine de 20°C sous une pression de 1,75 bar pendant 5 heures en présence de 0,4 g de charbon palladié à 10%. On filtre le catalyseur sous atmosphère inerte et acidifie le filtrat avec 5 ml d'acide chlorhydrique N. Le précipité formé est filtré, lavé deux fois à l'eau distillée et séché à 60°C sous vide (1 mm Hg; 0,13 kPa). On obtient 0,42 g d'acide 10-[(1-méthylimidazol-2-yl)méthyl]-4,5-dihydro-4-oxo-10H-imidazo [1,2-a]indéno[1,2-e]pyrazine-2-carboxylique, chlorhydrate sous forme de solide marron clair fondant au-dessus de 260°C (Analyse % calculé C: 57,37, H: 4,05, CI: 8,91, N: 17,60, O: 12,07, % trouvé C: 56,9, H: 6,1, N: 18,1).

### EXEMPLE 20

On opère comme à l'exemple 19 mais à partir d'acide 10-[(1-méthylimidazol-5-yl)méthylène]-4,5-dihydro-4-oxo-imidazo[1,2-a]indéno[1,2-e]pyrazine-2-carboxylique brut (le reste de l'acide brut décrit dans l'exemple 18), 50 ml d'eau distillée, 3 ml de soude N et 0,4 g de charbon palladié à 10%. On obtient 0,45 g d'acide 10-[(1-méthylimidazol-5-yl)méthyl]-4,5-dihydro-4-oxo-10H-imidazo[1,2-a]indéno[1,2-e]pyrazine-2-carboxylique, chlorhydrate sous forme de solide marron fondant au-dessus de 260°C (Analyse % calculé C : 57,37, H : 4,05, Cl : 8,91, N : 17,60, O : 12,07, % trouvé C : 58,3, H : 4,8, N : 17,1).

### EXEMPLE 21

Sous couverture d'argon, à une solution agitée de 2 g d'acide 10-hydroxyimino-4,5-dihydro-4-oxo-imidazo[1,2-a]indéno[1,2-e]pyrazine-2-carboxylique dans 40 ml de diméthylsulfoxyde on ajoute par portions, à une température comprise entre 20 et 25°C, 1,1 g d'hydrure de sodium à 80%, puis en dix minutes une solution de 1,1 ml de 4-bromobutyrate d'éthyle dans 2 ml de diméthysulfoxyde et on poursuit l'agitation pendant la nuit. Au mélange réactionnnel on ajoute lentement 5 ml d'acide acétique. Le milieu réactionnel est alors versé sur 100 g de glace pilée, ajusté à pH 1 avec de l'acide chlorhydrique 6 N et additionné de 100 ml de méthanol. La suspension résultante est centrifugée et le solide ainsi isolé est lavé deux fois au méthanol et séché sous pression réduite. Le solide vert obtenu est cristallisé dans le diméthylformamide et, après séchage à 80°C sous vide (1 mm Hg; 0,13 kPa), on obtient 0,84 g d'acide 4-[10-(2-carboxy-4,5-dihydro-4-oxo-imidazo[1,2-a]indéno[1,2-e]pyrazinylidène)aminooxy]butyrique, mélange 85-15 des formes Z et E, sous forme de solide jaune fondant au-dessus de 260°C (Analyse % calculé C : 56,55, H : 3,69, N : 14,65, O : 25,11, % trouvé C : 56,6, H : 3,4, N : 15,1, O : 25,1).

### EXEMPLE 22

Une solution de 3,42 g de 1-(5-fluoro-1-oxoindan-2-yl)imidazole-2,4-dicarboxylate de diéthyle et de 7,2 g d'acétate d'ammonium dans 25 ml d'acide acétique est chauffée à l'ébullition pendant 2 heures puis refroidie à une température voisine de 20°C. Le solide apparu est séparé par filtration, lavé successivement avec 3 ml d'acide acétique, 6 fois avec 30 ml au total d'eau distillée et 2 fois avec 10 ml au total d'acétone puis séché à l'air. 0,52 g de produit obtenu (sur les 2,95 g obtenus au total) est maintenu en suspension pendant 5 minutes dans 5 ml d'éthanol bouillant, séparé par filtration, lavé 2 fois avec 6 ml au total d'éthanol bouillant et séché sous pression réduite (1 mm Hg; 0,13 kPa) à 60°C. On obtient ainsi 0,49 g de 8-fluoro-4-oxo-4,5-dihydro-10H-imidazo[1,2-a]indéno[1,2-e]pyrazine-2-carboxylate d'éthyle se décomposant sans fondre au-dessus de 260°C (Spectre ¹H dans DMSO-d6, T=300K, δ en ppm (250 MHz): 1,32 (3H, t, J=6Hz, CH₃), 3,98 (2H, s, CH₂), 4,10 (2H, q, J=6Hz, OCH₂), 7,20 (1H, t, J=7Hz, CH arom.), 7,45 (1H, d, J=7Hz, CH arom.), 7,80 (1H, dd, J=7 et 5Hz, CH arom.), 8,47 (1H, s, CH arom.), 12,4 (1H, s, NH)).

Le 1-(5-fluoro-1-oxoindan-2-yl)imidazole-2,4-dicarboxylate de diéthyle peut être préparé de la manière suivante : On ajoute 10,4 g de carbonate de potassium à une solution de 3,2 g d'imidazole-2,4-dicarboxylate de diéthyle dans 80 ml d'acétone et, sur la suspension maintenue à l'ébullition, on coule goutte à goutte pendant 10 minutes une solution de 4,8 g de 2-bromo-5-fluoroindanone dans 20 ml d'acétone. Le mélange est agité à l'ébullition pendant 2 heures et, après refroidissement, pendant 16 heures à une température voisine de 20°C puis concentré à sec sous pression réduite (10 mm Hg; 1,3 kPa) à 40°C. Le produit obtenu (18 g) est additionné de 60 ml d'eau distillée et de 150 ml de dichlorométhane et, après décantation, la solution organique est lavée 2 fois avec 120 ml au total d'eau distillée, séchée sur du sulfate de magnésium anhydre et concentrée à sec sous pression réduite (10 mm Hg; 1,3 kPa) à 40°C. Le produit obtenu (5,5 g) est chromatographié sur 320 g de gel de silice neutre (0,020-0,045) contenus dans une colonne de 4,8 cm de diamètre en éluant avec un mélange acétate d'éthyle-cyclohexane (70-30 en volumes) en recueillant des fractions de 120 ml. Les fractions 35 à 70 sont réunies et concentrées à sec sous pression réduite (10 mm Hg; 1,3 kPa) à 40°C. On obtient ainsi 3,48 g de 1-(5-fluoro-1-oxoindan-2-yl)imidazole-2,4-dicarboxylate de diéthyle fondant à 138°C.

### EXEMPLE 23

Une suspension agitée de 0,47 g de 8-fluoro-4-oxo-4,5-dihydro-10H-imidazo[1,2-a]indéno[1,2-e]pyrazine-2-carboxylate d'éthyle dans 10 ml d'acide chlorhydrique 6N est maintenue à l'ébullition pendant 16 heures puis refroidie à une température voisine de 20°C. L'insoluble est séparé par filtration, lavé 2 fois avec 10 ml au total d'eau distillée et avec 5 ml d'acétone puis séché sous pression réduite (1 mm Hg; 0,13 kPa) à 60°C. On obtient ainsi 0,17 g d'acide 8-fluoro-4-oxo-4,5-dihydro-10H-imidazo[1,2-a]indéno[1,2-e]pyrazine-2-carboxylique se décomposant sans fondre au-dessus de 260°C (Spectre ¹H dans DMSO-d6, T=300K, δ en ppm (300 MHz): 4,00 (2H, s, CH₂), 7,22 (1H, t, J=7Hz, CH arom.), 7,45 (1H, d, J=7Hz, CH arom.), 7,82 (1H, dd, J=7 et 5Hz, CH arom.), 8,47 (1H, s, CH arom.), 12,4 (1H, s, NH), 12,8 (1H, s, COOH)).

### EXEMPLE 24

Une solution de 0,5 g de 2,5-diméthoxy-2,5-dihydrofuranne dans 1 ml d'acide acétique est ajoutée goutte à goutte en 1 minute à 50°C à une suspension de 1 g de chlorhydrate de l'acide 10-amino-4-oxo-4,5-dihydro-10H-imidazo[1,2-a]indéno[1-2-e]pyrazine-2-carboxylique et de 0,57 g d'acétate de sodium dans 15 ml d'acide acétique. Le mélange est agité pendant 2 heures à 65°C et, après refroidissement, pendant 16 heures à une température voisine de 20°C puis concentré à sec sous pression réduite (10 mmHg; 1,3 kPa) à 60°C. Le produit obtenu (2,7 g) est agité en suspension dans 30 ml d'eau distillée, séparé par filtration, lavé 2 fois avec 20 ml au total d'eau distillée et avec 10 ml d'acétone puis séché à l'air. 0,48 g de produit obtenu (sur 0,68 g obtenu au total) est chromatographié sur 29 g de gel de silice neutre (0,020-0,045) contenus dans une colonne de 2,7 cm de diamètre en éluant sous pression avec un mélange chloroforme-méthanol-ammoniaque à 28% (12/6/1 en volumes). Les fractions contenant le produit attendu sont réunies et concentrées à sec sous pression réduite (10 mm Hg; 1,3 kPa) à 40°C. Le produit (0,23 g) est mis en suspension dans 5 ml d'éther isopropylique, séparé par filtration, lavé avec 5 ml d'éther isopropylique et séché sous pression réduite (1 mm Hg; 0,13 kPa) à 60°C. On obtient ainsi 0,19 g de 4-oxo-10-[1-(2-oxo-2,5-dihydropyrrolyl)]-4,5-dihydro-10H-imidazo[1,2-a]indéno[1,2-e]pyrazine-2-carboxylate d'ammonium se décomposant sans fondre au-dessus de 260°C (Spectre ¹H dans DMSO-d6, T=300K, δ en ppm (250 MHz) : 3,48 et 3,70 (1H chacun, d, J=16Hz, NCH₂), 6,33 (1H, d, J=4Hz, COCH), 6,39 (1H, s, NCH), entre 7,30 et 7,60 (4H, m, CH arom.), 7,60 (1H, s, CH arom.), 7,90 (1H, d, J=7Hz, CH arom.)).

Les médicaments selon l'invention sont constitués par un composé de formule (I) ou un sel d'un tel composé, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles pour le traitement et/ou la prévention des conditions qui requièrent l'administration d'un antagoniste du récepteur AMPA ou d'un antagoniste du récepteur NMDA. Ces composés sont notamment utiles pour traiter ou prévenir toutes les ischémies et en particulier l'ischémie cérébrale, les effets dus à une anoxie, l'évolution de maladies neurodégénératives, de la chorée d'HUNTINGTON, de la maladie d'ALZHEIMER, de la sclérose latérale amyotrophique, de l'atrophie olivo-pontocérébelleuse et de la maladie de PARKINSON, vis-à-vis des manifestations épileptogènes et/ou convulsives, pour le traitement des traumatismes cérébraux et spinaux, des traumatismes liés à le dégénérescence de l'oreille interne ou de la rétine, de l'anxiété, de la dépression, de la schizophrénie, du syndrome de TOURETTE, de l'encéphalopathie hépatique, en tant qu'analgésiques, antiinflammatoires, antianorexiques, antimigraineux, antiémétiques et pour traiter les empoisonnements par des neurotoxines ou d'autres substances agonistes du récepteur NMDA, ainsi que les troubles neurologiques associés aux maladies virales telles que le sida, la rage, la rougeole et le tétanos. Ces composés sont aussi utiles pour la prévention des symptomes d'abstinence aux drogues et à l'alcool et de l'inhibition de l'accoutumance et de la dépendance aux opiacés ainsi que pour le traitement des déficits liés à des anomalies mitochondriales telles que la myopathie mitochondriale, le syndrome de LEBER, l'encéphalopathies de WERNICKE, le syndrome de RETT, l'homocystéinémie, l'hyperprolinémie, l'hydroxybutirique-aminoacidurie, l'encéphalopathie saturnine (intoxication au plomb) et la déficience en sulfite oxydase.

Les doses dépendent de l'effet recherché, de la durée du traitement et de la voie d'administration utilisée; elles sont généralement comprises entre 10 mg et 100 mg par jour par voie orale pour un adulte avec des doses unitaires allant de 5 mg à 50 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Composé de formule (I) 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Composé de formule (I) 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante:
- Composé de formule (I) 10 mg
- Acide benzoïque 80 mg
- Alcool benzylique 0,06 ml
- Benzoate de sodium 80 mg
- Ethanol à 95 % 0,4 ml
- Hydroxyde de sodium 24 mg
- Propylène glycol 1,6 ml
- Eau q.s.p 4 ml

## Revendications

1. Composés de formule : 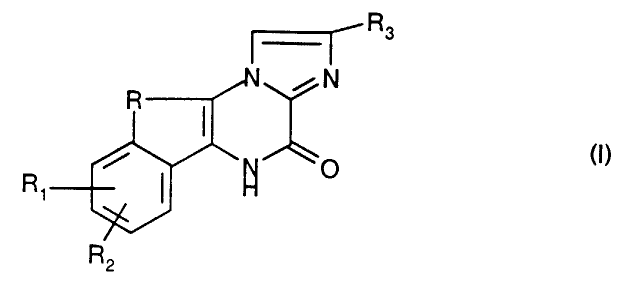 dans laquelle,
- R représente un radical N-alk, C(R₄)R₅, CH-R₆ ou C=R₇,
- R₁ et R₂, identiques ou différents, représentent des atomes d'hydrogène ou d'halogène ou des radicaux alkyle, alcoxy, amino, -N=CH-N(alk)alk', nitro, cyano, phényle, imidazolyle, SO₃H, hydroxy, polyfluoroalcoxy, carboxy, alcoxycarbonyle, -NH-CO-NR₁₁R₁₂, -N(alk)-CO-NR₁₁R₁₂, -N(alk-Ar)-CO-NR₁₁R₁₂, -NH-CS-NR₁₁R₁₂, -N(alk)-CS-NR₁₁R₁₂, -NH-CO-R₁₁, -NH-CS-R₂₄, -NH-C(=NR₂₇)-NR₁₀R₁₂, -N(alk)-C(=NR₂₇)-NR₁₀R₁₂, -CO-NR₁₀R₁₂, -NH-SO₂-NR₁₀R₁₂, -N(alk)-SO₂-NR₁₀R₁₂, -NH-SO₂-CF₃, -NH-SO₂-alk, -NR₁₀R₁₃, -S(O)ₘ-alk-Ar, -SO₂-NR₁₀R₁₂, 2-oxo-1-imidazolidinyle dont la position -3 est éventuellement substituée par un radical alkyle ou 2-oxo-1-perhydropyrimidinyle dont la position -3 est éventuellement substituée par un radical alkyle,
- R₃ représente un radical carboxy, alcoxycarbonyle ou carboxamido,
- R₄ représente un radical alkyle, -alk-Het ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀,
- R₅ représente un radical alkyle (1-11C en chaîne droite ou ramifiée), -alk-Het, -NR₈R₉, -NH-CHO, -NH-COOR₁₇, -NH-SO₂R₂₄, -COOR₁₀, -alk-COOR₁₀, -alk-CONR₁₀R₁₈, -alk-NR₁₀R₁₈, -alk-OH, -alk-CN, phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, -NH-CO-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, -NH-CO-Het, -NH-CO-alk-Het, -NH-CO-alk-COOR₁₀, -NH-CO-alk-NR₁₀R₁₈, -NH-CO-alk-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, pyrrol-1-yle éventuellement substitué par un radical -COOR₁₀, -NH-CO-NH-alk-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, -NH-CO-NH-Het, -NH-CO-NH-alk-Het, -NH-CO-NH-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, -NH-COalk, -NH-COcycloalkyle, -NH-CO-NH-alk ou -NH-CO-NH₂,
ou bien R₄ et R₅ forment ensemble avec l'atome de carbone auquel ils sont rattachés un radical cycloalkyle,
- R₆ représente un atome d'hydrogène ou un radical hydroxy, alkyle (1-11C en chaîne droite ou ramifiée), -alk-OH, -NR₁₄R₁₅, -alk-NR₁₄R₁₅, -alk-Het, -NH-CHO, -COOalk, -alk-COOR₁₀, -alk-CO-NR₁₀R₂₁, phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, -R₁₆-COOR₁₀, -CO-COOR₁₀, pyrrol-1-yle éventuellement substitué par un radical -COOR₁₀ ou 2-oxo-2,5-dihydropyrrol-1-yle,
- R₇ représente un atome d'oxygène ou un radical NOH, NO-alk-COOR₁₀, NO-alk, CHR₁₉, NR₁₀, C(COOR₁₀)R₂₀ ou C(CONR₁₀R₂₁)R₂₀,
- R₈ représente un atome d'hydrogène ou un radical alkyle, -alk-COOR₁₀, -alk-NR₁₀R₂₁, -alk-Het ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀,
- R₉ représente un atome d'hydrogène ou un radical alkyle,
- R₁₀ représente un atome d'hydrogène ou un radical alkyle,
- R₁₁ représente un atome d'hydrogène ou un radical alkyle (1-9C en chaîne droite ou ramifiée), -alk-COOR₁₀, -alk-Het, -alk-NR₁₂R₁₀, phénylalkyle dont le noyau phényle est éventuellement substitué par un ou plusieurs substituants choisis parmi les atomes d'halogène et les radicaux alkyle, alcoxy, nitro, amino, hydroxy, -alk-NH₂, carboxy, alcoxycarbonyle, cyano et -alk-COOR₁₀, phényle éventuellement substitué par un ou plusieurs substituants choisis parmi les atomes d'halogène et les radicaux alkyle, alcoxy, nitro, amino, hydroxy, -alk-NH₂, carboxy, alcoxycarbonyle, cyano et -alk-COOR₁₀ ou -Het,
- R₁₂ représente un atome d'hydrogène ou un radical alkyle,
- R₁₃ représente un radical alkyle, Het ou alcoxycarbonyle,
- R₁₄ et R₁₅, identiques ou différents représentent chacun un radical alkyle ou bien R₁₄ représente un atome d'hydrogène et R₁₅ représente un atome d'hydrogène ou un radical alkyle, -COR₂₂, -CSR₂₃ ou -SO₂R₂₄,
- R₁₆ représente une chaîne -CHOH- ou -CH(OH)-alk(1-5C)-,
- R₁₇ représente un radical alkyle ou phénylalkyle,
- R₁₈ représente un atome d'hydrogène ou un radical alkyle,
- R₁₉ représente un radical hydroxy, alkyle, -alk-Het, -NR₂₅R₂₆, -alk-COOR₁₀, -Het, phényle éventuellement substitué par un ou plusieurs substituants choisis parmi les atomes d'halogène et les radicaux alkyle, alcoxy, nitro, amino, hydroxy, -alk-NH₂, -COOR₁₀, cyano et -alk-COOR₁₀ ou phénylalkyle dont le noyau phényle est éventuellement substitué par un ou plusieurs substituants choisis parmi les atomes d'halogène et les radicaux alkyle, alcoxy, nitro, amino, hydroxy, -alk-NH₂, -COOR₁₀, cyano et -alk-COOR₁₀,
- R₂₀ représente un atome d'hydrogène ou un radical alkyle,
- R₂₁ représente un atome d'hydrogène ou un radical alkyle,
- R₂₂ représente un radical alkyle, cycloalkyle, -COOalk, -alk-COOR₁₀, phényle éventuellement substitué par un ou plusieurs substituants choisis parmi les atomes d'halogène et les radicaux alkyle, alcoxy, nitro, amino, hydroxy, -alk-NH₂, -COOR₁₀, cyano et -alk-COOR₁₀, phénylalkyle dont le noyau phényle est éventuellement substitué par un ou plusieurs substituants choisis parmi les atomes d'halogène et les radicaux alkyle, alcoxy, nitro, amino, hydroxy, -alk-NH₂, -COOR₁₀, cyano et -alk-COOR₁₀, -alk-NR₁₀R₁₂, -NH-Ar dont Ar est éventuellement substitué par un ou plusieurs substituants choisis parmi les atomes d'halogène et les radicaux alkyle, alcoxy, nitro, amino, hydroxy, -alk-NH₂, -COOR₁₀, cyano et -alk-COOR₁₀, -Het, -alk-Het, -OR₁₇, -NH-alk-Ar dont Ar est éventuellement substitué par un ou plusieurs substituants choisis parmi les atomes d'halogène et les radicaux alkyle, alcoxy, nitro, amino, hydroxy, -alk-NH₂, -COOR₁₀, cyano et -alk-COOR₁₀, -NH-alk-Het, -NH-alk, -NH₂ ou -NH-Het,
- R₂₃ représente un radical -NH-alk, -NH-Ar, -NH-Het ou -NH₂,
- R₂₄ représente un radical alkyle ou phényle,
- R₂₅ et R₂₆, identiques ou différents, représentent chacun un radical alkyle ou cycloalkyle,
- R₂₇ représente un atome d'hydrogène ou un radical alkyle,
- alk représente un radical alkyle ou alkylène,
- alk' représente un radical alkyle,
- m est égal à 0, 1 ou 2,
- Ar représente un radical phényle,
- Het représente un hétérocycle mono ou polycyclique saturé ou insaturé contenant 1 à 9 atomes de carbone et un ou plusieurs hétéroatomes (O, S, N) éventuellement substitué par un ou plusieurs radicaux alkyle, phényle ou phénylalkyle,
étant entendu que les radicaux et portions alkyle, alkylène et alcoxy contiennent 1 à 6 atomes de carbone et sont en chaîne droite ou ramifiée, les radicaux et portions acyle contiennent 2 à 4 atomes de carbone et les radicaux cycloalkyle contiennent 3 à 6 atomes de carbone,
ainsi que pour les composés pour lesquels R₇ représente un radical NO-alk, C(COOR₁₀)R₂₀ ou C(CONR₁₀R₂₁)R₂₀, CHR₁₉ leurs isomères (E et Z), pour les composés pour lesquels R représente un radical CH-R₆ et R₆ représente un radical -CO-COOR₁₀ leurs formes tautomères (E et Z), pour les composés pour lesquels R représente un radical C(R₄)R₅ ou CH-R₆ leurs énantiomères et diastéréoisomères et les sels de ces composés.

2. Composés de formule (I) selon la revendication 1 pour lesquels Het est choisi parmi les cycles pyrrolyle éventuellement substitué par un ou plusieurs radicaux alkyle, phényle ou phénylalkyle, pyridyle éventuellement substitué par un ou plusieurs radicaux alkyle, phényle ou phénylalkyle, pyrimidinyle éventuellement substitué par un ou plusieurs radicaux alkyle, phényle ou phénylalkyle, imidazolyle éventuellement substitué par un ou plusieurs radicaux alkyle, phényle ou phénylalkyle, thiazolyle éventuellement substitué par un ou plusieurs radicaux alkyle, phényle ou phénylalkyle, oxazolinyle éventuellement substitué par un ou plusieurs radicaux alkyle, phényle ou phénylalkyle, thiazolinyle éventuellement substitué par un ou plusieurs radicaux alkyle, phényle ou phénylalkyle, pyrazinyle éventuellement substitué par un ou plusieurs radicaux alkyle, phényle ou phénylalkyle, tétrazolyle éventuellement substitué par un ou plusieurs radicaux alkyle, phényle ou phénylalkyle ou triazolyle éventuellement substitué par un ou plusieurs radicaux alkyle, phényle ou phénylalkyle.

3. Procédé de préparation des composés de formule (I) selon la revendication 1 pour lesquels R représente un radical N-alk ou CH-R₆, R₆ représente un atome d'hydrogène et R₃ représente un radical alcoxycarbonyle à l'exception de tert-butoxycarbonyle caractérisé en ce que l'on cyclise en présence d'acétate d'ammonium un dérivé de formule : 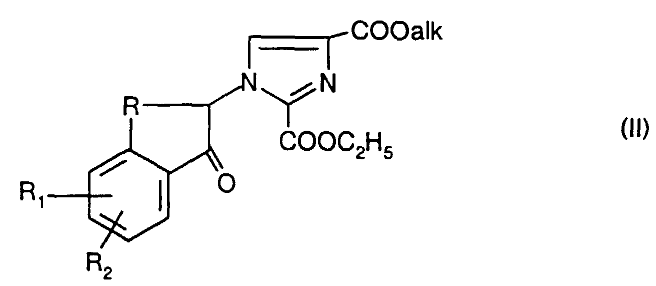 dans laquelle R représente un radical N-alk ou CH-R₆, R₆ représente un atome d'hydrogène, R₁ et R₂ ont les mêmes significations que dans la revendication 1 et -COOalk représente un radical alcoxycarbonyle sauf tert-butoxycarbonyle, isole le produit et le transforme éventuellement en sel.

4. Procédé de préparation des composés de formule (I) selon la revendication 1 pour lesquels R représente un radical N-alk ou CH-R₆, R₆ représente un atome d'hydrogène et R₃ représente un radical tert-butoxycarbonyle caractérisé en ce que l'on fait réagir de l'isobutène sur un composé de formule (I) correspondant pour lequel R représente un radical N-alk ou CH-R₆, R₆ représente un atome d'hydrogène et R₃ représente un radical carboxy, isole le produit et le transforme éventuellement en sel.

5. Procédé de préparation des composés de formule (I) selon la revendication 1 pour lesquels R représente un radical N-alk ou CH-R₆, R₆ représente un atome d'hydrogène et R₃ représente un radical carboxy caractérisé en ce que l'on cyclise en présence d'acétate d'ammonium un dérivé de formule : 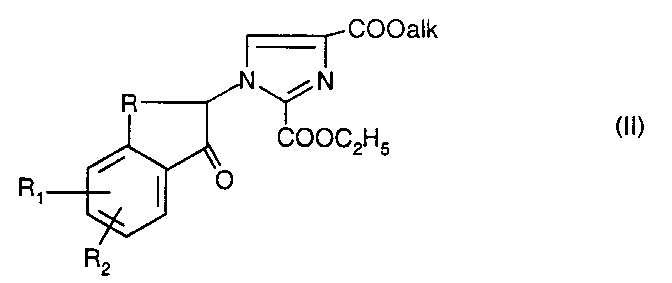 dans laquelle R représente un radical N-alk ou CH-R₆, R₆ représente un atome d'hydrogène et COOalk représente un radical tert-butoxycarbonyle, isole le produit et le transforme éventuellement en sel.

6. Procédé de préparation des composés de formule (I) selon la revendication 1 pour lesquels R représente un radical N-alk ou CH-R₆, R₆ représente un atome d'hydrogène et R₃ représente un radical carboxamido caractérisé en ce que l'on cyclise un dérivé de formule : 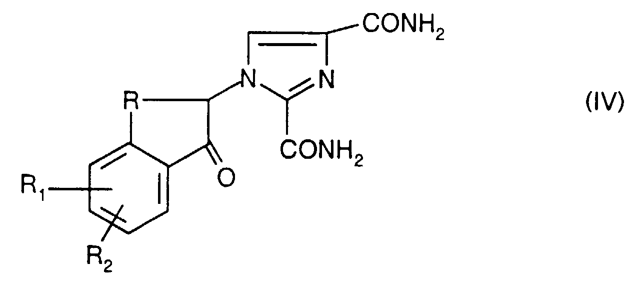 dans laquelle R représente un radical N-alk ou CH-R₆, R₆ représente un atome d'hydrogène, R₁ et R₂ ont les mêmes significations que dans la revendication 1, isole le produit et le transforme éventuellement en sel.

7. Procédé de préparation des composés de formule (I) selon la revendication 1 pour lesquels R représente un radical C=R₇ dans lequel R₇ représente un atome d'oxygène caractérisé en ce que l'on hydrolyse un composé de formule (I) correspondant pour lequel R représente un radical C=R₇ et R₇ représente un radical NOH, suivie pour les composés pour lesquels R₃ représente un radical alcoxycarbonyle d'une estérification de l'acide correspondant, isole le produit et le transforme éventuellement en sel.

8. Procédé de préparation des composés de formule (I) selon la revendication 1 pour lesquels R représente un radical C=R₇ et R₇ représente un radical NOH caractérisé en ce que l'on fait réagir un nitrite d'alkyle sur un composé de formule (I) correspondant pour lequel R représente un radical CH-R₆ et R₆ représente un atome d'hydrogène, isole le produit et le transforme éventuellement en sel.

9. Procédé de préparation des composés de formule (I) selon la revendication 1 pour lesquels R représente un radical C=R₇ et R₇ représente un radical NO-alk-COOR₁₀ ou NO-alk caractérisé en ce que l'on fait réagir un composé de formule (I) correspondant pour lequel R représente un radical C=R₇ et R₇ représente un radical NOH sur un halogènure Hal-Ra pour lequel Hal représente un atome d'halogène et Ra représente un radical alkyle ou -alk-COOR₁₀, alk et R₁₀ ayant les mêmes significations que dans la revendication 1, isole le produit et le transforme éventuellement en sel.

10. Procédé de préparation des composés de formule (I) selon la revendication 1 pour lesquels R représente un radical C=R₇ et R₇ représente un radical CH-R₁₉ dans lequel R₁₉ représente un radical hydroxy caractérisé en ce que l'on hydrolyse un composé de formule (I) correspondant pour lequel R₁₉ représente un radical -NR₂₅R₂₆, suivie pour les composés pour lesquels R₃ représente un radical alcoxycarbonyle d'une estérification de l'acide correspondant, isole le produit et le transforme éventuellement en sel.

11. Procédé de préparation des composés de formule (I) selon la revendication 1 pour lesquels R représente un radical C=R₇ et R₇ représente un radical CH-R₁₉ dans lequel R₁₉ représente un radical -NR₂₅R₂₆ caractérisé en ce que l'on fait réagir un composé de formule (I) correspondant pour lequel R représente un radical -CH-R₆ et R₆ représente un atome d'hydrogène sur un dérivé HC(Rb)(Rc)Rd dans laquelle soit Rb et Rd, identiques ou différents, représentent chacun un radical -NR₂₅R₂₆ dans lequel R₂₅ et R₂₆ ont les mêmes significations que dans la revendication 1 et Rc représente un radical alcoxy, soit Rb, Rc et Rd, identiques, représentent chacun un radical -NR₂₅R₂₆ dans lequel R₂₅ et R₂₆ ont les mêmes significations que dans la revendication 1, isole le produit et le transforme éventuellement en sel.

12. Procédé de préparation des composés de formule (I) selon la revendication 1 pour lesquels R représente un radical C=R₇, R₇ représente un radical CHR₁₉ et R₁₉ représente un radical alkyle, phényle éventuellement substitué, -alk-Het, phénylalkyle dont le noyau phényle est éventuellement substitué, -Het ou -alk-COOR₁₀ caractérisé en ce que l'on fait réagir un composé de formule (I) correspondant pour lequel R représente un radical CH-R₆ et R₆ représente un atome d'hydrogène sur un aldéhyde de formule OHC-Re dans laquelle Re représente un radical alkyle, phényle éventuellement substitué par un ou plusieurs substituants choisis parmi les atomes d'halogène et les radicaux alkyle, alcoxy, nitro, amino, hydroxy, -alk-NH₂, -COOR₁₀, cyano et -alk-COOR₁₀, -alk-Het, phénylalkyle dont le noyau phényle est éventuellement substitué par un ou plusieurs substituants choisis parmi les atomes d'halogène et les radicaux alkyle, alcoxy, nitro, amino, hydroxy, -alk-NH₂, -COOR₁₀, cyano et -alk-COOR₁₀, -Het ou -alk-COOR₁₀ dans lesquels alk, Het et R₁₀ ont les mêmes significations que dans la revendication 1, isole le produit et le transforme éventuellement en sel.

13. Procédé de préparation des composés de formule (I) selon la revendication 1 pour lesquels R représente un radical C=R₇, R₇ représente un radical NR₁₀ caractérisé en ce que l'on fait réagir le trifluoroacétate d'éthyle sur un dérivé de formule : 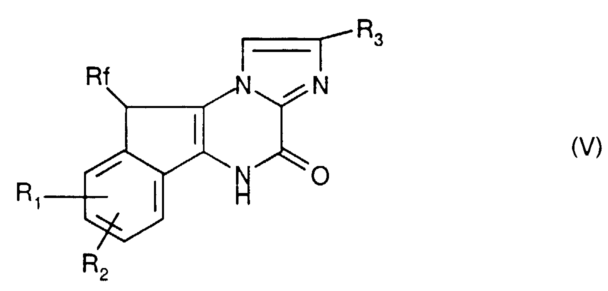 dans laquelle R₁, R₂ et R₃ ont les mêmes significations que dans la revendication 1 et Rf représente un radical -NH₂ ou -NH-alk, alk ayant les mêmes significations que dans la revendication 1, isole le produit et le transforme éventuellement en sel.

14. Procédé de préparation des composés de formule (I) selon la revendication 1 pour lesquels R représente un radical C=R₇ et R₇ représente un radical C(COOR₁₀)R₂₀ caractérisé en ce que l'on déshydrate un composé de formule : 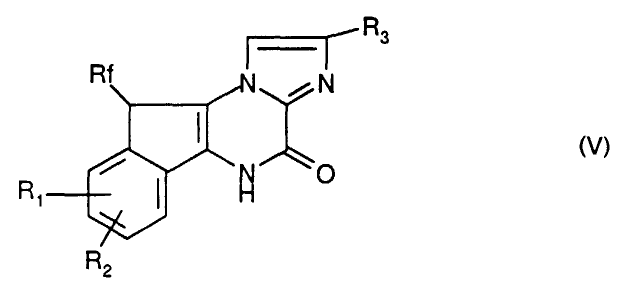 pour lequel R₁, R₂ et R₃ ont les mêmes significations que dans la revendication 1 et Rf représente un radical -C(R₂₀)(OH)-COOR₁₀ dans lequel R₂₀ et R₁₀ ont les mêmes significations que dans la revendication 1, isole le produit et le transforme éventuellement en sel.

15. Procédé de préparation des composés de formule (I) selon la revendication 1 pour lesquels R représente un radical C=R₇ et R₇ représente un radical C(CONR₁₀R₂₁)R₂₀ caractérisé en ce que l'on fait réagir un composé de formule (I) correspondant pour lequel R représente un radical C=R₇ et R₇ représente un radical C(COOR₁₀)R₂₀ sur une amine HNR₁₀R₂₁ dans laquelle R₁₀ et R₂₁ ont les mêmes significations que dans la revendication 1, isole le produit et le transforme éventuellement en sel.

16. Procédé de préparation des composés de formule (I) selon la revendication 1 pour lesquels R représente un radical C(R₄)R₅, R₄ représente un radical alkyle, -alk-Het ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀ et R₅ est identique à R₄ caractérisé en ce que l'on fait réagir un composé de formule (I) correspondant pour lequel R représente un radical CH-R₆ et R₆ représente un atome d'hydrogène sur un halogénure de formule Hal-Rg dans laquelle Rg représente un radical alkyle, -alk-Het ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, alk, Het et R₁₀ ayant les mêmes significations que dans la revendication 1, isole le produit et le transforme éventuellement en sel.

17. Procédé de préparation des composés de formule (I) selon la revendication 1 pour lesquels R représente un radical C(R₄)R₅, R₄ représente un radical alkyle, -alk-Het ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀ et R₅ représente un radical alkyle (1-11C en chaîne droite ou ramifiée), -alk-CN, -alk-Het, -alk-NR₁₀R₁₈, -alk-CO-NR₁₀R₁₈ ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, caractérisé en ce que l'on fait réagir un composé de formule (I) correspondant pour lequel R représente un radical CH-R₆ et R₆ représente un radical alkyle, -alk-Het ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀ sur un halogénure Hal-Rh pour lequel Rh représente un radical alkyle (1-11C en chaîne droite ou ramifiée), -alk-Het, -alk-CN, -alk-NR₁₀R₁₈, -alk-CO-NR₁₀R₁₈ ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, alk, Het, R₁₀ et R₁₈ ayant les mêmes significations que dans la revendication 1, isole le produit et le transforme éventuellement en sel.

18. Procédé de préparation des composés de formule (I) selon la revendication 1 pour lesquels R représente un radical C(R₄)R₅ et R₄ et R₅ forment avec l'atome de carbone auquel ils sont rattachés un radical cycloalkyle caractérisé en ce que l'on fait réagir un composé de formule (I) correspondant pour lequel R représente un radical CH-R₆ et R₆ représente un atome d'hydrogène sur un dérivé de formule Hal-alk-Hal dans laquelle Hal représente un atome d'halogène et alk représente un radical alkyle (2-5C), isole le produit et le transforme éventuellement en sel.

19. Procédé de préparation des composés de formule (I) selon la revendication 1 pour lesquels R représente un radical C(R₄)R₅ dans lequel R₅ représente un radical -NR₈R₉, R₈ et R₉ représentent des atomes d'hydrogène caractérisé en ce que l'on fait réagir un halogénure Hal-R₄ dans lequel Hal représente un atome d'halogène et R₄ a les mêmes significations que dans la revendication 1, sur un composé de formule (I) correspondant pour lequel R représente un radical CH-R₆, R₆ représente un radical -NR₁₄R₁₅, R₁₄ représente un atome d'hydrogène, R₁₅ représente un radical -COR₂₂ et R₂₂ représente un radical alkyle (1C), suivie d'une hydrolyse, isole le produit et le transforme éventuellement en sel.

20. Procédé de préparation des composés de formule (I) selon la revendication 1 pour lesquels R représente un radical C(R₄)R₅ dans lequel R₅ représente un radical -NR₈R₉, R₉ représente un atome d'hydrogène et R₈ représente un radical alkyle, -alk-COOR₁₀, -alk-NR₁₀R₂₁, -alk-Het ou phénylalkyle dont le noyau phényle est éventuellement substitué caractérisé en ce que l'on fait réagir un composé de formule (I) correspondant pour lequel R représente un radical C(R₄)R₅, R₅ représente un radical -NR₈R₉, R₈ et R₉ représentent des atomes d'hydrogène sur un halogènure Hal-R₈ dans lequel R₈ a les mêmes significations que précédemment, isole le produit et le transforme éventuellement en sel.

21. Procédé de préparation des composés de formule (I) selon la revendication 1 pour lesquels R représente un radical C(R₄)R₅ dans lequel R₅ représente un radical -NR₈R₉, R₉ représente un atome d'hydrogène ou un radical alkyle et R₈ représente un radical alkyle (2-6C) caractérisé en ce que l'on fait réagir un composé de formule (I) correspondant pour lequel R représente un radical C(R₄)R₅, R₅ représente un radical -NR₈R₉, R₉ représente un atome d'hydrogène ou un radical alkyle et R₈ représente un atome d'hydrogène sur un halogènure d'acyle (2-6C) puis réduction du produit obtenu, isole le produit et le transforme éventuellement en sel.

22. Procédé de préparation des composés de formule (I) selon la revendication 1 pour lesquels R représente un radical C(R₄)R₅ dans lequel R₅ représente un radical -NR₈R₉ et R₉ représente un radical alkyle caractérisé en ce que l'on fait réagir un composé de formule (I) correspondant pour lequel R représente un radical C(R₄)R₅, R₅ représente un radical -NR₈R₉, R₈ a les mêmes significations que dans la revendication 1 et R₉ représente un atome d'hydrogène sur un dérivé de formule Hal-alk dans laquelle Hal représente un atome d'halogène et alk représente un radical alkyle, isole le produit et le transforme éventuellement en sel.

23. Procédé de préparation des composés de formule (I) selon la revendication 1 pour lesquels R représente un radical C(R₄)R₅ dans lequel R₅ représente un radical -NR₈R₉, R₉ représente un atome d'hydrogène ou un radical alkyle et R₈ représente un radical -alk(2-6C)-NR₁₀R₂₁ caractérisé en ce que l'on réduit un dérivé de formule : 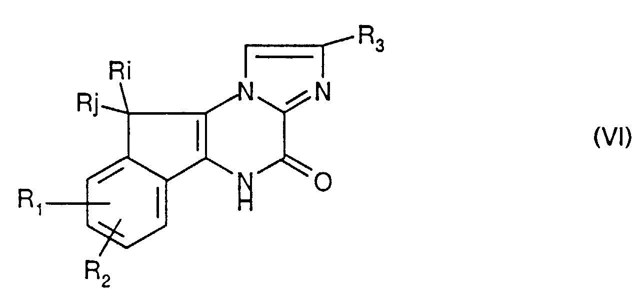 dans laquelle R₁, R₂ et R₃ ont les mêmes significations que dans la formule (I), Ri représente un radical alkyle, phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀ ou -alk-Het et Rj représente un radical -NH-CO-alk(1-5C)-NR₁₀R₂₁ ou -N(alk)-CO-alk(1-5C)-NR₁₀R₂₁ dans lesquels alk, Het, R₁₀ et R₂₁ ont les mêmes significations que dans la revendication 1, isole le produit et le transforme éventuellement en sel.

24. Procédé de préparation des composés de formule (I) selon la revendication 1 pour lesquels R représente un radical C(R₄)R₅ dans lequel R₅ représente un radical -COOR₁₀ et R₁₀ représente un atome d'hydrogène caractérisé en ce que l'on hydrolyse un composé de formule (I) correspondant pour lequel R représente un radical C(R₄)R₅ dans lequel R₅ représente un radical -COOR₁₀ et R₁₀ représente un radical alkyle, isole le produit et le transforme éventuellement en sel.

25. Procédé de préparation des composés de formule (I) selon la revendication 1 pour lesquels R représente un radical C(R₄)R₅ dans lequel R₅ représente un radical -COOR₁₀ et R₁₀ représente un radical alkyle caractérisé en ce que l'on fait réagir un composé de formule (I) correspondant pour lequel R représente un radical CH-R₆ et R₆ représente un radical alkyle, -alk-Het ou phénylalkyle éventuellement substitué sur un halogènure de formule Hal-COOR₁₀ dans lequel R₁₀ représente un radical alkyle et Hal représente un atome d'halogène, isole le produit et le transforme éventuellement en sel.

26. Procédé de préparation des composés de formule (I) selon la revendication 1 pour lesquels R représente un radical C(R₄)R₅ dans lequel R₅ représente un radical -alk-COOR₁₀ caractérisé en ce que l'on fait réagir un composé de formule (I) correspondant pour lequel R représente un radical CH-R₆, R₆ représente un radical -alk-COOR₁₀ et R₁₀ a les mêmes significations que dans la revendication 1 sur un halogènure Hal-R₄ dans lequel R₄ a les mêmes significations que dans la revendication 1, isole le produit et le transforme éventuellement en sel.

27. Procédé de préparation des composés de formule (I) selon la revendication 1 pour lesquels R représente un radical C(R₄)R₅ dans lequel R₅ représente un radical -alk-CN dans lequel alk contient 1 atome de carbone caractérisé en ce que l'on fait réagir du cyanure de sodium sur un dérivé de formule : 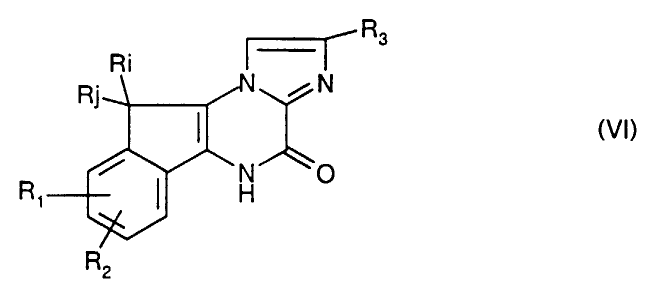 pour lequel R₁, R₂ et R₃ ont les mêmes significations que dans la revendication 1, Ri représente un radical -CH₂OTs dans lequel Ts représente un reste tosylate et Rj représente un radical alkyle, -alk-Het ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, alk, Het et R₁₀ ayant les mêmes significations que dans la revendication 1, isole le produit et le transforme éventuellement en sel.

28. Procédé de préparation des composés de formule (I) selon la revendication 1 pour lesquels R représente un radical C(R₄)R₅ dans lequel R₅ représente un radical -alk(2-6C)OH caractérisé en ce que l'on fait réagir du (COCl)₂ sur un composé de formule (I) correspondant pour lequel R représente un radical C(R₄)R₅ dans lequel R₅ représente un radical -alk-COOR₁₀ et R₁₀ représente un atome d'hydrogène suivie d'une réduction, isole le produit et le transforme éventuellement en sel.

29. Procédé de préparation des composés de formule (I) selon la revendication 1 pour lesquels R représente un radical C(R₄)R₅ dans lequel R₅ représente un radical -alk(1C)OH caractérisé en ce que l'on fait réagir du chlorure de triméthylsilane sur un dérivé de formule : 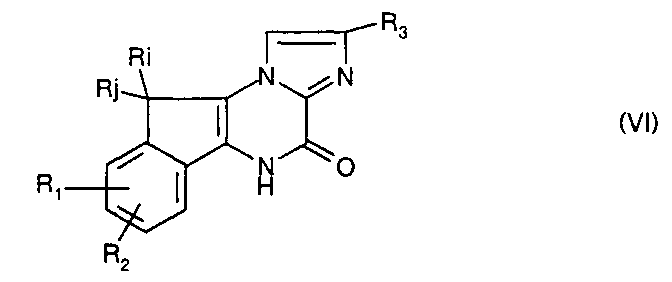 pour lequel R₁, R₂ et R₃ ont les mêmes significations que dans la revendication 1, Ri représente un atome d'hydrogène et Rj représente un radical alkyle, -alk-Het ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, alk, Het et R₁₀ ayant les mêmes significations que dans la revendication 1, puis du trioxane, isole le produit et le transforme éventuellement en sel.

30. Procédé de préparation des composés de formule (I) selon la revendication 1 pour lesquels R représente un radical C(R₄)R₅ dans lequel R₅ représente un radical -NH-CHO caractérisé en ce que l'on fait réagir un dérivé de formule : 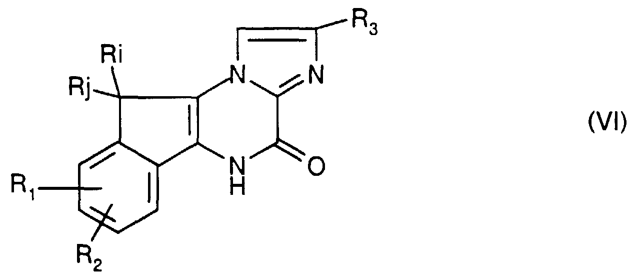 dans laquelle R₁, R₂ et R₃ ont les mêmes significations que dans la revendication 1, Ri représente un radical alkyle, -alk-Het ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, et Rj représente un radical amino, alk, Het et R₁₀ ayant les mêmes significations que dans la revendication 1 sur CH₃COOCHO, isole le produit et le transforme éventuellement en sel.

31. Procédé de préparation des composés de formule (I) selon la revendication 1 pour lesquels R représente un radical C(R₄)R₅ dans lequel R₅ représente un radical -NH-COOR₁₇, -NH-CO-Het, -NH-CO-alk-COOR₁₀, -NH-CO-alk-NR₁₀R₁₈, -NH-CO-Ar dont Ar est éventuellement substitué, -NH-CO-alk-Ar dont Ar est éventuellement substitué, -NH-SO₂-R₂₄, -NH-CO-alk-Het, -NH-CO-alk ou -NH-CO-cycloalkyle caractérisé en ce que l'on fait réagir un composé de formule (I) correspondant pour lequel R représente un radical C(R₄)R₅, R₅ représente un radical -NR₈R₉, R₈ et R₉ représentent des atomes d'hydrogène sur un dérivé Hal-Rk dans lequel Hal représente un atome d'halogène et Rk représente un radical -COOR₁₇, -CO-Het, -CO-alk-COOR₁₀, -CO-alk-NR₁₀R₁₈, -CO-alk-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, -SO₂-R₂₄, -CO-alk-Het, -CO-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, -CO-alk ou -CO-cycloalkyle, R₇, R₁₀, R₁₇, R₁₈, R₂₄, Het, Ar et alk ayant les mêmes significations que dans la revendication 1, isole le produit et le transforme éventuellement en sel.

32. Procédé de préparation des composés de formule (I) selon la revendication 1 pour lesquels R représente un radical C(R₄)R₅ dans lequel R₅ représente un radical -NH-CO-Het, -NH-CO-alk-COOR₁₀, -NH-CO-alk-NR₁₀R₁₈, -NH-CO-Ar dont Ar est éventuellement substitué, -NH-CO-alk-Ar dont Ar est éventuellement substitué, -NH-CO-alk-Het, -NH-CO-alk ou -NH-CO-cycloalkyle caractérisé en ce que l'on fait réagir un composé de formule (I) correspondant pour lequel R représente un radical C(R₄)R₅, R₅ représente un radical -NR₈R₉, R₈ et R₉ représentent des atomes d'hydrogène sur un dérivé HO-RI dans lequel RI représente un radical -CO-Het, -CO-alk-COOR₁₀, -CO-alk-NR₁₀R₁₈, -CO-alk-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, -CO-alk-Het, -CO-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, -CO-alk ou -CO-cycloalkyle, R₁₀, R₁₈, Het, Ar et alk ayant les mêmes significations que dans la revendication 1, isole le produit et le transforme éventuellement en sel.

33. Procédé de préparation des composés de formule (I) selon la revendication 1 pour lesquels R représente un radical C(R₄)R₅ dans lequel R₅ représente un radical pyrrol-1-yle éventuellement substitué par un radical -COOR₁₀ caractérisé en ce que l'on fait réagir un dérivé de formule : 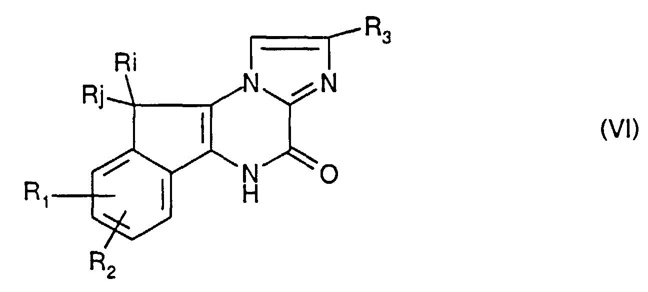 pour lequel R₁, R₂ et R₃ ont les mêmes significations que dans la revendication 1, Ri représente un radical alkyle, -alk-Het ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, alk, Het et R₁₀ ayant les mêmes significations que dans la formule (I) et Rj représente un radical amino, sur un dérivé de formule : 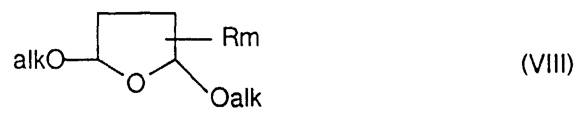 dans laquelle Rm représente un atome d'hydrogène ou un radical -COOR₁₀, alk et R₁₀ ont les mêmes significations que dans la revendication 1, isole le produit et le transforme éventuellement en sel.

34. Procédé de préparation des composés de formule (I) selon la revendication 1 pour lesquels R représente un radical C(R₄)R₅ dans lequel R₅ représente un radical -NH-CO-NH-alk-Ar dont Ar est éventuellement substitué, -NH-CO-NH-Het, -NH-CO-NH-alk-Het, -NH-CO-NH-Ar dont Ar est éventuellement substitué, -NH-CO-NH-alk ou -NH-CO-NH₂ caractérisé en ce que l'on fait réagir un composé de formule (I) correspondant pour lequel R représente un radical C(R₄)R₅, R₅ représente un radical -NR₈R₉, R₈ et R₉ représentent des atomes d'hydrogène sur un dérivé O=C=N-Rn dans lequel Rn représente un radical triméthylsilyle, alkyle, -Het, -alk-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, -alk-Het, phényle éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀ dans lesquels R₁₀, alk, Ar et Het ont les mêmes significations que dans la revendication 1, isole le produit et le transforme éventuellement en sel.

35. Procédé de préparation des composés de formule (I) selon la revendication 1 pour lesquels R représente un radical CH-R₆ dans lequel R₆ représente un radical hydroxy caractérisé en ce que l'on réduit un composé de formule (I) correspondant pour lequel R représente un radical C=R₇ et R₇ représente un atome d'oxygène, isole le produit et le transforme éventuellement en sel.

36. Procédé de préparation des composés de formule (I) selon la revendication 1 pour lesquels R représente un radical CH-R₆ dans lequel R₆ représente un radical alkyle (2-11C), phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀ ou -alk-Het caractérisé en ce que l'on hydrogène un dérivé de formule : 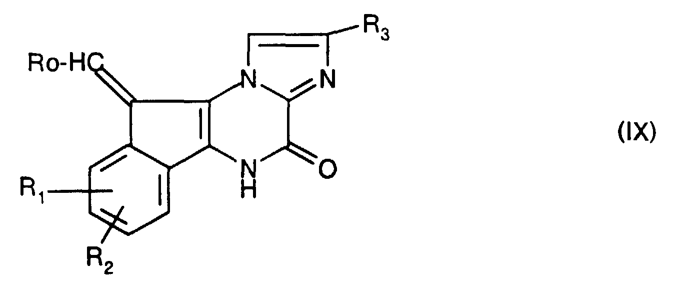 dans laquelle R₁, R₂ et R₃ ont les mêmes significations que dans la revendication 1, Ro représente un radical alkyle en chaîne droite ou ramifiée contenant 1 à 10 atomes de carbone, phényle, phénylalkyle(1-5C) dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, -Het, -alk(1-5C)-Het dans lesquels alk, Het et R₁₀ ont les mêmes significations que dans la revendication 1, isole le produit et le transforme éventuellement en sel.

37. Procédé de préparation des composés de formule (I) selon la revendication 1 pour lesquels R représente un radical CH-R₆ dans lequel R₆ représente un radical phénylalkyle éventuellement substitué ou -alk-Het caractérisé en ce que l'on fait réagir un composé de formule (I) correspondant pour lequel R représente un radical CH-R₆ et R₆ représente un atome d'hydrogène sur un halogénure de formule Hal-Rp dans laquelle Rp représente un radical phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀ ou -alk-Het dans lesquels alk, Het et R₁₀ ont les mêmes significations que dans la revndication 1, isole le produit et le transforme éventuellement en sel.

38. Procédé de préparation des composés de formule (I) selon la revendication 1 pour lesquels R représente un radical CH-R₆ dans lequel R₆ représente un radical alkyle (1C) caractérisé en ce que l'on réduit un composé de formule (I) correspondant pour lequel R représente un radical C=R₇, R₇ représente un radical CH-R₁₉ et R₁₉ représente un radical hydroxy ou -NR₂₅R₂₆, isole le produit et le transforme éventuellement en sel.

39. Procédé de préparation des composés de formule (I) selon la revendication 1 pour lesquels R représente un radical CHR₆ et R₆ représente un radical -alk(1C)-OH caractérisé en ce que l'on réduit un composé de formule (I) correspondant pour lequel R représente un radical C=R₇, R₇ représente un radical CH-R₁₉ et R₁₉ représente un radical hydroxy, isole le produit et le transforme éventuellement en sel.

40. Procédé de préparation des composés de formule (I) selon la revendication 1 pour lesquels R représente un radical CHR₆ et R₆ représente un radical -alk(2-6C)-OH caractérisé en ce que l'on réduit un dérivé de formule : 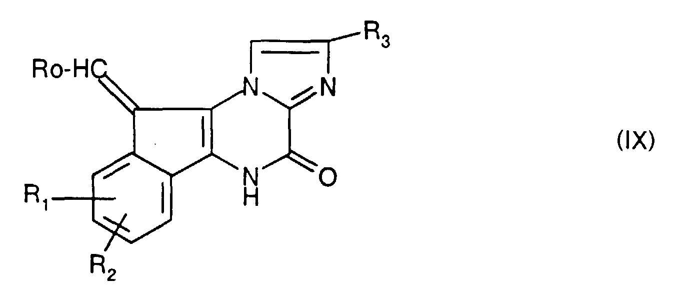 dans laquelle R₁, R₂ et R₃ ont les mêmes significations que dans la revendication 1 et Ro représente un radical -alk(1-5C)-O-CH₂-Ar, alk et Ar ayant les mêmes significations que dans la revendication 1, isole le produit et le transforme éventuellement en sel.

41. Procédé de préparation des composés de formule (I) selon la revendication 1 pour lesquels R représente un radical CH-R₆ dans lequel R₆ représente un radical -NR₁₄R₁₅, R₁₄ et R₁₅ représentent chacun un atome d'hydrogène caractérisé en ce que l'on hydrolyse un composé de formule (I) correspondant pour lequel R représente un radical CH-R₆ dans lequel R₆ représente un radical -NR₁₄R₁₅, R₁₄ représente un atome d'hydrogène, R₁₅ représente un radical -COR₂₂ et R₂₂ représente un radical alkyle, suivie pour les composés pour lesquels R₃ représente un radical alcoxycarbonyle d'une estérification de l'acide correspondant, isole le produit et le transforme éventuellement en sel.

42. Procédé de préparation des composés de formule (I) selon la revendication 1 pour lesquels R représente un radical CH-R₆ dans lequel R₆ représente un radical -NR₁₄R₁₅, R₁₄ représente un atome d'hydrogène, R₁₅ représente un radical -COR₂₂ et R₂₂ représente un radical alkyle caractérisé en ce que l'on fait réagir un agent réducteur sur un composé de formule (I) correspondant pour lequel R représente un radical C=R₇ et R₇ représente un radical NOH, puis un anhydride d'acide (alkCO)₂O dans lequel alk représente un radical alkyle, isole le produit et le transforme éventuellement en sel.

43. Procédé de préparation des composés de formule (I) selon la revendication 1 pour lesquels R représente un radical CH-R₆ et R₆ représente un radical -NR₁₄R₁₅ ou -alk-NR₁₄R₁₅, dans lesquels R₁₄ et R₁₅, identiques ou différents, représentent chacun un radical alkyle ou bien R₁₄ représente un atome d'hydrogène et R₁₅ représente un radical alkyle, -COR₂₂ ou -SO₂R₂₄, R₂₂ représente un radical alkyle, cycloalkyle, -COOalk, -alk-COOR₁₀, phényle éventuellement substitué par un ou plusieurs substituants choisis parmi les atomes d'halogène et les radicaux alkyle, alcoxy, nitro, amino, hydroxy, -alk-NH₂, -COOR₁₀, cyano et -alk-COOR₁₀, phénylalkyle dont le noyau phényle est éventuellement substitué par un ou plusieurs substituants choisis parmi les atomes d'halogène et les radicaux alkyle, alcoxy, nitro, amino, hydroxy, -alk-NH₂, -COOR₁₀, cyano et -alk-COOR₁₀, -OR₁₇, -Het, -alk-Het, -alk-NR₁₀R₁₂ et R₂₄ représente un radical alkyle ou phényle caractérisé en ce que l'on fait réagir un composé de formule (I) correspondant pour lequel R₁₄ et R₁₅ représentent chacun un atome d'hydrogène sur un halogènure de formule Hal-Rr dans laquelle Rr représente un radical alkyle, -COR₂₂ ou -SO₂R₂₄, R₂₂ représente un radical alkyle, cycloalkyle, -COOalk, -alk-COOR₁₀, phényle éventuellement substitué par un ou plusieurs substituants choisis parmi les atomes d'halogène et les radicaux alkyle, alcoxy, nitro, amino, hydroxy, -alk-NH₂, -COOR₁₀, cyano et -alk-COOR₁₀, phénylalkyle dont le noyau phényle est éventuellement substitué par un ou plusieurs substituants choisis parmi les atomes d'halogène et les radicaux alkyle, alcoxy, nitro, amino, hydroxy, -alk-NH₂, -COOR₁₀, cyano et -alk-COOR₁₀, -OR₁₇, -Het, -alk-Het, -alk-NR₁₀R₁₂ et R₂₄ représente un radical alkyle ou phényle, alk, Het, R₁₂, R₁₇ et R₁₀ ayant les mêmes significations que dans la revendication 1, isole le produit et le transforme éventuellement en sel.

44. Procédé de préparation des composés de formule (I) selon la revendication 1 pour lesquels R représente un radical CH-R₆ et R₆ représente un radical -NR₁₄R₁₅ ou -alk-NR₁₄R₁₅ dans lesquels R₁₄ représente un atome d'hydrogène, R₁₅ représente un radical -COR₂₂ ou -CSR₂₃, R₂₂ représente un radical -NH-alk, -NH₂, -NH-Ar dont Ar est éventuellement substitué, -NH-alk-Ar dont Ar est éventuellement substitué, -NH-alk-Het ou -NH-Het et R₂₃ représente un radical -NH-alk, -NH₂, -NH-Ar ou -NH-Het caractérisé en ce que l'on fait réagir un composé de formule (I) correspondant pour lequel R représente un radical CH-R₆, R₆ représente un radical -NR₁₄R₁₅, R₁₄ et R₁₅ représentent chacun un atome d'hydrogène sur un dérivé Rs-N=C=Rt pour lequel Rs représente un radical triméthylsilyle, alkyle, phényle éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, -alk-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, -alk-Het ou -Het dans lesquels Het, alk, R₁₀ et Ar ont les mêmes significations que dans la revendication 1 et Rt représente un atome d'oxygène ou de soufre, suivie éventuellement par une hydrolyse, isole le produit et le transforme éventuellement en sel.

45. Procédé de préparation des composés de formule (I) selon la revendication 1 pour lesquels R représente un radical CH-R₆ et R₆ représente un radical -NR₁₄R₁₅ ou -alk-NR₁₄R₁₅ dans lesquels R₁₄ représente un atome d'hydrogène, R₁₅ représente un radical -COR₂₂ et R₂₂ représente un radical -alk (1C)-NR₁₀R₁₂ dans lequel R₁₀ et R₁₂ sont des atomes d'hydrogène caractérisé en ce que l'on fait réagir un composé de formule (I) correspondant pour lequel R₁₄ et R₁₅ représentent chacun un atome d'hydrogène sur un acide HOOC-CH₂-NH-Ru dans lequel Ru représente un groupe protecteur de la fonction amine suivie d'une hydrolyse, isole le produit et le transforme éventuellement en sel.

46. Procédé de préparation des composés de formule (I) selon la revendication 1 pour lesquels R représente un radical CH-R₆, R₆ représente un radical -alk-NR₁₄R₁₅, R₁₄ et R₁₅ représentent chacun un atome d'hydrogène à l'exception de ceux pour lesquels R₃ représente un radical carboxamido caractérisé en ce que l'on fait réagir du brome et de la soude sur un composé de formule (I) correspondant pour lequel R représente un radical CH-R₆, R₆ représente un radical -alk-CONR₁₀R₂₁ et R₁₀ et R₂₁ représentent des atomes d'hydrogène, isole le produit et le transforme éventuellement en sel.

47. Procédé de préparation des composés de formule (I) selon la revendication 1 pour lesquels R représente un radical CH-R₆, R₆ représente un radical -alk-NR₁₄R₁₅, R₁₄ et R₁₅ représentent chacun un atome d'hydrogène et R₃ représente un radical carboxamido caractérisé en ce que l'on fait réagir de l'ammoniac sur un composé de formule (I) correspondant pour lequel R représente un radical CH-R₆, R₆ représente un radical -alk-NR₁₄R₁₅, R₁₄ et R₁₅ représentent chacun un atome d'hydrogène et R₃ représente un radical alcoxycarbonyle, isole le produit et le transforme éventuellement en sel.

48. Procédé de préparation des composés de formule (I) selon la revendication 1 pour lesquels R représente un radical CH-R₆, R₆ représente un radical -NR₁₄R₁₅ ou -alk-NR₁₄R₁₅ dans lesquels R₁₄ repprésente un atome d'hydrogène et R₁₅ représente un radical -COR₂₂, R₂₂ représente un radical alkyle, cycloalkyle, -alk-COOR₁₀, phényle éventuellement substitué, phénylalkyle dont le phényle est éventuellement substitué, Het, -alk-Het ou -alk-NR₁₀R₁₂ caractérisé en ce que l'on fait réagir un composé de formule (I) correspondant pour lequel R représente un radical CH-R₆, R₆ représente un radical -NR₁₄R₁₅, R₁₄ représente un atome d'hydrogène et R₁₅ représente un atome d'hydrogène sur un acide HOOC-R₂₂ dans lequel R₂₂ représente un radical alkyle, cycloalkyle, -Het, -alk-COOR₁₀, -alk-NR₁₀R₁₂, phénylalkyle dont le phényle est éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, -alk-Het, phényle éventuellement substitué par un ou plusieurs substituants choisis parmi les atomes d'halogène et les radicaux alkyle, alcoxy, nitro, amino, hydroxy, cyano, -alk-NH₂, -COOR₁₀ et -alk-COOR₁₀, alk, Het, R₁₀, R₁₂ ayant les mêmes significations que dans la revendication 1 ou l'anhydride correspondant, isole le produit et le transforme éventuellement en sel.

49. Procédé de préparation des composés de formule (I) selon la revendication 1 pour lesquels R représente un radical CH-R₆ et R₆ représente un radical -alk-COOR₁₀ caractérisé en ce que l'on hydrogène un dérivé de formule : 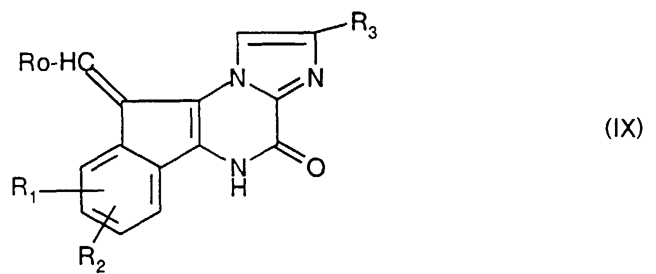 correspondant pour lequel R₁, R₂ et R₃ ont les mêmes significations que dans la revendication 1 et Ro représente un radical -alk(1-5C)-COOR₁₀ ou -COOR₁₀ dans lesquels alk a les mêmes significations que dans la revendication 1, R₁₀ représente un radical alkyle ou phénylalkyle, suivie éventuellement par une saponification de l'ester ainsi obtenu, isole le produit et le transforme éventuellement en sel.

50. Procédé de préparation des composés de formule (I) selon la revendication 1 pour lesquels R représente un radical CH-R₆, R₆ représente un radical -alk-CO-NR₁₀R₂₁ caractérisé en ce que l'on fait réagir un composé de formule (I) correspondant pour lequel R représente un radical CH-R₆ et R₆ représente un radical -alk-COOR₁₀, alk ayant les mêmes significations que dans la frevendication 1 et R₁₀ représente un atome d'hydrogène ou un radical alkyle sur une amine HNR₁₀R₂₁ dans laquelle R₁₀ et R₂₁ ont les mêmes significations que dans la revendication 1, isole le produit et le transforme éventuellement en sel.

51. Procédé de préparation des composés de formule (I) selon la revendication 1 pour lesquels R représente un radical CH-R₆, R₆ représente un radical -alk(C1)-CO-NR₁₀R₂₁ et R₁₀ et R₂₁ représentent des atomes d'hydrogène caractérisé en ce que l'on hydrogène un dérivé de formule : 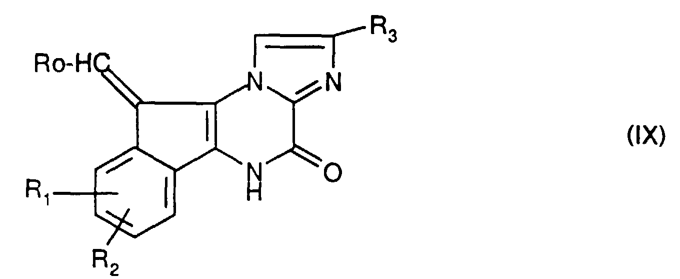 dans laquelle R₁, R₂ et R₃ ont les mêmes significations que dans la formule (I) et Ro représente un radical -CONH₂, isole le produit et le transforme éventuellement en sel.

52. Procédé de préparation des composés de formule (I) selon la revendication 1 pour lesquels R représente un radical CH-R₆ et R₆ représente un radical -R₁₆-COOR₁₀ caractérisé en ce que l'on fait réagir un composé de formule (I) correspondant pour lequel R représente un radical CH-R₆ et R₆ représente un atome d'hydrogène sur un dérivé de formule OHC-alk(0-5C)-COOR₁₀ dans laquelle alk et R₁₀ ont les mêmes significations que dans la revendication 1, isole le produit et le transforme éventuellement en sel.

53. Procédé de préparation des composés de formule (I) selon la revendication 1 pour lesquels R représente un radical CH-R₆ et R₆ représente un radical -NH-CHO caractérisé en ce que l'on fait réagir un composé de formule (I) correspondant pour lequel R représente un radical CH-R₆, R₆ représente un radical -NR₁₄R₁₅, R₁₄ et R₁₅ représentent des atomes d'hydrogène sur CH₃COOCHO, isole le produit et le transforme éventuellement en sel.

54. Procédé de préparation des composés de formule (I) selon la revendication 1 pour lesquels R représente un radical CH-R₆ et R₆ représente un radical -CO-COOR₁₀ caractérisé en ce que l'on oxyde un composé de formule (I) correspondant pour lequel R représente un radical CH-R₆, R₆ représente un radical -R₁₆-COOR₁₀ et R₁₆ représente un radical -CHOH- suivie éventuellement d'une estérification, isole le produit et le transforme éventuellement en sel.

55. Procédé de préparation des composés de formule (I) selon la revendication 1 pour lesquels R représente un radical CH-R₆, R₆ représente un radical pyrrol-1-yle éventuellement substitué par un radical -COOR₁₀ caractérisé en ce que l'on fait réagir un composé de formule (I) correspondant pour lequel R représente un radical CH-R₆ et R₆ représente un radical -NR₁₄R₁₅, R₁₄ et R₁₅ représentent des atomes d'hydrogène sur un dérivé de formule : 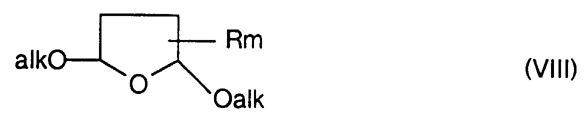 dans laquelle Rm représente un atome d'hydrogène ou un radical -COOR₁₀, R₁₀ ayant les mêmes significations que dans la revendication 1, isole le produit et le transforme éventuellement en sel.

56. Procédé de préparation des composés de formule (I) selon la revendication 1 pour lesquels R représente un radical CH-R₆ et R₆ représente un radical -COOalk caractérisé en ce que l'on fait réagir un acide minéral sur un dérivé de formule : 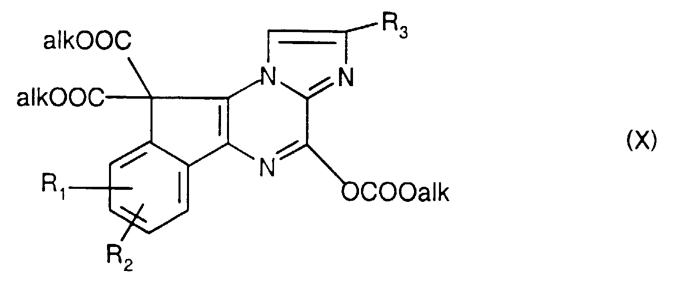 dans laquelle R₁, R₂ et R₃ ont les mêmes significations que dans la revendication 1 et alk représente un radical alkyle, isole le produit et le transforme éventuellement en sel.

57. Procédé de préparation des composés de formule (I) selon la revendication 1 pour lesquels R₃ représente un radical carboxy caractérisé en ce que l'on hydrolyse un composé de formule (I) correspondant pour lequel R₃ représente un radical alcoxycarbonyle, isole le produit et le transforme éventuellement en sel.

58. Procédé de préparation des composés de formule (I) selon la revendication 1 pour lesquels R₁ et/ou R₂ représentent un radical -NH-CO-NR₁₁R₁₂ dans lequel R₁₁ représente un atome d'hydrogène ou un radical alkyle (1-9C en chaîne droite ou ramifiée), -alk-COOR₁₀, -alk-Het, -alk-NR₁₂R₁₀, phénylalkyle dont le noyau phényle est éventuellement substitué par un ou plusieurs substituants choisis parmi les atomes d'halogène et les radicaux alkyle, alcoxy, nitro, amino, hydroxy, -alk-NH₂, carboxy, alcoxycarbonyle, cyano et -alk-COOR₁₀, phényle éventuellement substitué par un ou plusieurs substituants choisis parmi les atomes d'halogène et les radicaux alkyle, alcoxy, nitro, amino, hydroxy, -alk-NH₂, carboxy, alcoxycarbonyle, cyano et -alk-COOR₁₀ ou -Het et R₁₂ représente un atome d'hydrogène caractérisé en ce que l'on fait réagir un composé de formule (I) correspondant pour lequel R₁ et/ou R₂ représente un radical amino avec un isocyanate O=C=NR₁₁ dans lequel R₁₁ a les mêmes significations que précédemment ou représente un radical triméthylsilyle, isole le produit et le transforme éventuellement en sel.

59. Procédé de préparation des composés de formule (I) selon la revendication 1 pour lesquels R₁ et/ou R₂ représentent un radical amino caractérisé en ce que l'on réduit un composé de formule (I) correspondant pour lequel R₁ et/ou R₂ représentent un radical nitro, isole le produit et le transforme éventuellement en sel.

60. Procédé de préparation des composés de formule (I) selon la revendication 1 pour lesquels R représente un radical CH-R₆, R₆ représente un radical -NR₁₄R₁₅, R₁₄ et R₁₅ représentent des atomes d'hydrogène caractérisé en ce que l'on réduit en présence d'un agent réducteur un composé de formule (I) correspondant pour lequel R représente un radical C=R₇ et R₇ représente un radical NOH, isole le produit et le transforme éventuellement en sel.

61. Procédé de préparation des composés de formule (I) selon la revendication 1 pour lesquels R représente un radical C=R₇, R₇ représente un radical CH-R₁₉, R₁₉ représente un radical phényle substitué par un radical -COOR₁₀ et R₁₀ représente un atome d'hydrogène caractérisé en ce que l'on hydrolyse un composé de formule (I) correspondant pour lequel R représente un radical C=R₇, R₇ représente un radical CH-R₁₉, R₁₉ représente un radical phényle substitué par un radical cyano, isole le produit et le transforme éventuellement en sel.

62. Procédé de préparation des composés de formule (I) selon la revendication 1 pour lesquels R représente un radical CH-R₆ et R₆ représente un radical 2-oxo-2,5-dihydropyrrol-1-yle caractérisé en ce que l'on fait réagir un composé de formule (I) correspondant pour lequel R représente un radical CH-R₆, R₆ représente un radical -NR₁₄R₁₅, R₁₄ et R₁₅ représentent des atomes d'hydrogène sur le 2,5-diméthoxy-2,5-dihydrofuranne, isole le produit et le transforme éventuellement en sel.

63. Composés de formule (I) selon la revendication 1 pour lesquels R représente un radical CH-R₆, C=R₇ ou C(R₄)R₅, R₁ représente un atome d'hydrogène ou d'halogène ou un radical -NH-CO-NR₁₁R₁₂ dans lequel R₁₁ est un atome d'hydrogène et R₁₂ est un radical alkyle, R₂ représente un atome d'hydrogène, R₃ représente un radical carboxy, alcoxycarbonyle ou carboxamido, R₄ représente un radical alkyle, R₅ représente un radical -alk-COOR₁₀, R₆ représente un atome d'hydrogène ou un radical -alk-Het, 2-oxo-2,5-dihydropyrrol-1-yle, pyrrol-1-yle substitué par -COOR₁₀ ou -NR₁₄R₁₅, R₇ représente un radical CH-R₁₉, NO-alk-COOR₁₀ ou NOH, R₁₉ représente un radical Het ou phényle substitué par -COOR₁₀, soit R₁₄ et R₁₅ sont des atomes d'hydrogène soit R₁₄ est un atome d'hydrogène et R₁₅ est un radical -COR₂₂ dans lequel R₂₂ est un radical -alk-COOR₁₀ ou -NH-Ar et leurs sels.

64. Médicaments contenant en tant que principe actif au moins un composé de formule (I) selon l'une des revendications 1, 2 et 63 ou un sel d'un tel composé.

## Patentansprüche

1. Verbindungen der Formel (I) 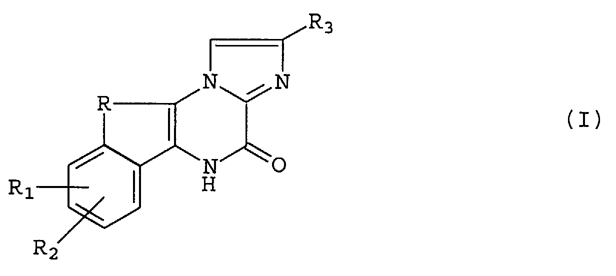 in der
- R einen Rest N-alk, C(R₄)R₅, CH-R₆ oder C=R₇ darstellt,
- R₁ und R₂, gleich oder verschieden, Wasserstoffatome oder Halogenatome oder Reste Alkyl, Alkoxy, Amino, -N=CH-N-(alk)alk', Nitro, Cyano, Phenyl, Imidazolyl, SO₃H, Hydroxy, Polyfluoralkoxy, Carboxy, Alkoxycarbonyl, -NH-CO-NR₁₁R₁₂, -N(alk)-CO-NR₁₁R₁₂, -N(alk-Ar)-CO-NR₁₁R₁₂, -NH-CS-NR₁₁R₁₂, -N(alk)-CS-NR₁₁R₁₂, -NH-CO-R₁₁, -NH-CS-R₂₄, -NH-C(=NR₂₇)-NR₁₀R₁₂, -N(alk)-C(=NR₂₇)-NR₁₀R₁₂, -CO-NR₁₀R₁₂, -NH-SO₂-NR₁₀R₁₂, -N(alk)-SO₂-NR₁₀R₁₂, -NH-SO₂-CF₃, -NH-SO₂-alk, -NR₁₀R₁₃, -S(O)ₘ-alk-Ar, -SO₂-NR₁₀R₁₂, 2-Oxo-1-imidazolidinyl, bei dem die Position -3 gegebenenfalls substituiert ist durch einen Rest Alkyl oder 2-Oxo-1-perhydropyrimidinyl, bei dem die Position -3 gegebenenfalls substituiert ist durch einen Rest Alkyl, bedeuten,
- R₃ ein Rest Carboxy, Alkoxycarbonyl oder Carboxamido ist,
- R₄ einen Rest Alkyl, -alk-Het oder Phenylalkyl darstellt, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀,
- R₅ einen Rest Alkyl (1 bis 11 Kohlenstoffatome in gerader oder verzweigter Kette), -alk-Het, -NR₈R₉, -NH-CHO, -NH-COOR₁₇, -NH-SO₂R₂₄, -COOR₁₀, -alk-COOR₁₀, -alk-CONR₁₀R₁₈, -alk-NR₁₀R₁₈, -alk-OH, -alk-CN, Phenylalkyl, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, -NH-CO-Ar, bei dem Ar gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, -NH-CO-Het, -NH-CO-alk-Het, -NH-CO-alk-COOR₁₀, NH-CO-alk-NR₁₀R₁₈, -NH-CO-alk-Ar, bei dem Ar gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, Pyrrol-1-yl, gegebenenfalls substituiert durch einen Rest -COOR₁₀, NH-CO-NH-alk-Ar, bei dem Ar gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, -NH-CO-NH-Het, NH-CO-NH-alk-Het, -NH-CO-NH-Ar, bei dem Ar gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, -NH-CO-alk, -NH-CO-cycloalkyl, -NH-CO-NH-alk oder -NH-CO-NH₂ bedeutet,
oder auch R₄ und R₅ zusammen mit dem Kohlenstoffatom, an das sie gebunden sind, einen Rest Cycloalkyl bilden,
- R₆ ein Wasserstoffatom oder einen Rest Hydroxy, Alkyl (1 bis 11 Kohlenstoffatome in gerader oder verzweigter Kette), -alk-OH, -NR₁₄R₁₅, -alk-NR₁₄R₁₅, -alk-Het, -NH-CHO, -COOalk, -alk-COOR₁₀, -alk-CO-NR₁₀R₂₁, Phenylalkyl, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, -R₁₆COOR₁₀, -CO-COOR₁₀, Pyrrol-1-yl, gegebenenfalls substituiert durch einen Rest -COOR₁₀ oder 2-Oxo-2,5-dihydropyrrol-1-yl darstellt,
- R₇ ein Sauerstoffatom oder einen Rest NOH, NO-alk-COOR₁₀, NO-alk, CHR₁₉, NR₁₀, C(COOR₁₀)R₂₀ oder C(CONR₁₀R₂₁)R₂₀ bedeutet,
- R₈ ein Wasserstoffatom oder einen Rest Alkyl, -alk-COOR₁₀, -alk-NR₁₀R₂₁, -alk-Het oder Phenylalkyl bedeutet, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und alk-COOR₁₀, darstellt,
- R₉ ein Wasserstoffatom oder ein Rest Alkyl ist,
- R₁₀ ein Wasserstoffatom oder ein Rest Alkyl ist,
- R₁₁ ein Wasserstoffatom oder einen Rest Alkyl (1 bis 9 Kohlenstoffatome in gerader oder verzweigter Kette), -alk-COOR₁₀, -alk-Het, -alk-NR₁₂R₁₀, Phenylalkyl, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, -alk-NH₂, Carboxy, Alkoxycarbonyl, Cyano und -alk-COOR₁₀, Phenyl, gegebenenfalls substituiert durch einen oder mehrere Substituenten, ausgewählt unter den Halogenatomen und den Resten Alkyl, Alkoxy, Nitro, Amino, Hydroxy, -alk-NH₂, Carboxy, Alkoxycarbonyl, Cyano und -alk-COOR₁₀, oder -Het bedeutet,
- R₁₂ ein Wasserstoffatom oder ein Rest Alkyl ist,
- R₁₃ ein Rest Alkyl, Het oder Alkoxycarbonyl ist,
- R₁₄ und R₁₅, gleich oder verschieden, jeweils einen Rest Alkyl darstellen oder auch R₁₄ ein Wasserstoffatom ist und R₁₅ ein Wasserstoffatom oder einen Rest Alkyl, -COR₂₂, -CSR₂₃ oder -SO₂R₂₄ bedeutet,
- R₁₆ eine Kette -CHOH- oder -CH(OH)-alk(1-5C)- darstellt,
- R₁₇ ein Rest Alkyl oder Phenylalkyl ist,
- R₁₈ ein Wasserstoffatom oder ein Rest Alkyl ist,
- R₁₉ einen Rest Hydroxy, Alkyl, -alk-Het, -NR₂₅R₂₆, -alk-COOR₁₀, -Het, Phenyl, gegebenenfalls substituiert durch einen oder mehrere Substituenten, ausgewählt unter den Halogenatomen und den Resten Alkyl, Alkoxy, Nitro, Amino, Hydroxy, -alk-NH₂, -COOR₁₀, Cyano und -alk-COOR₁₀ oder Phenylalkyl, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, -alk-NH₂, -COOR₁₀, Cyano und -alk-COOR₁₀ darstellt,
- R₂₀ ein Wasserstoffatom oder ein Rest Alkyl ist,
- R₂₁ ein Wasserstoffatom oder ein Rest Alkyl ist,
- R₂₂ einen Rest Alkyl, Cycloalkyl, -COOalk, -alk-COOR₁₀, Phenyl, gegebenenfalls substituiert durch einen oder mehrere Substituenten, ausgewählt unter den Halogenatomen und den Resten Alkyl, Alkoxy, Nitro, Amino, Hydroxy, -alk-NH₂, -COOR₁₀, Cyano und -alk-COOR₁₀, Phenylalkyl, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, -alk-NH₂, -COOR₁₀, Cyano und -alk-COOR₁₀, -alk-NR₁₀R₁₂, -NH-Ar, bei dem Ar gegebenenfalls substituiert ist durch einen oder mehrere Substituenten, ausgewählt unter den Halogenatomen und den Resten Alkyl, Alkoxy, Nitro, Amino, Hydroxy, -alk-NH₂, -COOR₁₀, Cyano und -alk-COOR₁₀, -Het, -alk-Het, -OR₁₇, -NH-alk-Ar, bei dem Ar gegebenenfalls substituiert ist durch einen oder mehrere Substituenten, ausgewählt unter den Halogenatomen und den Resten Alkyl, Alkoxy, Nitro, Amino, Hydroxy, -alk-NH₂, -COOR₁₀, Cyano und -alk-COOR₁₀, -NH-alk-Het, -NH-alk, -NH₂ oder -NH-Het bedeutet,
- R₂₃ einen Rest -NH-alk, -NH-Ar, -NH-Het oder -NH₂ darstellt,
- R₂₄ ein Rest Alkyl oder Phenyl ist,
- R₂₅ und R₂₆, gleich oder verschieden, jeweils einen Rest Alkyl oder Cycloalkyl bedeuten,
- R₂₇ ein Wasserstoffatom oder ein Rest Alkyl ist,
- alk ein Rest Alkyl oder Alkylen ist,
- alk' ein Rest Alkyl ist,
- m gleich 0, 1 oder 2 ist,
- Ar ein Rest Phenyl ist,
- Het einen gesättigten oder ungesättigten, mono- oder polycyclischen Heterocyclus mit 1 bis 9 Kohlenstoffatomen und einem oder mehreren Heteroatomen (O, S, N) darstellt, gegebenenfalls substituiert durch einen oder mehrere Reste Alkyl, Phenyl oder Phenylalkyl,
mit der Maßgabe, daß die Reste und Teile Alkyl, Alkylen und Alkoxy 1 bis 6 Kohlenstoffatome enthalten und gerade oder verzweigt sind, die Reste und Teile Acyl 2 bis 4 Kohlenstoffatome enthalten und die Reste Cycloalkyl 3 bis 6 Kohlenstoffatome enthalten, sowie bei den Verbindungen, worin R₇ einen Rest NO-alk, C(COOR₁₀)R₂₀ oder C(CONR₁₀R₂₁)R₂₀, CHR₁₉ darstellt, ihre Isomeren (E und Z), bei den Verbindungen, worin R einen Rest CH-R₆ bedeutet und R₆ ein Rest -CO-COOR₁₀ ist, ihre tautomeren Formen (E und Z), und bei den Verbindungen, worin R einen Rest C(R₄)R₅ oder CH-R₆ darstellt, ihre Enantiomeren und Diastereoisomeren und die Salze dieser Verbindungen.

2. Verbindungen der Formel (I) nach Anspruch 1, worin Het ausgewählt wird unter den Ringen Pyrrolyl, gegebenenfalls substituiert durch einen oder mehrere Reste Alkyl, Phenyl oder Phenylalkyl, Pyridyl, gegebenenfalls substituiert durch einen oder mehrere Reste Alkyl, Phenyl oder Phenylalkyl, Pyrimidinyl, gegebenenfalls substituiert durch einen oder mehrere Reste Alkyl, Phenyl oder Phenylalkyl, Imidazolyl, gegebenenfalls substituiert durch einen oder mehrere Reste Alkyl, Phenyl oder Phenylalkyl, Thiazolyl, gegebenenfalls substituiert durch einen oder mehrere Reste Alkyl, Phenyl oder Phenylalkyl, Oxazolinyl, gegebenenfalls substituiert durch einen oder mehrere Reste Alkyl, Phenyl oder Phenylalkyl, Thiazolinyl, gegebenenfalls substituiert durch einen oder mehrere Reste Alkyl, Phenyl oder Phenylalkyl, Pyrazinyl, gegebenenfalls substituiert durch einen oder mehrere Reste Alkyl, Phenyl oder Phenylalkyl, Tetrazolyl, gegebenenfalls substituiert durch einen oder mehrere Reste Alkyl, Phenyl oder Phenylalkyl oder Triazolyl, gegebenenfalls substituiert durch einen oder mehrere Reste Alkyl, Phenyl oder Phenylalkyl.

3. Verfahren zur Herstellung der Verbindungen der Formel (I) nach Anspruch 1, worin R einen Rest N-alk oder CH-R₆ darstellt, R₆ ein Wasserstoffatom ist und R₃ einen Rest Alkoxycarbonyl bedeutet, mit der Ausnahme, tert.-Butoxycarbonyl darzustellen, dadurch gekennzeichnet, daß man in Anwesenheit von Ammoniumacetat ein Derivat der Formel (II) 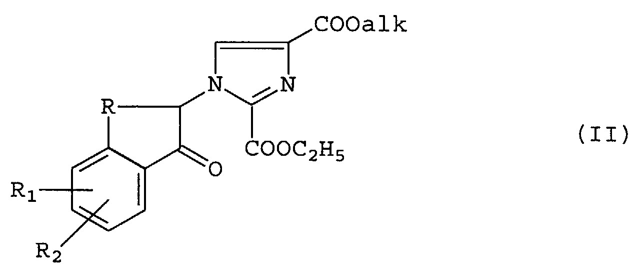 cyclisiert, in der R einen Rest N-alk oder CH-R₆ darstellt, R₆ ein Wasserstoffatom ist, R₁ und R₂ die gleichen Bedeutungen wie in Anspruch besitzen und -COOalk einen Rest Alkoxycarbonyl außer tert.-Butoxycarbonyl bedeutet, das Produkt isoliert und gegebenenfalls in Salz überführt.

4. Verfahren zur Herstellung der Verbindungen der Formel (I) nach Anspruch 1, worin R einen Rest N-alk oder CH-R₆ darstellt, R₆ ein Wasserstoffatom ist und R₃ einen Rest tert.-Butoxycarbonyl bedeutet, dadurch gekennzeichnet, daß man Isobuten mit einer entsprechenden Verbindung der Formel (I) zur Reaktion bringt, in der R einen Rest N-alk oder CH-R₆ darstellt, R₆ ein Wasserstoffatom ist und R₃ einen Rest Carboxy bedeutet, das Produkt isoliert und gegebenenfalls in Salz überführt.

5. Verfahren zur Herstellung der Verbindungen der Formel (I) nach Anspruch 1, worin R einen Rest N-alk oder CH-R₆ darstellt, R₆ ein Wasserstoffatom ist und R₃ einen Rest Carboxy bedeutet, dadurch gekennzeichnet, daß man in Anwesenheit von Ammoniumacetat ein Derivat der Formel (II) 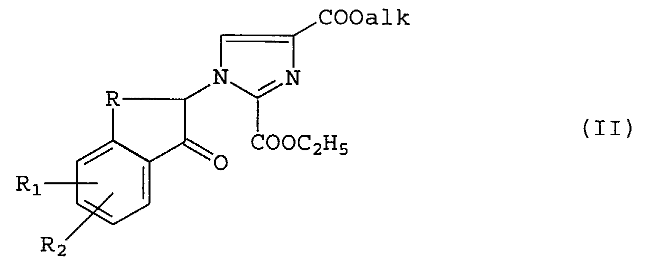 cyclisiert, in der R einen Rest N-alk oder CH-R₆ darstellt, R₆ ein Wasserstoffatom ist und COOalk einen Rest tert.-Butoxycarbonyl bedeutet, das Produkt isoliert und gegebenenfalls in Salz überführt.

6. Verfahren zur Herstellung der Verbindungen der Formel (I) nach Anspruch 1, worin R einen Rest N-alk oder CH-R₆ darstellt, R₆ ein Wasserstoffatom ist und R₃ einen Rest Carboxamido bedeutet, dadurch gekennzeichnet, daß man ein Derivat der Formel (IV) 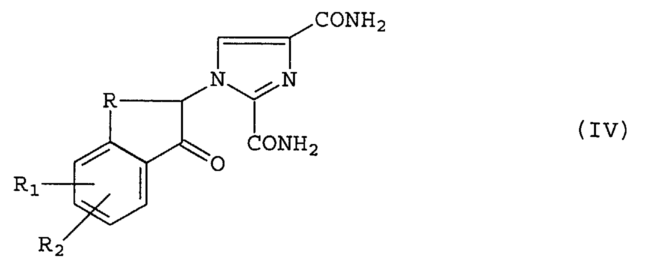 cyclisiert, in der R einen Rest N-alk oder CH-R₆ darstellt, R₆ ein Wasserstoffatom ist und R₁ und R₂ die gleichen Bedeutungen wie in Anspruch 1 besitzen, das Produkt isoliert und gegebenenfalls in Salz überführt.

7. Verfahren zur Herstellung der Verbindungen der Formel (I) nach Anspruch 1, worin R einen Rest C =R₇ darstellt und R₇ ein Sauerstoffatom ist, dadurch gekennzeichnet, daß man eine entsprechende Verbindung der Formel (I), worin R einen Rest C =R₇ darstellt und R₇ ein Rest NOH ist, hydrolysiert, gefolgt bei den Verbindungen, worin R₃ einen Rest Alkoxycarbonyl bedeutet, von einer Veresterung der entsprechenden Säure, das Produkt isoliert und gegebenenfalls in Salz überführt.

8. Verfahren zur Herstellung der Verbindungen der Formel (I) nach Anspruch 1, worin R einen Rest C =R₇ darstellt und R₇ ein Rest NOH ist, dadurch gekennzeichnet, daß man ein Alkylnitrit mit einer entsprechenden Verbindung der Formel (I), worin R einen Rest CH-R₆ darstellt und R₆ ein Wasserstoffatom ist, zur Reaktion bringt, das Produkt isoliert und gegebenenfalls in Salz überführt.

9. Verfahren zur Herstellung der Verbindungen der Formel (I) nach Anspruch 1, worin R einen Rest C =R₇ darstellt und R₇ ein Rest NO-alk-COOR₁₀ oder NO-alk ist, dadurch gekennzeichnet, daß man eine entsprechende Verbindung der Formel (I), worin R einen Rest C =R₇ darstellt und R₇ ein Rest NOH ist, mit einem Halogenid Hal-Ra zur Reaktion bringt, worin Hal ein Halogenatom ist und Ra einen Rest Alkyl oder -alk-COOR₁₀ darstellt und alk und R₁₀ die gleichen Bedeutungen wie in Anspruch 1 besitzen, das Produkt isoliert und gegebenenfalls in Salz überführt.

10. Verfahren zur Herstellung der Verbindungen der Formel (I) nach Anspruch 1, worin R einen Rest C =R₇ darstellt und R₇ ein Rest CH-R₁₉ ist, worin R₁₉ einen Rest Hydroxy bedeutet, dadurch gekennzeichnet, daß man eine entsprechende Verbindung der Formel (I), worin R₁₉ einen Rest -NR₂₅R₂₆ darstellt, hydrolysiert, gefolgt bei den Verbindungen, worin R₃ einen Rest Alkoxycarbonyl bedeutet, von einer Veresterung der entsprechenden Säure, das Produkt isoliert und gegebenenfalls in Salz überführt.

11. Verfahren zur Herstellung der Verbindungen der Formel (I) nach Anspruch 1, worin R einen Rest C =R₇ darstellt und R₇ ein Rest CH-R₁₉ ist, worin R₁₉ einen Rest -NR₂₅R₂₆ bedeutet, dadurch gekennzeichnet, daß man eine entsprechende Verbindung der Formel (I), worin R einen Rest -CH-R₆ darstellt und R₆ ein Wasserstoffatom ist, mit einem Derivat HC(Rb) (Rc)Rd zur Reaktion bringt, worin entweder Rb und Rd, gleich oder verschieden, jeweils einen Rest -NR₂₅R₂₆ darstellen, worin R₂₅ und R₂₆ die gleichen Bedeutungen wie in Anspruch 1 besitzen und Rc ein Rest Alkoxy ist, oder Rb, Rc und Rd identisch sind und jeweils einen Rest -NR₂₅R₂₆ darstellen, worin R₂₅ und R₂₆ die gleichen Bedeutungen wie in Anspruch 1 besitzen, das Produkt isoliert und gegebenenfalls in Salz überführt.

12. Verfahren zur Herstellung der Verbindungen der Formel (I) nach Anspruch 1, worin R einen Rest C =R₇ darstellt, R₇ ein Rest CHR₁₉ ist und R₁₉ einen Rest Alkyl, Phenyl, gegebenenfalls substituiert, -alk-Het, Phenylalkyl, dessen Phenylkern gegebenenfalls substituiert ist, -Het oder -alk-COOR₁₀ bedeutet, dadurch gekennzeichnet, daß man eine entsprechende Verbindung der Formel (I), worin R einen Rest -CH-R₆ darstellt und R₆ ein Wasserstoffatom ist, mit einem Aldehyd der Formel OHC-Re zur Reaktion bringt, worin Re einen Rest Alkyl, Phenyl, gegebenenfalls substituiert durch einen oder mehrere Substituenten, ausgewählt unter den Halogenatomen und den Resten Alkyl, Alkoxy, Nitro, Amino, Hydroxy, -alk-NH₂, -COOR₁₀, Cyano und -alk-COOR₁₀, -alk-Het, Phenylalkyl, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, -alk-NH₂, -COOR₁₀, Cyano und -alk-COOR₁₀, -Het oder -alk-COOR₁₀ darstellt, worin alk, Het und R₁₀ die gleichen Bedeutungen wie in Anspruch 1 besitzen, das Produkt isoliert und gegebenenfalls in Salz überführt.

13. Verfahren zur Herstellung der Verbindungen der Formel (I) nach Anspruch 1, worin R einen Rest C =R₇ darstellt und R₇ ein Rest NR₁₀ ist, dadurch gekennzeichnet, daß man Trifluoressigsäureethylester mit einem Derivat der Formel (V) 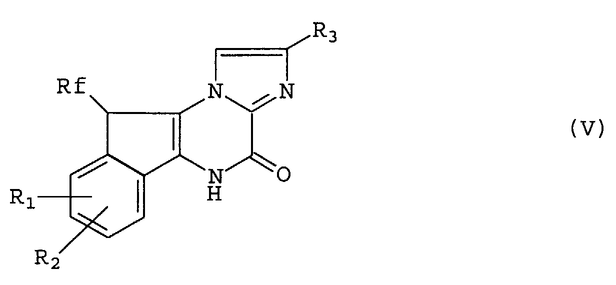 zur Reaktion bringt, in der R₁, R₂ und R₃ die gleichen Bedeutungen wie in Anspruch 1 besitzen und Rf einen Rest -NH₂ oder -NH-alk darstellt, worin alk die gleichen Bedeutungen wie in Anspruch 1 besitzt, das Produkt isoliert und gegebenenfalls in Salz überführt.

14. Verfahren zur Herstellung der Verbindungen der Formel (I) nach Anspruch 1, worin R einen Rest C =R₇ darstellt und R₇ ein Rest C(COOR₁₀)R₂₀ ist, dadurch gekennzeichnet, daß man eine Verbindung der Formel (V) 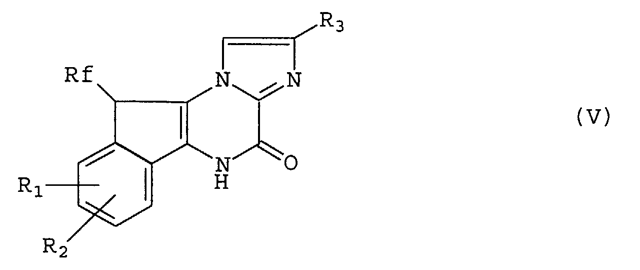 dehydratisiert, in der R₁, R₂ und R₃ die gleichen Bedeutungen wie in Anspruch 1 besitzen und Rf einen Rest -C(R₂₀)(OH)-COOR₁₀ darstellt, worin R₂₀ und R₁₀ die gleichen Bedeutungen wie in Anspruch 1 besitzen, das Produkt isoliert und gegebenenfalls in Salz überführt.

15. Verfahren zur Herstellung der Verbindungen der Formel (I) nach Anspruch 1, worin R einen Rest C =R₇ darstellt und R₇ ein Rest C(CONR₁₀R₂₁)R₂₀ ist, dadurch gekennzeichnet, daß man eine entsprechende Verbindung der Formel (I), worin R einen Rest CH=R₇ darstellt und R₇ ein Rest C(COOR₁₀)R₂₀ ist, mit einem Amin HNR₁₀R₂₁ zur Reaktion bringt, worin R₁₀ und R₂₁ die gleichen Bedeutungen wie in Anspruch 1 besitzen, das Produkt isoliert und gegebenenfalls in Salz überführt.

16. Verfahren zur Herstellung der Verbindungen der Formel (I) nach Anspruch 1, worin R einen Rest C(R₄)R₅ darstellt, R₄ ein Rest Alkyl, -alk-Het oder Phenylalkyl ist, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, und R₅ mit R₄ identisch ist, dadurch gekennzeichnet, daß man eine entsprechende Verbindung der Formel (I), worin R einen Rest CH-R₆ darstellt und R₆ ein Wasserstoffatom ist, mit einem Halogenid der Formel Hal-Rg zur Reaktion bringt, worin Rg einen Rest Alkyl, -alk-Het oder Phenylalkyl darstellt, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, und alk, Het und R₁₀ die gleichen Bedeutungen wie in Anspruch 1 besitzen, das Produkt isoliert und gegebenenfalls in Salz überführt.

17. Verfahren zur Herstellung der Verbindungen der Formel (I) nach Anspruch 1, worin R einen Rest C(R₄)R₅ darstellt, R₄ ein Rest Alkyl, -alk-Het oder Phenylalkyl ist, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, und R₅ einen Rest Alkyl (1 bis 11 Kohlenstoffatome in gerader oder verzweigter Kette), -alk-CN, -alk-Het, -alk-NR₁₀R₁₈, -alk-CONR₁₀R₁₈ oder Phenylalkyl bedeutet, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, dadurch gekennzeichnet, daß man eine entsprechende Verbindung der Formel (I), worin R einen Rest CH-R₆ darstellt und R₆ einen Rest Alkyl, -alk-Het oder Phenylalkyl bedeutet, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, mit einem Halogenid Hal-Rh zur Reaktion bringt, worin Rh einen Rest Alkyl (1 bis 11 Kohlenstoffatome in gerader oder verzweigter Kette), -alk-Het, -alk-CN, -alk-NR₁₀R₁₈, -alk-CONR₁₀R₁₈ oder Phenylalkyl darstellt, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, und alk, Het, R₁₀ und R₁₈ die gleichen Bedeutungen wie in Anspruch 1 besitzen, das Produkt isoliert und gegebenenfalls in Salz überführt.

18. Verfahren zur Herstellung der Verbindungen der Formel (I) nach Anspruch 1, worin R einen Rest C(R₄)R₅ darstellt und R₄ und R₅ zusammen mit dem Kohlenstoffatom, an das sie gebunden sind, einen Rest Cycloalkyl bilden, dadurch gekennzeichnet, daß man eine entsprechende Verbindung der Formel (I), worin R einen Rest CH-R₆ darstellt und R₆ ein Wasserstoffatom ist, mit einem Derivat der Formel Hal-alk-Hal zur Reaktion bringt, worin Hal ein Halogenatom ist und alk einen Rest Alkyl (2 bis 5 Kohlenstoffatome) darstellt, das Produkt isoliert und gegebenenfalls in Salz überführt.

19. Verfahren zur Herstellung der Verbindungen der Formel (I) nach Anspruch 1, worin R einen Rest C(R₄)R₅ darstellt und R₅ ein Rest -NR₈R₉ ist, worin R₈ und R₉ Wasserstoffatome sind, dadurch gekennzeichnet, daß man ein Halogenid Hal-R₄, worin Hal ein Halogenatom ist und R₄ die gleichen Bedeutungen wie in Anspruch 1 besitzt, mit einer entsprechenden Verbindung der Formel (I) zur Reaktion bringt, worin R einen Rest CH-R₆ darstellt, R₆ einen Rest -NR₁₄R₁₅ bedeutet, R₁₄ ein Wasserstoffatom ist, R₁₅ einen Rest -COR₂₂ darstellt und R₂₂ ein Rest Alkyl (1 Kohlenstoffatom) ist, eine Hydrolyse anschließt, das Produkt isoliert und gegebenenfalls in Salz überführt.

20. Verfahren zur Herstellung der Verbindungen der Formel (I) nach Anspruch 1, worin R einen Rest C(R₄)R₅ darstellt und R₅ ein Rest -NR₈R₉ ist, worin R₉ ein Wasserstoffatom ist und R₈ einen Rest Alkyl, -alk-COOR₁₀, -alk-NR₁₀R₂₁, -alk-Het oder Phenylalkyl bedeutet, dessen Phenylkern gegebenenfalls substituiert ist, dadurch gekennzeichnet, daß man eine entsprechende Verbindung der Formel (I), worin R einen Rest C(R₄)R₅ darstellt und R₅ ein Rest -NR₈R₉ ist, worin R₈ und R₉ Wasserstoffatome sind, mit einem Halogenid Hal-R₈ zur Reaktion bringt, worin R₈ die gleichen Bedeutungen wie vorstehend besitzt, das Produkt isoliert und gegebenenfalls in Salz überführt.

21. Verfahren zur Herstellung der Verbindungen der Formel (I) nach Anspruch 1, worin R einen Rest C(R₄)R₅ darstellt und R₅ ein Rest -NR₈R₉ ist, worin R₉ ein Wasserstoffatom oder ein Rest Alkyl ist und R₈ einen Rest Alkyl (2 bis 6 Kohlenstoffatome) bedeutet, dadurch gekennzeichnet, daß man eine entsprechende Verbindung der Formel (I), worin R einen Rest C(R₄)R₅ darstellt und R₅ ein Rest -NR₈R₉ ist, worin R₉ ein Wasserstoffatom oder einen Rest Alkyl bedeutet und R₈ ein Wasserstoffatom ist, mit einem Acylhalogenid (2 bis 6 Kohlenstoffatome) zur Reaktion bringt, anschließend die Reduktion des erhaltenen Produktes durchführt, das Produkt isoliert und gegebenenfalls in Salz überführt.

22. Verfahren zur Herstellung der Verbindungen der Formel (I) nach Anspruch 1, worin R einen Rest C(R₄)R₅ darstellt und R₅ ein Rest -NR₈R₉ ist, worin R₉ einen Rest Alkyl bedeutet, dadurch gekennzeichnet, daß man eine entsprechende Verbindung der Formel (I), worin R einen Rest C(R₄)R₅ darstellt und R₅ ein Rest -NR₈R₉ ist, worin R₈ die gleichen Bedeutungen wie in Anspruch 1 besitzt und R₉ ein Wasserstoffatom ist, mit einem Derivat der Formel Hal-alk zur Reaktion bringt, in der Hal ein Halogenatom ist und alk einen Rest Alkyl darstellt, das Produkt isoliert und gegebenenfalls in Salz überführt.

23. Verfahren zur Herstellung der Verbindungen der Formel (I) nach Anspruch 1, worin R einen Rest C(R₄)R₅ darstellt und R₅ ein Rest -NR₈R₉ ist, worin R₉ ein Wasserstoffatom oder einen Rest Alkyl bedeutet und R₈ einen Rest -alk(2-6C)-NR₁₀R₂₁ darstellt, dadurch gekennzeichnet, daß man ein Derivat der Formel (VI) 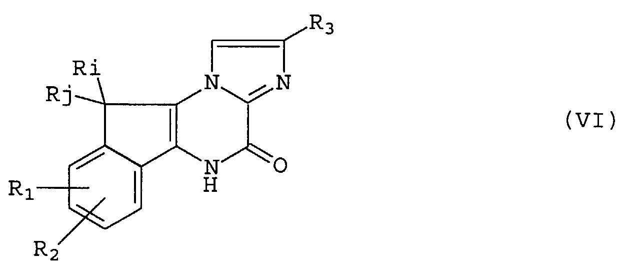 reduziert, in der R₁, R₂ und R₃ die gleichen Bedeutungen wie in Formel (I) besitzen, Ri einen Rest Alkyl, Phenylalkyl, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, oder alk-Het darstellt und Rj einen Rest -NH-CO-alk(1-5C)-NR₁₀R₂₁ oder -N(alk)-CO-alk(1-5C)-NR₁₀R₂₁ bedeutet, worin alk, Het, R₁₀ und R₂₁ die gleichen Bedeutungen wie in Anspruch 1 besitzen, das Produkt isoliert und gegebenenfalls in Salz überführt.

24. Verfahren zur Herstellung der Verbindungen der Formel (I) nach Anspruch 1, worin R einen Rest C(R₄)R₅ darstellt und R₅ ein Rest -COOR₁₀ ist, worin R₁₀ ein Wasserstoffatom bedeutet, dadurch gekennzeichnet, daß man eine entsprechende Verbindung der Formel (I), worin R einen Rest C(R₄)R₅ darstellt und R₅ ein Rest -COOR₁₀ ist, worin R₁₀ einen Rest Alkyl bedeutet, hydrolysiert, das Produkt isoliert und gegebenenfalls in Salz überführt.

25. Verfahren zur Herstellung der Verbindungen der Formel (I) nach Anspruch 1, worin R einen Rest C(R₄)R₅ darstellt und R₅ ein Rest -COOR₁₀ ist, worin R₁₀ einen Rest Alkyl bedeutet, dadurch gekennzeichnet, daß man eine entsprechende Verbindung der Formel (I), worin R einen Rest C-R₆ darstellt und R₆ ein Rest Alkyl, alk-Het oder Phenylalkyl ist, gegebenenfalls substituiert, mit einem Halogenid der Formel Hal-COOR₁₀ zur Reaktion bringt, worin R₁₀ einen Rest Alkyl bedeutet und Hal ein Halogenatom ist, das Produkt isoliert und gegebenenfalls in Salz überführt.

26. Verfahren zur Herstellung der Verbindungen der Formel (I) nach Anspruch 1, worin R einen Rest C(R₄)R₅ darstellt und R₅ ein Rest -alk-COOR₁₀ ist, dadurch gekennzeichnet, daß man eine entsprechende Verbindung der Formel (I), worin R einen Rest C-R₆ darstellt und R₆ ein Rest -alk-COOR₁₀ ist, worin R₁₀ die gleichen Bedeutungen wie in Anspruch 1 besitzt, mit einem Halogenid Hal-R₄ zur Reaktion bringt, worin R₄ die gleichen Bedeutungen wie in Anspruch 1 besitzt, das Produkt isoliert und gegebenenfalls in Salz überführt.

27. Verfahren zur Herstellung der Verbindungen der Formel (I) nach Anspruch 1, worin R einen Rest C(R₄)R₅ darstellt und R₅ ein Rest -alk-CN ist, worin alk 1 Kohlenstoffatom enthält, dadurch gekennzeichnet, daß man Natriumcyanid mit einem Derivat der Formel (VI) 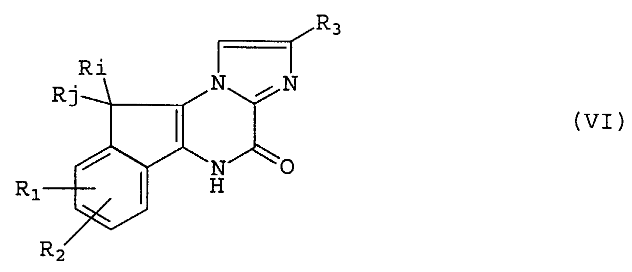 zur Reaktion bringt, in der R₁, R₂ und R₃ die gleichen Bedeutungen wie in Anspruch 1 besitzen, Ri einen Rest -CH₂OTs darstellt, worin Ts ein Rest Tosylat ist und Rj einen Rest Alkyl, -alk-Het oder Phenylalkyl, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, bedeutet und alk, Het und R₁₀ die gleichen Bedeutungen wie in Anspruch 1 besitzen, das Produkt isoliert und gegebenenfalls in Salz überführt.

28. Verfahren zur Herstellung der Verbindungen der Formel (I) nach Anspruch 1, worin R einen Rest C(R₄)R₅ darstellt und R₅ ein Rest -alk(2-6C)OH ist, dadurch gekennzeichnet, daß man (COCl)₂ mit einer entsprechenden Verbindung der Formel (I) zur Reaktion bringt, worin R einen Rest C(R₄)R₅ darstellt und R₅ ein Rest -alk-COOR₁₀ ist, worin R₁₀ ein Wasserstoffatom bedeutet, anschließend eine Reduktion durchführt, das Produkt isoliert und gegebenenfalls in Salz überführt.

29. Verfahren zur Herstellung der Verbindungen der Formel (I) nach Anspruch 1, worin R einen Rest C(R₄)R₅ darstellt und R₅ ein Rest -alk(1C)OH ist, dadurch gekennzeichnet, daß man Trimethylsilanchlorid mit einem Derivat der Formel (VI) 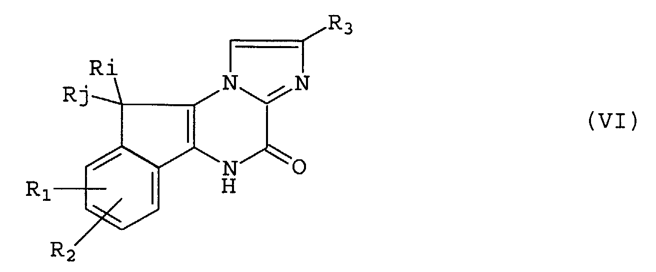 zur Reaktion bringt, in der R₁, R₂ und R₃ die gleichen Bedeutungen wie in Anspruch 1 besitzen, Ri ein Wasserstoffatom darstellt und Rj einen Rest Alkyl, -alk-Het oder Phenylalkyl, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, bedeutet und alk, Het und R₁₀ die gleichen Bedeutungen wie in Anspruch 1 besitzen, anschließend mit Trioxan umsetzt, das Produkt isoliert und gegebenenfalls in Salz überführt.

30. Verfahren zur Herstellung der Verbindungen der Formel (I) nach Anspruch 1, worin R einen Rest C(R₄)R₅ darstellt und R₅ ein Rest -NH-CHO ist, dadurch gekennzeichnet, daß man ein Derivat der Formel (VI) 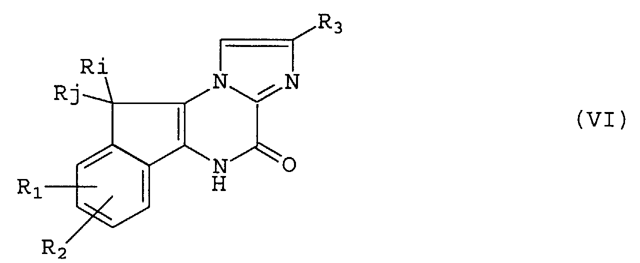 in der R₁, R₂ und R₃ die gleichen Bedeutungen wie in Anspruch 1 besitzen, Ri einen Rest Alkyl, -alk-Het oder Phenylalkyl, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, darstellt und Rj ein Rest Amino ist, und alk, Het und R₁₀ die gleichen Bedeutungen wie in Anspruch 1 besitzen, mit CH₃COOCHO zur Reaktion bringt, das Produkt isoliert und gegebenenfalls in Salz überführt.

31. Verfahren zur Herstellung der Verbindungen der Formel (I) nach Anspruch 1, worin R einen Rest C(R₄)R₅ darstellt und R₅ einen Rest -NH-COOR₁₇, -NH-CO-Het, -NH-CO-alk-COOR₁₀, -NH-CO-alk-NR₁₀R₁₈, -NH-CO-Ar, bei dem Ar gegebenenfalls substituiert ist, -NH-CO-alk-Ar, bei dem Ar gegebenenfalls substituiert ist, -NH-SO₂-R₂₄, -NH-CO-alk-Het, -NH-CO-alk oder -NH-CO-cycloalkyl bedeutet, dadurch gekennzeichnet, daß man eine entsprechende Verbindung der Formel (I), worin R einen Rest C(R₄)R₅ darstellt und R₅ einen Rest -NR₈R₉ bedeutet, worin R₈ und R₉ Wasserstoffatome sind, mit einem Derivat Hal-Rk zur Reaktion bringt, worin Hal ein Halogenatom ist und Rk einen Rest -COOR₁₇, -CO-Het, -CO-alk-COOR₁₀, -CO-alk-NR₁₀R₁₈, -CO-alk-Ar, bei dem Ar gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, -SO₂-R₂₄, -CO-alk-Het, -CO-Ar, bei dem Ar gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, -CO-alk- oder -CO-cycloalkyl darstellt, und R₇, R₁₀, R₁₇, R₁₈, R₂₄, Het, Ar und alk die gleichen Bedeutungen wie in Anspruch 1 besitzen, das Produkt isoliert und gegebenenfalls in Salz überführt.

32. Verfahren zur Herstellung der Verbindungen der Formel (I) nach Anspruch 1, worin R einen Rest C(R₄)R₅ darstellt und R₅ einen Rest -NH-CO-Het, -NH-CO-alk-COOR₁₀, -NH-CO-alk-NR₁₀R₁₈, -NH-CO-Ar, bei dem Ar gegebenenfalls substituiert ist, -NH-CO-alk-Ar, bei dem Ar gegebenenfalls substituiert ist, -NH-CO-alk-Het, -NH-CO-alk oder -NH-CO-cycloalkyl bedeutet, dadurch gekennzeichnet, daß man eine entsprechende Verbindung der Formel (I), worin R einen Rest C(R₄)R₅ darstellt und R₅ einen Rest -NR₈R₉ bedeutet, worin R₈ und R₉ Wasserstoffatome sind, mit einem Derivat HO-Rl zur Reaktion bringt, worin Rl einen Rest -CO-Het, -CO-alk-COOR₁₀, -CO-alk-NR₁₀R₁₈, -CO-alk-Ar, bei dem Ar gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, -CO-alk-Het, -CO-Ar, bei dem Ar gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, -CO-alk- oder -CO-cycloalkyl darstellt, und R₁₀, R₁₈, Het, Ar und alk die gleichen Bedeutungen wie in Anspruch 1 besitzen, das Produkt isoliert und gegebenenfalls in Salz überführt.

33. Verfahren zur Herstellung der Verbindungen der Formel (I) nach Anspruch 1, worin R einen Rest C(R₄)R₅ darstellt und R₅ einen Rest Pyrrol-1-yl bedeutet, gegebenenfalls substituiert durch einen Rest -COOR₁₀, dadurch gekennzeichnet, daß man ein Derivat der Formel (VI) 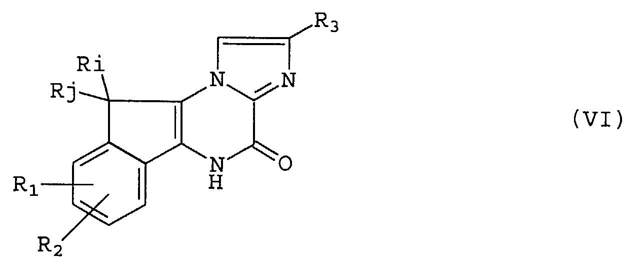 in der R₁, R₂ und R₃ die gleichen Bedeutungen wie in Anspruch 1 besitzen, Ri einen Rest Alkyl, -alk-Het oder Phenylalkyl, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, darstellt und alk, Het und R₁₀ die gleichen Bedeutungen wie in der Formel (I) besitzen und Rj ein Rest Amino ist, mit einem Derivat der Formel (VIII) 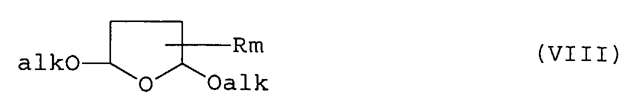 zur Reaktion bringt, worin Rm ein Wasserstoffatom oder einen Rest -COOR₁₀ bedeutet und alk und R₁₀ die gleichen Bedeutungen wie in Anspruch 1 besitzen, das Produkt isoliert und gegebenenfalls in Salz überführt.

34. Verfahren zur Herstellung der Verbindungen der Formel (I) nach Anspruch 1, worin R einen Rest C(R₄)R₅ darstellt und R₅ einen Rest -NH-CO-NH-alk-Ar, bei dem Ar gegebenenfalls substituiert ist, -NH-CO-NH-Het, -NH-CO-NH-alk-Het, -NH-CO-NH-Ar, bei dem Ar gegebenenfalls substituiert ist, -NH-CO-NH-alk oder -NH-CO-NH₂ bedeutet, dadurch gekennzeichnet, daß man eine entsprechende Verbindung der Formel (I), worin R einen Rest C(R₄)R₅ darstellt und R₅ einen Rest -NR₈R₉ bedeutet, worin R₈ und R₉ Wasserstoffatome sind, mit einem Derivat O=C=N-Rn zur Reaktion bringt, worin Rn einen Rest Trimethylsilyl, Alkyl, -Het, -alk-Ar, bei dem Ar gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, alk-Het, Phenyl, gegebenenfalls substituiert durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, darstellt, worin R₁₀, alk, Ar und Het die gleichen Bedeutungen wie in Anspruch 1 besitzen, das Produkt isoliert und gegebenenfalls in Salz überführt.

35. Verfahren zur Herstellung der Verbindungen der Formel (I) nach Anspruch 1, worin R einen Rest CH-R₆ darstellt und R₆ ein Rest Hydroxy ist, dadurch gekennzeichnet, daß man eine entsprechende Verbindung der Formel (I), worin R einen Rest C=R₇ darstellt und R₇ ein Sauerstoffatom ist, reduziert, das Produkt isoliert und gegebenenfalls in Salz überführt.

36. Verfahren zur Herstellung der Verbindungen der Formel (I) nach Anspruch 1, worin R einen Rest CH-R₆ darstellt und R₆ einen Rest Alkyl (2 bis 11 Kohlenstoffatome), Phenylalkyl, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, oder -alk-Het bedeutet, dadurch gekennzeichnet, daß man ein Derivat der Formel (IX) 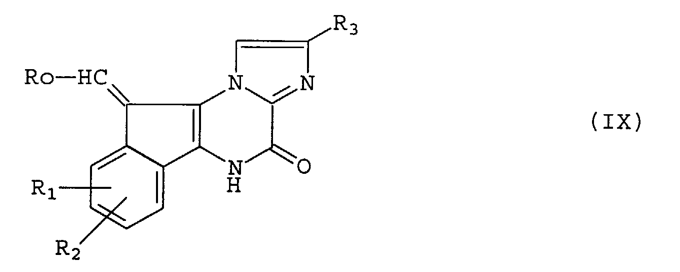 hydriert, in der R₁, R₂ und R₃ die gleichen Bedeutungen wie in Anspruch 1 besitzen, Ro einen Rest Alkyl mit 1 bis 10 Kohlenstoffatomen in gerader oder verzweigter Kette, Phenyl, Phenylalkyl (1-5C), dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, -Het, -alk(1-5C)-Het darstellt, worin alk, Het und R₁₀ die gleichen Bedeutungen wie in Anspruch 1 besitzen, das Produkt isoliert und gegebenenfalls in Salz überführt.

37. Verfahren zur Herstellung der Verbindungen der Formel (I) nach Anspruch 1, worin R einen Rest CH-R₆ darstellt und R₆ einen Rest Phenylalkyl, gegebenenfalls substituiert, oder -alk-Het bedeutet, dadurch gekennzeichnet, daß man eine entsprechende Verbindung der Formel (I), worin R einen Rest CH-R₆ darstellt und R₆ ein Wasserstoffatom ist, mit einem Halogenid der Formel Hal-Rp zur Reaktion bringt, worin Rp einen Rest Phenylalkyl, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, oder alk-Het darstellt, worin alk, Het und R₁₀ die gleichen Bedeutungen wie in Anspruch 1 besitzen, das Produkt isoliert und gegebenenfalls in Salz überführt.

38. Verfahren zur Herstellung der Verbindungen der Formel (I) nach Anspruch 1, worin R einen Rest CH-R₆ darstellt und R₆ einen Rest Alkyl(1C) bedeutet, dadurch gekennzeichnet, daß man eine entsprechende Verbindung der Formel (I), worin R einen Rest C=R₇ darstellt und R₇ ein Rest CH-R₁₉ ist, worin R₁₉ einen Rest Hydroxy oder -NR₂₅R₂₆ bedeutet, reduziert, das Produkt isoliert und gegebenenfalls in Salz überführt.

39. Verfahren zur Herstellung der Verbindungen der Formel (I) nach Anspruch 1, worin R einen Rest CH-R₆ darstellt und R₆ einen Rest -alk(1C)-OH bedeutet, dadurch gekennzeichnet, daß man eine entsprechende Verbindung der Formel (I), worin R einen Rest C=R₇ darstellt und R₇ ein Rest CH-R₁₉ ist, worin R₁₉ einen Rest Hydroxy bedeutet, reduziert, das Produkt isoliert und gegebenenfalls in Salz überführt.

40. Verfahren zur Herstellung der Verbindungen der Formel (I) nach Anspruch 1, worin R einen Rest CH-R₆ darstellt und R₆ einen Rest -alk(2-6C)-OH bedeutet, dadurch gekennzeichbet, daß man ein Derivat der Formel (IX) 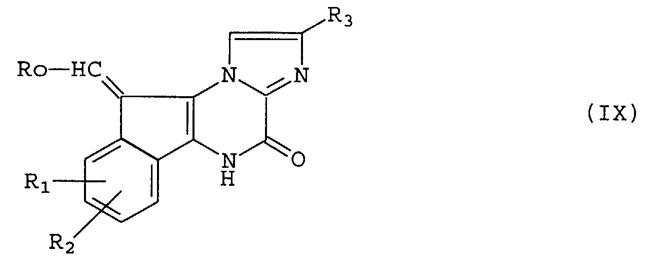 in der R₁, R₂ und R₃ die gleichen Bedeutungen wie in Anspruch 1 besitzen und Ro einen Rest -alk(1-5C)-O-CH₂-Ar darstellt, worin alk und Ar die gleichen Bedeutungen wie in Anspruch 1 besitzen, reduziert, das Produkt isoliert und gegebenenfalls in Salz überführt.

41. Verfahren zur Herstellung der Verbindungen der Formel (I) nach Anspruch 1, worin R einen Rest CH-R₆ darstellt und R₆ einen Rest -NR₁₄R₁₅ bedeutet, worin R₁₄ und R₁₅ jeweils ein Wasserstoffatom sind, dadurch gekennzeichnet, daß man eine entsprechende Verbindung der Formel (I), worin R einen Rest CH-R₆ darstellt und R₆ einen Rest -NR₁₄R₁₅ bedeutet, worin R₁₄ ein Wasserstoffatom ist, R₁₅ einen Rest -COR₂₂ bedeutet und R₂₂ ein Rest Alkyl ist, hydrolysiert, gefolgt bei den Verbindungen, worin R₃ einen Rest Alkoxycarbonyl darstellt, von einer Veresterung der entsprechenden Säure, das Produkt isoliert und gegebenenfalls in Salz überführt.

42. Verfahren zur Herstellung der Verbindungen der Formel (I) nach Anspruch 1, worin R einen Rest CH-R₆ darstellt und R₆ einen Rest -NR₁₄R₁₅ bedeutet, worin R₁₄ ein Wasserstoffatom ist, R₁₅ einen Rest -COR₂₂ darstellt und R₂₂ ein Rest Alkyl ist, dadurch gekennzeichnet, daß man ein Reduktionsmittel mit einer entsprechenden Verbindung der Formel (I), worin R einen Rest C=R₇ darstellt und R₇ ein Rest NOH ist, zur Reaktion bringt und anschließend mit einem Säureanhydrid (alkCO)₂O umsetzt, worin alk ein Rest Alkyl ist das Produkt isoliert und gegebenenfalls in Salz überführt.

43. Verfahren zur Herstellung der Verbindungen der Formel (I) nach Anspruch 1, worin R einen Rest CH-R₆ darstellt und R₆ einen Rest -NR₁₄R₁₅ oder -alk-NR₁₄R₁₅ bedeutet, worin R₁₄ und R₁₅ gleich oder verschieden, jeweils einen Rest Alkyl darstellen, oder R₁₄ ein Wasserstoffatom ist und R₁₅ einen Rest Alkyl, -COR₂₂ oder -SO₂R₂₄bedeutet, R₂₂ einen Rest Alkyl, Cycloalkyl, -COOalk, -alk-COOR₁₀, Phenyl, gegebenenfalls substituiert durch einen oder mehrere Substituenten, ausgewählt unter den Halogenatomen und den Resten Alkyl, Alkoxy, Nitro, Amino, Hydroxy, -alk-NH₂, -COOR₁₀, Cyano und -alk-COOR₁₀, Phenylalkyl, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, -alk-NH₂, -COOR₁₀, Cyano und -alk-COOR₁₀, -OR₁₇, -Het, -alk-Het, -alk-NR₁₀R₁₂ darstellt und R₂₄ einen Rest Alkyl oder Phenyl bedeutet, dadurch gekennzeichnet, daß man eine entsprechende Verbindung der Formel (I), worin R₁₄ und R₁₅ jeweils ein Wasserstoffatom sind, mit einem Halogenid der Formel Hal-Rr zur Reaktion bringt, worin Rr einen Rest Alkyl, -COR₂₂ oder -SO₂R₂₄ bedeutet, R₂₂ einen Rest Alkyl, Cycloalkyl, -COOalk, -alk-COOR₁₀, Phenyl, gegebenenfalls substituiert durch einen oder mehrere Substituenten, ausgewählt unter den Halogenatomen und den Resten Alkyl, Alkoxy, Nitro, Amino, Hydroxy, -alk-NH₂, -COOR₁₀, Cyano und -alk-COOR₁₀, Phenylalkyl, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, -alk-NH₂, -COOR₁₀, Cyano und -alk-COOR₁₀, -OR₁₇, -Het, -alk-Het, -alk-NR₁₀R₁₂ darstellt und R₂₄ einen Rest Alkyl oder Phenyl bedeutet, worin alk, Het, R₁₂, R₁₇ und R₁₀ die gleichen Bedeutungen wie in Anspruch 1 besitzen, das Produkt isoliert und gegebenenfalls in Salz überführt.

44. Verfahren zur Herstellung der Verbindungen der Formel (I) nach Anspruch 1, worin R einen Rest CH-R₆ darstellt und R₆ einen Rest -NR₁₄R₁₅ oder -alk-NR₁₄R₁₅ bedeutet, worin R₁₄ ein Wasserstoffatom ist und R₁₅ einen Rest Alkyl, -COR₂₂ oder -CSR₂₃ darstellt, R₂₂ einen Rest -NH-alk, -NH₂, -NH-Ar, bei dem Ar gegebenenfalls substituiert ist, -NH-alk-Ar, bei dem Ar gegebenenfalls substituiert ist, -NH-alk-Het oder -NH-Het bedeutet und R₂₃ ein Rest -NH-alk, -NH₂, -NH-Ar oder -NH-Het ist, dadurch gekennzeichnet, daß man eine entsprechende Verbindung der Formel (I), worin R einen Rest CH-R₆ darstellt und R₆ einen Rest -NR₁₄R₁₅ bedeutet, worin R₁₄ und R₁₅ jeweils ein Wasserstoffatom sind, mit einem Derivat Rs-N=C=Rt zur Reaktion bringt, worin Rs einen Rest Trimethylsilyl, Alkyl, Phenyl, gegebenenfalls substituiert durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, -alk-Ar, bei dem Ar gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, -alk-Het oder -Het darstellt, worin Het, alk, R₁₀ und Ar die gleichen Bedeutungen wie in Anspruch 1 besitzen und Rt ein Sauerstoffatom oder Schwefelatom ist, gegebenenfalls eine Hydrolyse anschließt, das Produkt isoliert und gegebenenfalls in Salz überführt.

45. Verfahren zur Herstellung der Verbindungen der Formel (I) nach Anspruch 1, worin R einen Rest CH-R₆ darstellt und R₆ einen Rest -NR₁₄R₁₅ oder -alk-NR₁₄R₁₅ bedeutet, worin R₁₄ ein Wasserstoffatom ist und R₁₅ einen Rest -COR₂₂ darstellt und R₂₂ ein Rest -alk(1C)-NR₁₀R₁₂ ist, worin R₁₀ und R₁₂ Wasserstoffatome sind, dadurch gekennzeichnet, daß man eine entsprechende Verbindung der Formel (I), worin R₁₄ und R₁₅ jeweils ein Wasserstoffatom sind, mit einer Säure HOOC-CH₂-NH-Ru zur Reaktion bringt, worin Ru eine Schutzgruppe für die Aminfunktion darstellt, eine Hydrolyse anschließt, das Produkt isoliert und gegebenenfalls in Salz überführt.

46. Verfahren zur Herstellung der Verbindungen der Formel (I) nach Anspruch 1, worin R einen Rest CH-R₆ darstellt und R₆ einen Rest -alk-NR₁₄R₁₅ bedeutet, worin R₁₄ und R₁₅ jeweils ein Wasserstoffatom sind, mit Ausnahme von den Verbindungen, wo R₃ ein Rest Carboxamido ist, dadurch gekennzeichnet, daß man Brom und Natriumhydroxid mit einer entsprechenden Verbindung der Formel (I) zur Reaktion bringt, worin R einen Rest CH-R₆ darstellt und R₆ einen Rest -alk-CO-NR₁₀R₂₁ bedeutet, worin R₁₀ und R₂₁ jeweils ein Wasserstoffatom sind, das Produkt isoliert und gegebenenfalls in Salz überführt.

47. Verfahren zur Herstellung der Verbindungen der Formel (I) nach Anspruch 1, worin R einen Rest CH-R₆ darstellt und R₆ einen Rest -alk-NR₁₄R₁₅ bedeutet, worin R₁₄ und R₁₅ jeweils ein Wasserstoffatom sind und R₃ ein Rest Carboxamido ist, dadurch gekennzeichnet, daß man Ammoniak mit einer entsprechenden Verbindung der Formel (I) zur Reaktion bringt, worin R einen Rest CH-R₆ darstellt und R₆ einen Rest -alk-NR₁₄R₁₅ bedeutet, worin R₁₄ und R₁₅ jeweils ein Wasserstoffatom sind und R₃ einen Rest Alkoxycarbonyl darstellt, das Produkt isoliert und gegebenenfalls in Salz überführt.

48. Verfahren zur Herstellung der Verbindungen der Formel (I) nach Anspruch 1, worin R einen Rest CH-R₆ darstellt und R₆ einen Rest -NR₁₄R₁₅ oder -alk-NR₁₄R₁₅ bedeutet, worin R₁₄ ein Wasserstoffatom ist und R₁₅ einen Rest -COR₂₂ darstellt, R₂₂ einen Rest Alkyl, Cycloalkyl, -alk-COOR₁₀, Phenyl, gegebenenfalls substituiert, Phenylalkyl, dessen Phenylkern gegebenenfalls substituiert ist, Het, -alk-Het oder -alk-NR₁₀R₁₂ darstellt, dadurch gekennzeichnet, daß man eine entsprechende Verbindung der Formel (I), worin R einen Rest CH-R₆ darstellt und R₆ einen Rest -NR₁₄R₁₅ bedeutet, worin R₁₄ ein Wasserstoffatom ist und R₁₅ ein Wasserstoffatom ist, mit einer Säure HOOC-R₂₂ oder dem entsprechenden Anhydrid zur Reaktion bringt, worin R₂₂ einen Rest Alkyl, Cycloalkyl, Het, -alk-COOR₁₀, -alk-NR₁₀R₁₂, Phenylalkyl, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, -alk-Het, Phenyl, gegebenenfalls substituiert durch einen oder mehrere Substituenten, ausgewählt unter den Halogenatomen und den Resten Alkyl, Alkoxy, Nitro, Amino, Hydroxy, Cyano, -alk-NH₂, -COOR₁₀ und -alk-COOR₁₀, darstellt, worin alk, Het, R₁₀ und R₁₂ die gleichen Bedeutungen wie in Anspruch 1 besitzen, das Produkt isoliert und gegebenenfalls in Salz überführt.

49. Verfahren zur Herstellung der Verbindungen der Formel (I) nach Anspruch 1, worin R einen Rest CH-R₆ darstellt und R₆ ein Rest -alk-COOR₁₀ ist, dadurch gekennzeichnet, daß man ein entsprechendes Derivat der Formel (IX) 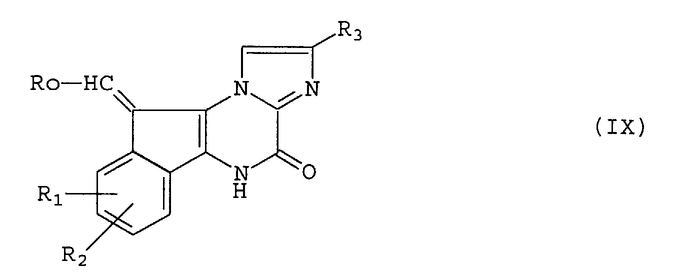 hydriert, worin R₁, R₂ und R₃ die gleichen Bedeutungen wie in Anspruch 1 besitzen und Ro einen Rest -alk(1-5C)-COOR₁₀ oder -COOR₁₀ darstellt, worin alk die gleichen Bedeutungen wie in Anspruch 1 besitzt und R₁₀ einen Rest Alkyl oder Phenylalkyl bedeutet, gegebenenfalls eine Verseifung des auf diese Weise erhaltenen Esters anschließt, das Produkt isoliert und gegebenenfalls in Salz überführt.

50. Verfahren zur Herstellung der Verbindungen der Formel (I) nach Anspruch 1, worin R einen Rest CH-R₆ darstellt und R₆ ein Rest -alk-CO-NR₁₀R₂₁ ist, dadurch gekennzeichnet, daß man eine entsprechende Verbindung der Formel (I), worin R einen Rest CH-R₆ darstellt und R₆ ein Rest -alk-COOR₁₀ ist, worin alk die gleichen Bedeutungen wie in Anspruch 1 besitzt und R₁₀ ein Wasserstoffatom oder einen Rest Alkyl darstellt, mit einem Amin HNR₁₀R₂₁ zur Reaktion bringt, worin R₁₀ und R₂₁ die gleichen Bedeutungen wie in Anspruch 1 besitzen, das Produkt isoliert und gegebenenfalls in Salz überführt.

51. Verfahren zur Herstellung der Verbindungen der Formel (I) nach Anspruch 1, worin R einen Rest CH-R₆ darstellt und R₆ ein Rest -alk(C1)-CO-NR₁₀R₂₁ ist, worin R₁₀ und R₂₁ jeweils Wasserstoffatome bedeuten, dadurch gekennzeichnet, daß man ein Derivat der Formel (IX) 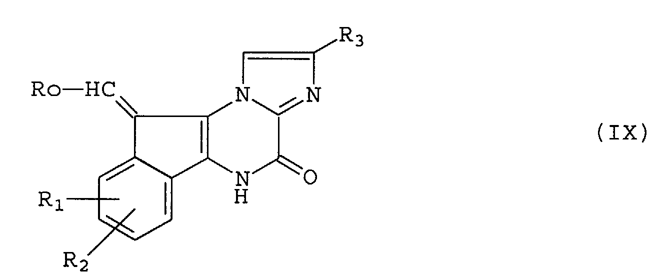 hydriert, worin R₁, R₂ und R₃ die gleichen Bedeutungen wie in Anspruch 1 besitzen und Ro einen Rest -CONH₂ darstellt, das Produkt isoliert und gegebenenfalls in Salz überführt.

52. Verfahren zur Herstellung der Verbindungen der Formel (I) nach Anspruch 1, worin R einen Rest CH-R₆ darstellt und R₆ ein Rest -R₁₆-COOR₁₀ ist, dadurch gekennzeichnet, daß man eine entsprechende Verbindung der Formel (I), worin R einen Rest CH-R₆ darstellt und R₆ ein Wasserstoffatom ist, mit einem Derivat der Formel OHC-alk(0-5C)-COOR₁₀ zur Reaktion bringt, worin alk und R₁₀ die gleichen Bedeutungen wie in Anspruch 1 besitzen, das Produkt isoliert und gegebenenfalls in Salz überführt.

53. Verfahren zur Herstellung der Verbindungen der Formel (I) nach Anspruch 1, worin R einen Rest CH-R₆ darstellt und R₆ ein Rest -NH-CHO ist, dadurch gekennzeichnet, daß man eine entsprechende Verbindung der Formel (I), worin R einen Rest CH-R₆ darstellt und R₆ ein Rest -NR₁₄R₁₅ ist, worin R₁₄ und R₁₅ Wasserstoffatome bedeuten, mit CH₃COOCHO zur Reaktion bringt, das Produkt isoliert und gegebenenfalls in Salz überführt.

54. Verfahren zur Herstellung der Verbindungen der Formel (I) nach Anspruch 1, worin R einen Rest CH-R₆ darstellt und R₆ ein Rest -CO-COOR₁₀ ist, dadurch gekennzeichnet, daß man eine entsprechende Verbindung der Formel (I) oxidiert, worin R einen Rest CH-R₆ darstellt und R₆ ein Rest -R₁₆-COOR₁₀ ist, worin R₁₆ einen Rest -CHOH- bedeutet, gegebenenfalls eine Veresterung anschließt, das Produkt isoliert und gegebenenfalls in Salz überführt.

55. Verfahren zur Herstellung der Verbindungen der Formel (I) nach Anspruch 1, worin R einen Rest CH-R₆ darstellt und R₆ ein Rest Pyrrol-1-yl ist, gegebenenfalls substituiert durch einen Rest -COOR₁₀, dadurch gekennzeichnet, daß man eine entsprechende Verbindung der Formel (I), worin R einen Rest CH-R₆ darstellt und R₆ ein Rest -NR₁₄R₁₅ ist, worin R₁₄ und R₁₅ Wasserstoffatome bedeuten, mit einem Derivat der Formel (VIII) 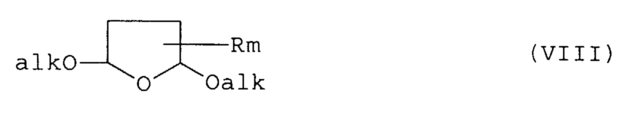 zur Reaktion bringt, worin Rm ein Wasserstoffatom oder ein Rest -COOR₁₀ ist und R₁₀ die gleichen Bedeutungen wie in Anspruch 1 besitzt, das Produkt isoliert und gegebenenfalls in Salz überführt.

56. Verfahren zur Herstellung der Verbindungen der Formel (I) nach Anspruch 1, worin R einen Rest CH-R₆ darstellt und R₆ ein Rest -COOalk ist, dadurch gekennzeichnet, daß man eine Mineralsäure mit einem Derivat der Formel (X) 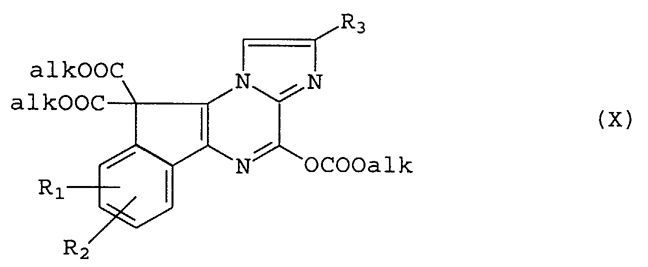 zur Reaktion bringt, worin R₁, R₂ und R₃ die gleichen Bedeutungen wie in Anspruch 1 besitzen und alk einen Rest Alkyl darstellt, das Produkt isoliert und gegebenenfalls in Salz überführt.

57. Verfahren zur Herstellung der Verbindungen der Formel (I) nach Anspruch 1, worin R₃ einen Rest Carboxy darstellt, dadurch gekennzeichnet, daß man eine entsprechende Verbindung der Formel (I), worin R₃ einen Rest Alkoxycarbonyl darstellt, hydrolysiert, das Produkt isoliert und gegebenenfalls in Salz überführt.

58. Verfahren zur Herstellung der Verbindungen der Formel (I) nach Anspruch 1, worin R₁ und/oder R₂ einen Rest -NH-CO-NR₁₁R₁₂ darstellen, worin R₁₁ ein Wasserstoffatom oder einen Rest Alkyl (1 bis 9 Kohlenstoffatome in gerader oder verzweigter Kette), -alk-COOR₁₀, -alk-Het, -alk-NR₁₂R₁₀, Phenylalkyl, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, -alk-NH₂, Carboxy, Alkoxycarbonyl, Cyano und -alk-COOR₁₀, Phenyl, gegebenenfalls substituiert durch einen oder mehrere Substituenten, ausgewählt unter den Halogenatomen und den Resten Alkyl, Alkoxy, Nitro, Amino, Hydroxy, -alk-NH₂, Carboxy, Alkoxycarbonyl, Cyano und -alk-COOR₁₀, oder Het darstellt und R₁₂ ein Wasserstoffatom ist, dadurch gekennzeichnet, daß man eine entsprechende Verbindung der Formel (I), worin R₁ und/oder R₂ einen Rest Amino bedeuten, mit einem Isocyanat O=C=NR₁₁ zur Reaktion bringt, worin R₁₁ die gleichen Bedeutungen wie vorstehend besitzt oder einen Rest Trimethylsilyl darstellt, das Produkt isoliert und gegebenenfalls in Salz überführt.

59. Verfahren zur Herstellung der Verbindungen der Formel (I) nach Anspruch 1, worin R₁ und/oder R₂ einen Rest Amino darstellen, dadurch gekennzeichnet, daß man eine entsprechende Verbindung der Formel (I) reduziert, worin R₁ und/oder R₂ einen Rest Nitro bedeuten, das Produkt isoliert und gegebenenfalls in Salz überführt.

60. Verfahren zur Herstellung der Verbindungen der Formel (I) nach Anspruch 1, worin R einen Rest CH-R₆ darstellt und R₆ ein Rest -NR₁₄R₁₅ ist, worin R₁₄ und R₁₅ Wasserstoffatome darstellen, dadurch gekennzeichnet, daß man in Anwesenheit eines Reduktionsmittels eine entsprechende Verbindung der Formel (I), worin R einen Rest C=R₇ darstellt und R₇ ein Rest NOH ist, reduziert, das Produkt isoliert und gegebenenfalls in Salz überführt.

61. Verfahren zur Herstellung der Verbindungen der Formel (I) nach Anspruch 1, worin R einen Rest C=R₇ darstellt und R₇ ein Rest CH-R₁₉ ist, worin R₁₉ einen Rest Phenyl bedeutet, substituiert durch einen Rest -COOR₁₀ und R₁₀ ein Wasserstoffatom ist, dadurch gekennzeichnet, daß man eine entsprechende Verbindung der Formel (I) hydrolysiert, worin R einen Rest C=R₇ darstellt und R₇ ein Rest CH-R₁₉ ist, worin R₁₉ einen Rest Phenyl bedeutet, substituiert durch einen Rest Cyano, das Produkt isoliert und gegebenenfalls in Salz überführt.

62. Verfahren zur Herstellung der Verbindungen der Formel (I) nach Anspruch 1, worin R einen Rest CH-R₆ darstellt und R₆ ein Rest 2-Oxo-2,5-dihydropyrrol-1-yl ist, dadurch gekennzeichnet, daß man eine entsprechende Verbindung der Formel (I), worin R einen Rest CH-R₆ darstellt und R₆ ein Rest -NR₁₄R₁₅ ist, worin R₁₄ und R₁₅ Wasserstoffatome bedeuten, mit 2,5-Dimethoxy-2,5-dihydrofuran zur Reaktion bringt, das Produkt isoliert und gegebenenfalls in Salz überführt.

63. Verbindungen der Formel (I) nach Anspruch 1, worin R einen Rest CH-R₆, C=R₇ oder C(R₄)R₅ darstellt, R₁ ein Wasserstoffatom oder Halogenatom oder einen Rest -NH-CO-NR₁₁R₁₂ bedeutet, worin R₁₁ ein Wasserstoffatom ist und R₁₂ einen Rest Alkyl darstellt, R₂ ein Wasserstoffatom ist, R₃ einen Rest Carboxy, Alkoxycarbonyl oder Carboxamido bedeutet, R₄ ein Rest Alkyl ist, R₅ einen Rest -alk-COOR₁₀ darstellt, R₆ ein Wasserstoffatom oder einen Rest -alk-Het, 2-Oxo-2,5-dihydropyrrol-1-yl, Pyrrol-1-yl, substituiert durch -COOR₁₀ oder -NR₁₄R₁₅, bedeutet, R₇ ein Rest CH-R₁₉, NO-alk-COOR₁₀ oder NOH ist, R₁₉ einen Rest Het oder Phenyl, substituiert durch -COOR₁₀, darstellt, entweder R₁₄ und R₁₅ Wasserstoffatome sind oder R₁₄ ein Wasserstoffatom ist und R₁₅ einen Rest -COR₂₂ darstellt, worin R₂₂ einen Rest -alk-COOR₁₀ oder -NH-Ar bedeutet, und ihre Salze.

64. Arzneimittel, enthaltend als Wirkstoff mindestens eine Verbindung der Formel (I) nach Anspruch 1, 2 oder 63 oder ein Salz einer derartigen Verbindung.

## Claims

1. Compounds of formula: 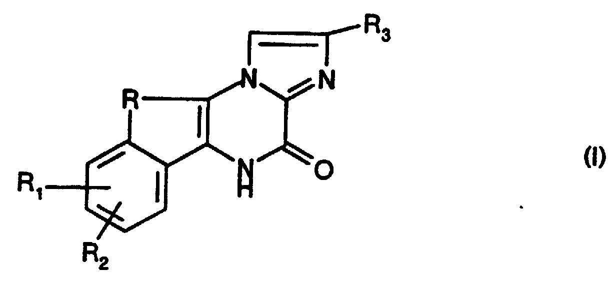 in which,
- R denotes an N-alk, C(R₄)R₅, CH-R₆ or C=R₇ radical,
- R₁ and R₂, which are identical or different, denote hydrogen or halogen atoms or radicals which are alkyl, alkoxy, amino, -N=CH-N(alk)alk', nitro, cyano, phenyl, imidazolyl, SO₃H, hydroxyl, polyfluoroalkoxy, carboxyl, alkoxycarbonyl, -NH-CO-NR₁₁R₁₂, -N(alk)-CO-NR₁₁R₁₂, -N(alk-Ar)-CO-NR₁₁R₁₂, -NH-CS-NR₁₁R₁₂, -N(alk)-CS-NR₁₁R₁₂, -NH-CO-R₁₁, -NH-CS-R₂₄, -NH-C(=NR₂₇)-NR₁₀R₁₂, -N(alk)-C(=NR₂₇)-NR₁₀R₁₂, -CO-NR₁₀R₁₂, -NH-SO₂-NR₁₀R₁₂, -N(alk)-SO₂-NR₁₀R₁₂, -NH-SO₂-CF₃, -NH-SO₂-alk, -NR₁₀R₁₃, -S(O)ₘ-alk-Ar, -SO₂-NR₁₀R₁₂, 2-oxo-1-imidazolidinyl in which position 3 is optionally substituted by an alkyl radical or 2-oxo-l-perhydropyrimidinyl in which position 3 is optionally substituted by an alkyl radical,
- R₃ denotes a carboxyl, alkoxycarbonyl or carboxamido radical,
- R₄ denotes an alkyl, -alk-Het or phenylalkyl radical in which the phenyl nucleus is optionally substituted by one or more substituents chosen from halogen atoms and alkyl, alkoxy, nitro, amino, hydroxyl, cyano, -alk-NH₂, -COOR₁₀ and -alk-COOR₁₀ radicals,
- R₅ denotes a radical which is alkyl (1-11 C as straight or branched chain), -alk-Het, -NR₈R₉, -NH-CHO, -NH-COOR₁₇, -NH-SO₂R₂₄, -COOR₁₀, -alk-COOR₁₀, -alk-CONR₁₀R₁₈, -alk-NR₁₀R₁₈, -alk-OH, -alk-CN, phenylalkyl in which the phenyl nucleus is optionally substituted by one or more substituents chosen from halogen atoms and alkyl, alkoxy, nitro, amino, hydroxyl, cyano, -alk-NH₂, -COOR₁₀ and -alk-COOR₁₀ radicals, -NH-CO-Ar in which Ar is optionally substituted by one or more substituents chosen from halogen atoms and alkyl, alkoxy, nitro, amino, hydroxyl, cyano, -alk-NH₂, -COOR₁₀ and -alk-COOR₁₀ radicals, -NH-CO-Het, -NH-CO-alk-Het, -NH-CO-alk-COOR₁₀, -NH-CO-alk-NR₁₀R₁₈, -NH-CO-alk-Ar in which Ar is optionally substituted by one or more substituents chosen from halogen atoms and alkyl, alkoxy, nitro, amino, hydroxyl, cyano, -alk-NH₂, -COOR₁₀ and -alk-COOR₁₀ radicals, 1-pyrrolyl optionally substituted by a -COOR₁₀ radical, -NH-CO-NH-alk-Ar in which Ar is optionally substituted by one or more substituents chosen from halogen atoms and alkyl, alkoxy, nitro, amino, hydroxyl, cyano, -alk-NH₂, -COOR₁₀ and -alk-COOR₁₀ radicals, -NH-CO-NH-Het, -NH-CO-NH-alk-Het, -NH-CO-NH-Ar in which Ar is optionally substituted by one or more substituents chosen from halogen atoms and alkyl, alkoxy, nitro, amino, hydroxyl, cyano, -alk-NH₂, -COOR₁₀ and -alk-COOR₁₀ radicals, -NH-COalk, -NH-COcycloalkyl, -NH-CO-NH-alk or -NH-CO-NH₂,
or else R₄ and R₅ form, together with the carbon atom to which they are attached, a cycloalkyl radical,
- R₆ denotes a hydrogen atom or a radical which is hydroxyl, alkyl (1-11 C as straight or branched chain), -alk-OH, -NR₁₄R₁₅, -alk-NR₁₄R₁₅, -alk-Het, -NH-CHO, -COOalk, alk-COOR₁₀, -alk-CO-NR₁₀R₂₁, phenylalkyl in which the phenyl nucleus is optionally substituted by one or more substituents chosen from halogen atoms and alkyl, alkoxy, nitro, amino, hydroxyl, cyano, -alk-NH₂, -COOR₁₀ and -alk-COOR₁₀ radicals, -R₁₆-COOR₁₀, -CO-COOR₁₀, 1-pyrrolyl optionally substituted by a -COOR₁₀ or 2-oxo-2,5-dihydropyrrol-1-yl radical,
- R₇ denotes an oxygen atom or an NOH, NO-alk-COOR₁₀, NO-alk, CHR₁₉, NR₁₀, C(COOR₁₀)R₂₀ or C(CONR₁₀R₂₁)R₂₀ radical,
- R₈ denotes a hydrogen atom or an alkyl, -alk-COOR₁₀, -alk-NR₁₀R₂₁, -alk-Het or phenylalkyl radical in which the phenyl nucleus is optionally substituted by one or more substituents chosen from halogen atoms and alkyl, alkoxy, nitro, amino, hydroxyl, cyano, -alk-NH₂, -COOR₁₀ and -alk-COOR₁₀ radicals,
- R₉ denotes a hydrogen atom or an alkyl radical,
- R₁₀ denotes a hydrogen atom or an alkyl radical,
- R₁₁ denotes a hydrogen atom or radical which is alkyl (1-9 C as straight or branched chain), -alk-COOR₁₀, -alk-Het, -alk-NR₁₂R₁₀ or phenylalkyl in which the phenyl nucleus is optionally substituted by one or more substituents chosen from halogen atoms and alkyl, alkoxy, nitro, amino, hydroxyl, -alk-NH₂, carboxyl, alkoxycarbonyl, cyano and -alk-COOR₁₀ radicals, phenyl optionally substituted by one or more substituents chosen from halogen atoms and alkyl, alkoxy, nitro, amino, hydroxyl, -alk-NH₂, carboxyl, alkoxycarbonyl, cyano and -alk-COOR₁₀ radicals or -Het,
- R₁₂ denotes a hydrogen atom or an alkyl radical,
- R₁₃ denotes an alkyl, Het or alkoxycarbonyl radical,
- each of R₁₄ and R₁₅, which are identical or different, denotes an alkyl radical or else R₁₄ denotes a hydrogen atom and R₁₅ denotes a hydrogen atom or an alkyl, -COR₂₂, -CSR₂₃ or -SO₂R₂₄ radical,
- R₁₆ denotes a -CHOH- or -CH(OH)-(1-5 C)alk- chain,
- R₁₇ denotes an alkyl or phenylalkyl radical,
- R₁₈ denotes a hydrogen atom or an alkyl radical,
- R₁₉ denotes a hydroxyl, alkyl, -alk-Het, -NR₂₅R₂₆, -alk-COOR₁₀, -Het or phenyl radical optionally substituted by one or more substituents chosen from halogen atoms and alkyl, alkoxy, nitro, amino, hydroxyl, -alk-NH₂, -COOR₁₀, cyano and -alk-COOR₁₀ radicals or phenylalkyl in which the phenyl nucleus is optionally substituted by one or more substituents chosen from halogen atoms and alkyl, alkoxy, nitro, amino, hydroxyl, -alk-NH₂, -COOR₁₀, cyano and -alk-COOR₁₀ radicals,
- R₂₀ denotes a hydrogen atom or an alkyl radical,
- R₂₁ denotes a hydrogen atom or an alkyl radical,
- R₂₂ denotes an alkyl, cycloalkyl, -COOalk, -alk-COOR₁₀ or phenyl radical optionally substituted by one or more substituents chosen from halogen atoms and alkyl, alkoxy, nitro, amino, hydroxyl, -alk-NH₂, -COOR₁₀, cyano and -alk-COOR₁₀ radicals, phenylalkyl in which the phenyl nucleus is optionally substituted by one or more substituents chosen from halogen atoms and alkyl, alkoxy, nitro, amino, hydroxyl, -alk-NH₂, -COOR₁₀, cyano and -alk-COOR₁₀ radicals, -alk-NR₁₀R₁₂, -NH-Ar in which Ar is optionally substituted by one or more substituents chosen from halogen atoms and alkyl, alkoxy, nitro, amino, hydroxyl, -alk-NH₂, -COOR₁₀, cyano and -alk-COOR₁₀ radicals, -Het, -alk-Het, -OR₁₇, -NH-alk-Ar in which Ar is optionally substituted by one or more substituents chosen from halogen atoms and alkyl, alkoxy, nitro, amino, hydroxyl, -alk-NH₂, -COOR₁₀, cyano and -alk-COOR₁₀ radicals, -NH-alk-Het, -NH-alk, -NH₂ or -NH-Het,
- R₂₃ denotes an -NH-alk, -NH-Ar, -NH-Het or -NH₂ radical,
- R₂₄ denotes an alkyl or phenyl radical,
- each of R₂₅ and R₂₆, which are identical or different, denotes an alkyl or cycloalkyl radical,
- R₂₇ denotes a hydrogen atom or an alkyl radical,
- alk denotes an alkyl or an alkylene radical,
- alk' denotes an alkyl radical,
- m is equal to 0, 1 or 2,
- Ar denotes a phenyl radical,
- Het denotes a saturated or unsaturated mono- or polycyclic heterocyclic ring containing 1 to 9 carbon atoms and one or more heteroatoms (O, S, N), which is optionally substituted by one or more alkyl, phenyl or phenylalkyl radicals,
it being understood that the alkyl, alkylene and alkoxy radicals and moieties contain 1 to 6 carbon atoms and form a straight or branched chain, the acyl radicals and moieties contain 2 to 4 carbon atoms and the cycloalkyl radicals contain 3 to 6 carbon atoms,
and in the case of the compounds in which R₇ denotes an NO-alk, C(COOR₁₀)R₂₀ or C(CONR₁₀R₂₁)R₂₀, CHR₁₉ radical, their isomers (E and Z), in the case of the compounds in which R denotes a CH-R₆ radical and R₆ denotes a -CO-COOR₁₀ radical their tautomeric (E and Z) forms, and in the case of the compounds in which R denotes a C(R₄)R₅ or CH-R₆ radical their enantiomers and diastereoisomers and the salts of these compounds.

2. Compounds of formula (I) according to claim 1 in which Het is chosen from rings which are pyrrolyl optionally substituted by one or more alkyl, phenyl or phenylalkyl radicals, pyridyl optionally substituted by one or more alkyl, phenyl or phenylalkyl radicals, pyrimidinyl optionally substituted by one or more alkyl, phenyl or phenylalkyl radicals, imidazolyl optionally substituted by one or more alkyl, phenyl or phenylalkyl radicals, thiazolyl optionally substituted by one or more alkyl, phenyl or phenylalkyl radicals, oxazolinyl optionally substituted by one or more alkyl, phenyl or phenylalkyl radicals, thiazolinyl optionally substituted by one or more alkyl, phenyl or phenylalkyl radicals, pyrazinyl optionally substituted by one or more alkyl, phenyl or phenylalkyl radicals, tetrazolyl optionally substituted by one or more alkyl, phenyl or phenylalkyl radicals or triazolyl optionally substituted by one or more alkyl, phenyl or phenylalkyl radicals.

3. Process for the preparation of compounds of formula (I) according to claim 1, in which R denotes an N-alk or CH-R₆ radical, R₆ denotes a hydrogen atom and R₃ denotes an alkoxycarbonyl radical with the exception of tert-butoxycarbonyl, characterized in that a derivative of formula: 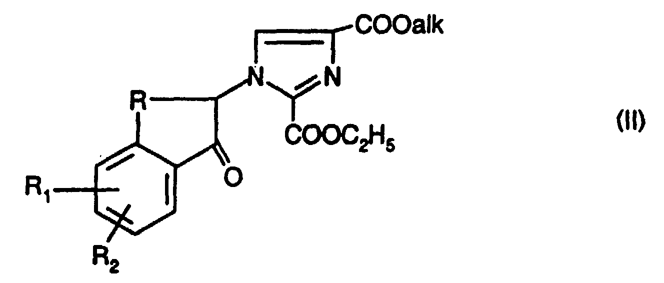 in which R denotes an N-alk or CH-R₆ radical, R₆ denotes a hydrogen atom, R₁ and R₂ have the same meanings as in claim 1 and -COOalk denotes an alkoxycarbonyl radical except for tert-butoxycarbonyl, is cyclized in the presence of ammonium acetate, the product is isolated and is optionally converted into salt.

4. Process for the preparation of compounds of formula (I) according to claim 1, in which R denotes an N-alk or CH-R₆ radical, R₆ denotes a hydrogen atom and R₃ denotes a tert-butoxycarbonyl radical, characterized in that isobutene is reacted with a corresponding compound of formula (I) in which R denotes an N-alk or CH-R₆ radical, R₆ denotes a hydrogen atom and R₃ denotes a carboxyl radical, the product is isolated and is optionally converted into salt.

5. Process for the preparation of compounds of formula (I) according to claim 1, in which R denotes an N-alk or CH-R₆ radical, R₆ denotes a hydrogen atom and R₃ denotes a carboxyl radical, characterized in that a derivative of formula: 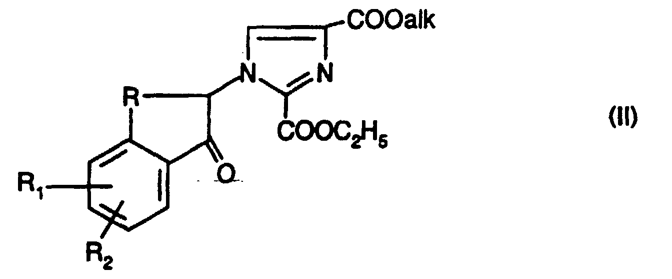 in which R denotes an N-alk or CH-R₆ radical, R₆ denotes a hydrogen atom and COOalk denotes a tert-butoxycarbonyl radical is cyclized in the presence of ammonium acetate, the product is isolated and is optionally converted into salt.

6. Process for the preparation of compounds of formula (I) according to claim 1, in which R denotes an N-alk or CH-R₆ radical, R₆ denotes a hydrogen atom and R₃ denotes a carboxamido radical, characterized in that a derivative of formula: 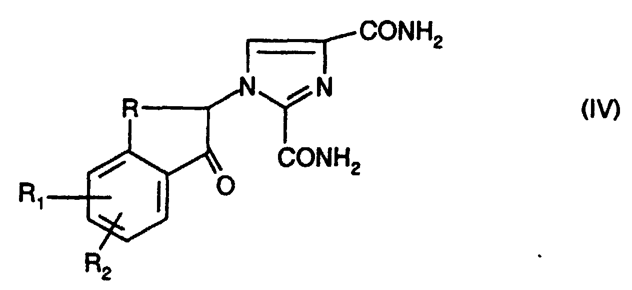 in which R denotes an N-alk or CH-R₆ radical, R₆ denotes a hydrogen atom and R₁ and R₂ have the same meanings as in claim 1 is cyclized, the product is isolated and is optionally converted into salt.

7. Process for the preparation of compounds of formula (I) according to claim 1, in which R denotes a C=R₇ radical in which R₇ denotes an oxygen atom, characterized in that a corresponding compound of formula (I) in which R denotes a C=R₇ radical and R₇ denotes an NOH radical is hydrolysed, followed in the case of the compounds in which R₃ denotes an alkoxy-carbonyl radical by an esterification of the corresponding acid, the product is isolated and is optionally converted into salt.

8. Process for the preparation of compounds of formula (I) according to claim 1, in which R denotes a C=R₇ radical and R₇ denotes an NOH radical, characterized in that an alkyl nitrite is reacted with a corresponding compound of formula (I) in which R denotes a CH-R₆ radical and R₆ denotes a hydrogen atom, the product is isolated and is optionally converted into salt.

9. Process for the preparation of compounds of formula (I) according to claim 1, in which R denotes a C=R₇ radical and R₇ denotes an NO-alk-COOR₁₀ or NO-alk radical, characterized in that a corresponding compound of formula (I) in which R denotes a C=R₇ radical and R₇ denotes an NOH radical is reacted with a halide Hal-Ra in which Hal denotes a halogen atom and Ra denotes an alkyl or -alk-COOR₁₀ radical, alk and R₁₀ having the same meanings as in claim 1, the product is isolated and is optionally converted into salt.

10. Process for the preparation of compounds of formula (I) according to claim 1, in which R denotes a C=R₇ radical and R₇ denotes a CH-R₁₉ radical in which R₁₉ denotes a hydroxyl radical, characterized in that a corresponding compound of formula (I) in which R₁₉ denotes an -NR₂₅R₂₆ radical is hydrolysed, fqllowed in the case of the compounds in which R₃ denotes an alkoxy-carbonyl radical by an esterification of the corresponding acid, the product is isolated and is optionally converted into salt.

11. Process for the preparation of compounds of formula (I) according to claim 1, in which R denotes a C=R₇ radical and R₇ denotes a CH-R₁₉ radical in which R₁₉ denotes an -NR₂₅R₂₆ radical, characterized in that a corresponding compound of formula (I) in which R denotes a -CH-R₆ radical and R₆ denotes a hydrogen atom is reacted with a derivative HC(Rb)(Rc)Rd in which either each of Rb and Rd, which are identical or different, denotes an -NR₂₅R₂₆ radical in which R₂₅ and R₂₆ have the same meanings as in claim 1 and Rc denotes an alkoxy radical, or each of Rb, Rc and Rd, which are identical, denotes an -NR₂₅R₂₆ radical in which R₂₅ and R₂₆ have the same meanings as in claim 1, the product is isolated and is optionally converted into salt.

12. Process for the preparation of compounds of formula (I) according to claim 1, in which R denotes a C=R₇ radical, R₇ denotes a CHR₁₉ radical and R₁₉ denotes a radical which is alkyl, optionally substituted phenyl, -alk-Het, phenylalkyl in which the phenyl nucleus is optionally substituted, -Het or -alk-COOR₁₀, characterized in that a corresponding compound of formula (I) in which R denotes a CH-R₆ radical and R₆ denotes a hydrogen atom is reacted with an aldehyde of formula OHC-Re in which Re denotes a radical which is alkyl, phenyl optionally substituted by one or more substituents chosen from halogen atoms and alkyl, alkoxy, nitro, amino, hydroxyl, -alk-NH₂, -COOR₁₀, cyano and -alk-COOR₁₀ radicals, -alk-Het, phenylalkyl in which the phenyl nucleus is optionally substituted by one or more substituents chosen from halogen atoms and alkyl, alkoxy, nitro, amino, hydroxyl, -alk-NH₂, -COOR₁₀, cyano and -alk-COOR₁₀ radicals, -Het or -alk-COOR₁₀ in which alk, Het and R₁₀ have the same meanings as in claim 1, the product is isolated and is optionally converted into salt.

13. Process for the preparation of compounds of formula (I) according to claim 1, in which R denotes a C=R₇ radical and R₇ denotes an NR₁₀ radical, characterized in that ethyl trifluoroacetate is reacted with a derivative of formula: 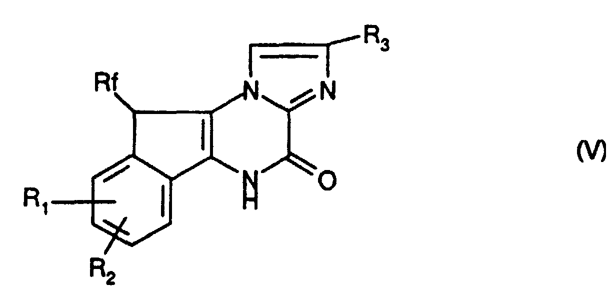 in which R₁, R₂ and R₃ have the same meanings as in claim 1 and Rf denotes an -NH₂ or -NH-alk radical, alk having the same meanings as in claim 1, the product is isolated and is optionally converted into salt.

14. Process for the preparation of compounds of formula (I) according to claim 1, in which R denotes a C=R₇ radical and R₇ denotes a C(COOR₁₀)R₂₀ radical, characterized in that a compound of formula: 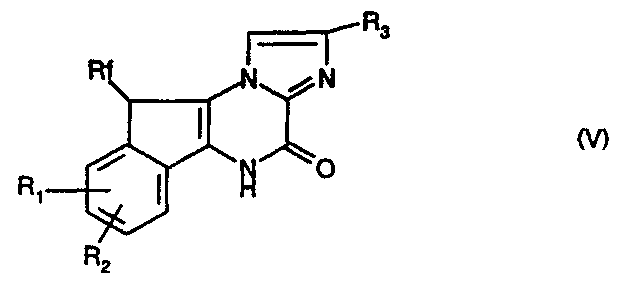 in which R₁, R₂ and R₃ have the same meanings as in claim 1 and Rf denotes a -C(R₂₀)(OH)-COOR₁₀ radical in which R₂₀ and R₁₀ have the same meanings as in claim 1 is dehydrated, the product is isolated and is optionally converted into salt.

15. Process for the preparation of compounds of formula (I) according to claim 1, in which R denotes a C=R₇ radical and R₇ denotes a C(CONR₁₀R₂₁)R₂₀ radical, characterized in that a corresponding compound of formula (I) in which R denotes a C=R₇ radical and R₇ denotes a C(COOR₁₀)R₂₀ radical is reacted with an amine HNR₁₀R₂₁ in which R₁₀ and R₂₁ have the same meanings as in claim 1, the product is isolated and is optionally converted into salt.

16. Process for the preparation of compounds of formula (I) according to claim 1, in which R denotes a C(R₄)R₅ radical, R₄ denotes an alkyl, -alk-Het or phenylalkyl radical in which the phenyl nucleus is optionally substituted by one or more substituents chosen from halogen atoms and alkyl, alkoxy, nitro, amino, hydroxyl, cyano, -alk-NH₂, -COOR₁₀ and -alk-COOR₁₀ radicals and R₅ is identical with R₄, characterized in that a corresponding compound of formula (I) in which R denotes a CH-R₆ radical and R₆ denotes a hydrogen atom is reacted with a halide of formula Hal-Rg, in which Rg denotes an alkyl, -alk-Het or phenylalkyl radical in which the phenyl nucleus is optionally substituted by one or more substituents chosen from halogen atoms and alkyl, alkoxy, nitro, amino, hydroxyl, cyano, -alk-NH₂, -COOR₁₀ and -alk-COOR₁₀ radicals, alk, Het and R₁₀ having the same meanings as in claim 1, the product is isolated and is optionally converted into salt.

17. Process for the preparation of compounds of formula (I) according to claim 1, in which R denotes a C(R₄)R₅ radical, R₄ denotes an alkyl, -alk-Het or phenylalkyl radical in which the phenyl nucleus is optionally substituted by one or more substituents chosen from halogen atoms and alkyl, alkoxy, nitro, amino, hydroxyl, cyano, -alk-NH₂, -COOR₁₀ and -alk-COOR₁₀ radicals and R₅ denotes a radical which is alkyl (1-11 C as a straight or branched chain), -alk-CN, -alk-Het, -alk-NR₁₀R₁₈, -alk-CO-NR₁₀R₁₈ or phenylalkyl in which the phenyl nucleus is optionally substituted by one or more substituents chosen from halogen atoms and alkyl, alkoxy, nitro, amino, hydroxyl, cyano, -alk-NH₂, -COOR₁₀ and -alk-COOR₁₀ radicals, characterized in that a corresponding compound of formula (I) in which R denotes a CH-R₆ radical and R₆ denotes an alkyl, -alk-Het or phenylalkyl radical in which the phenyl nucleus is optionally substituted by one or more substituents chosen from halogen atoms and alkyl, alkoxy, nitro, amino, hydroxyl, cyano, -alk-NH₂, -COOR₁₀ and -alk-COOR₁₀ radicals is reacted with a halide Hal-Rh in which Rh denotes a radical which is alkyl (1-11 C as a straight or branched chain), -alk-Het, -alk-CN, -alk-NR₁₀R₁₈, -alk-CO-NR₁₀R₁₈ or phenylalkyl radical in which the phenyl nucleus is optionally substituted by one or more substituents chosen from halogen atoms and alkyl, alkoxy, nitro, amino, hydroxyl, cyano, -alk-NH₂, -COOR₁₀ and -alk-COOR₁₀ radicals, alk, Het, R₁₀ and R₁₈ having the same meanings as in claim 1, the product is isolated and is optionally converted into salt.

18. Process for the preparation of compounds of formula (I) according to claim 1, in which R denotes a C(R₄)R₅ radical and R₄ and R₅ form, with the carbon atom to which they are attached, a cycloalkyl radical, characterized in that a corresponding compound of formula (I) in which R denotes a CH-R₆ radical and R₆ denotes a hydrogen atom is reacted with a derivative of formula Hal-alk-Hal in which Hal denotes a halogen atom and alk denotes an alkyl (2-5 C) radical, the product is isolated and is optionally converted into salt.

19. Process for the preparation of compounds of formula (I) according to claim 1, in which R denotes a C(R₄)R₅ radical in which R₅ denotes an -NR₈R₉ radical and R₈ and R₉ denote hydrogen atoms, characterized in that a halide Hal-R₄ in which Hal denotes a halogen atom and R₄ has the same meanings as in claim 1 is reacted with a corresponding compound of formula (I) in which R denotes a CH-R₆ radical, R₆ denotes an -NR₁₄R₁₅ radical, R₁₄ denotes a hydrogen atom, R₁₅ denotes a -COR₂₂ radical and R₂₂ denotes an alkyl radical (1 C), followed by a hydrolysis, the product is isolated and is optionally converted into salt.

20. Process for the preparation of compounds of formula (I) according to claim 1, in which R denotes a C(R₄)R₅ radical in which R₅ denotes an -NR₈R₉ radical, R₉ denotes a hydrogen atom and R₈ denotes an alkyl, -alk-COOR₁₀, -alk-NR₁₀R₂₁, -alk-Het or phenylalkyl radical in which the phenyl nucleus is optionally substituted, characterized in that a corresponding compound of formula (I) in which R denotes a C(R₄)R₅ radical, R₅ denotes an -NR₈R₉ radical and R₈ and R₉ denote hydrogen atoms is reacted with a halide Hal-R₈ in which R₈ has the same meanings as above, the product is isolated and is optionally converted into salt.

21. Process for the preparation of compounds of formula (I) according to claim 1, in which R denotes a C(R₄)R₅ radical in which R₅ denotes an -NR₈R₉ radical, R₉ denotes a hydrogen atom or an alkyl radical and R₈ denotes an alkyl (2-6 C) radical, characterized in that a corresponding compound of formula (I) in which R denotes a C(R₄)R₅ radical, R₅ denotes an -NR₈R₉ radical, R₉ denotes a hydrogen atom or an alkyl radical and R₈ denotes a hydrogen atom is reacted with an acyl (2-6 C) halide, followed by reduction of the product obtained, the product is isolated and is optionally converted into salt.

22. Process for the preparation of compounds of formula (I) according to claim 1, in which R denotes a C(R₄)R₅ radical in which R₅ denotes an -NR₈R₉ radical and R₉ denotes an alkyl radical, characterized in that a corresponding compound of formula (I) in which R denotes a C(R₄)R₅ radical, R₅ denotes an -NR₈R₉ radical, R₈ has the same meanings as in claim 1 and R₉ denotes a hydrogen atom is reacted with a derivative of the formula Hal-alk in which Hal denotes a halogen atom and alk denotes an alkyl radical, the product is isolated and is optionally converted into salt.

23. Process for the preparation of compounds of formula (I) according to claim 1, in which R denotes a C(R₄)R₅ radical in which R₅ denotes an -NR₈R₉ radical, R₉ denotes a hydrogen atom or an alkyl radical and R₈ denotes a -(2-6 C)alk-NR₁₀R₂₁ radical, characterized in that a derivative of formula: 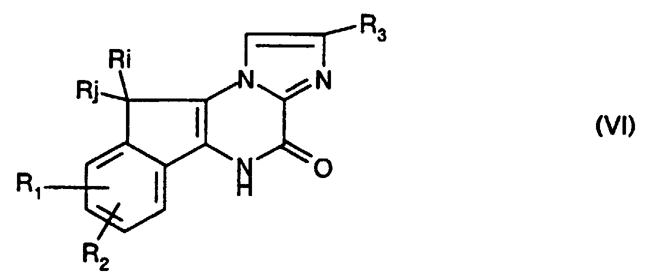 in which R₁, R₂ and R₃ have the same meanings as in formula (I), Ri denotes a radical which is alkyl, phenylalkyl in which the phenyl nucleus is optionally substituted by one or more substituents chosen from halogen atoms and alkyl, alkoxy, nitro, amino, hydroxyl, cyano, -alk-NH₂, -COOR₁₀ and -alk-COOR₁₀ or -alk-Het and Rj denotes an -NH-CO-(1-5 C)alk-NR₁₀R₂₁ or -N(alk)-CO-(1-5 C)alk-NR₁₀R₂₁ radical in which alk, Het, R₁₀ and R₂₁ have the same meanings as in claim 1, is reduced, the product is isolated and is optionally converted into salt.

24. Process for the preparation of compounds of formula (I) according to claim 1, in which R denotes a C(R₄)R₅ radical in which R₅ denotes a -COOR₁₀ radical and R₁₀ denotes a hydrogen atom, characterized in that a corresponding compound of formula (I) in which R denotes a C(R₄)R₅ radical in which R₅ denotes a -COOR₁₀ radical and R₁₀ denotes an alkyl radical is hydrolysed, the product is isolated and is optionally converted into salt.

25. Process for the preparation of compounds of formula (I) according to claim 1, in which R denotes a C(R₄)R₅ radical in which R₅ denotes a -COOR₁₀ radical and R₁₀ denotes an alkyl radical, characterized in that a corresponding compound of formula (I) in which R denotes a CH-R₆ radical and R₆ denotes an alkyl, -alk-Het or optionally substituted phenylalkyl radical is reacted with a halide of formula Hal-COOR₁₀ in which R₁₀ denotes an alkyl radical and Hal denotes a halogen atom, the product is isolated and is optionally converted into salt.

26. Process for the preparation of compounds of formula (I) according to claim 1, in which R denotes a C(R₄)R₅ radical in which R₅ denotes an -alk-COOR₁₀ radical, characterized in that a corresponding compound of formula (I) in which R denotes a CH-R₆ radical, R₆ denotes an -alk-COOR₁₀ radical and R₁₀ has the same meanings as in claim 1 is reacted with a halide Hal-R₄ in which R₄ has the same meanings as in claim 1, the product is isolated and is optionally converted into salt.

27. Process for the preparation of compounds of formula (I) according to claim 1, in which R denotes a C(R₄)R₅ in which R₅ denotes an -alk-CN radical in which alk contains 1 carbon atom, characterized in that sodium cyanide is reacted with a derivative of formula: 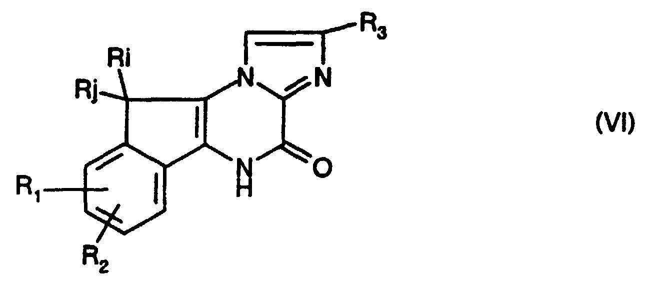 in which R₁, R₂ and R₃ have the same meanings as in claim 1, Ri denotes a -CH₂OTs radical in which Ts denotes a tosylate residue and Rj denotes an alkyl, -alk-Het or phenylalkyl radical in which the phenyl nucleus is optionally substituted by one or more substituents chosen from halogen atoms and alkyl, alkoxy, nitro, amino, hydroxyl, cyano, -alk-NH₂, -COOR₁₀ and -alk-COOR₁₀ radicals, alk, Het and R₁₀ having the same meanings as in claim 1, the product is isolated and is optionally converted into salt.

28. Process for the preparation of compounds of formula (I) according to claim 1 in which R denotes a C(R₄)R₅ radical in which R₅ denotes a -(2-6 C)alkOH radical, characterized in that (COCl)₂ is reacted with a corresponding compound of formula (I) in which R denotes a C(R₄)R₅ radical in which R₅ denotes an -alk-COOR₁₀ radical and R₁₀ denotes a hydrogen atom, followed by a reduction, the product is isolated and is optionally converted into salt.

29. Process for the preparation of compounds of formula (I) according to claim 1, in which R denotes a C(R₄)R₅ radical in which R₅ denotes a -(1 C)alkOH radical, characterized in that trimethylsilane chloride is reacted with a derivative of formula: 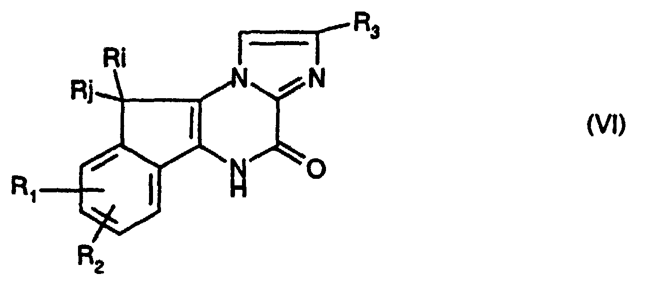 in which R₁, R₂ and R₃ have the same meanings as in claim 1, Ri denotes a hydrogen atom and Rj denotes an alkyl, -alk-Het or phenylalkyl radical in which the phenyl nucleus is optionally substituted by one or more substituents chosen from halogen atoms and alkyl, alkoxy, nitro, amino, hydroxyl, cyano, -alk-NH₂, -COOR₁₀ and -alk-COOR₁₀ radicals, alk, Het and R₁₀ having the same meanings as in claim 1, followed by trioxane, the product is isolated and is optionally converted into salt.

30. Process for the preparation of compounds of formula (I) according to claim 1, in which R denotes a C(R₄)R₅ radical in which R₅ denotes an -NH-CHO radical, characterized in that a derivative of formula: 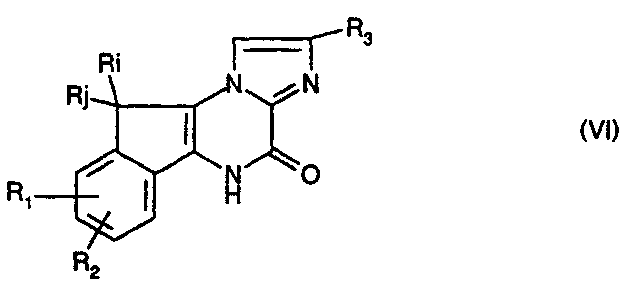 in which R₁, R₂ and R₃ have the same meanings as in claim 1, Ri denotes an alkyl, -alk-Het or phenylalkyl radical in which the phenyl nucleus is optionally substituted by one or more substituents chosen from halogen atoms and alkyl, alkoxy, nitro, amino, hydroxyl, cyano, -alk-NH₂, -COOR₁₀ and -alk-COOR₁₀ radicals and Rj denotes an amino radical, alk, Het and R₁₀ having the same meanings as in claim 1 is reacted with CH₃COOCHO, the product is isolated and is optionally converted into salt.

31. Process for the preparation of compounds of formula (I) according to claim 1, in which R denotes a C(R₄)R₅ radical in which R₅ denotes a radical which is -NH-COOR₁₇, -NH-CO-Het, -NH-CO-alk-COOR₁₀, -NH-CO-alk-NR₁₀R₁₈, -NH-CO-Ar in which Ar is optionally substituted, -NH-CO-alk-Ar in which Ar is optionally substituted, -NH-SO₂-R₂₄, -NH-CO-alk-Het, -NH-CO-alk or -NH-CO-cycloalkyl, characterized in that a corresponding compound of formula (I) in which R denotes a C(R₄)R₅ radical, R₅ denotes an -NR₈R₉ radical and R₈ and R₉ denote hydrogen atoms is reacted with a derivative Hal-Rk in which Hal denotes a halogen atom and Rk denotes a radical which is -COOR₁₇, -CO-Het, -CO-alk-COOR₁₀, -CO-alk-NR₁₀R₁₈, -CO-alk-Ar in which Ar is optionally substituted by one or more substituents chosen from halogen atoms and alkyl, alkoxy, nitro, amino, hydroxyl, cyano, -alk-NH₂, -COOR₁₀ and -alk-COOR₁₀ radicals, -SO₂-R₂₄, -CO-alk-Het, -CO-Ar in which Ar is optionally substituted by one or more substituents chosen from halogen atoms and alkyl, alkoxy, nitro, amino, hydroxyl, cyano, -alk-NH₂, -COOR₁₀ and -alk-COOR₁₀, -CO-alk or -CO-cycloalkyl radicals, R₇, R₁₀, R₁₇, R₁₈, R₂₄, Het, Ar and alk having the same meanings as in claim 1, the product is isolated and is optionally converted into salt.

32. Process for the preparation of compounds of formula (I) according to claim 1, in which R denotes a C(R₄)R₅ radical in which R₅ denotes a radical which is -NH-CO-Het, -NH-CO-alk-COOR₁₀, -NH-CO-alk-NR₁₀R₁₈, -NH-CO-Ar in which Ar is optionally substituted, -NH-CO-alk-Ar in which Ar is optionally substituted, -NH-CO-alk-Het, -NH-CO-alk or -NH-CO-cycloalkyl, characterized in that a corresponding compound of formula (I) in which R denotes a C(R₄)R₅ radical, R₅ denotes an -NR₈R₉ radical and R₈ and R₉ denote hydrogen atoms is reacted with a derivative HO-Rl in which Rl denotes a radical which is -CO-Het, -CO-alk-COOR₁₀, -CO-alk-NR₁₀R₁₈, -CO-alk-Ar in which Ar is optionally substituted by one or more substituents chosen from halogen atoms and alkyl, alkoxy, nitro, amino, hydroxyl, cyano, -alk-NH₂, -COOR₁₀ and -alk-COOR₁₀ radicals, -CO-alk-Het, -CO-Ar in which Ar is optionally substituted by one or more substituents chosen from halogen atoms and alkyl, alkoxy, nitro, amino, hydroxyl, cyano, -alk-NH₂, -COOR₁₀ and -alk-COOR₁₀, -CO-alk or -CO-cycloalkyl radicals, R₁₀, R₁₈, Het, Ar and alk having the same meanings as in claim 1, the product is isolated and is optionally converted into salt.

33. Process for the preparation of compounds of formula (I) according to claim 1, in which R denotes a C(R₄)R₅ radical in which R₅ denotes a 1-pyrrolyl radical optionally substituted by a -COOR₁₀ radical, characterized in that a derivative of formula: 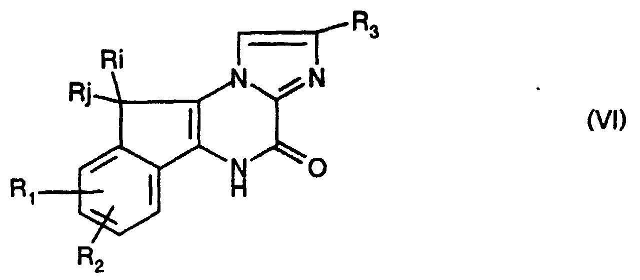 in which R₁, R₂ and R₃ have the same meanings as in claim 1, Ri denotes an alkyl, -alk-Het or phenylalkyl radical in which the phenyl nucleus is optionally substituted by one or more substituents chosen from halogen atoms and alkyl, alkoxy, nitro, amino, hydroxyl, cyano, -alk-NH₂, -COOR₁₀ and -alk-COOR₁₀ radicals, alk, Het and R₁₀ having the same meanings as in formula (I) and Rj denotes an amino radical, is reacted with a derivative of formula: 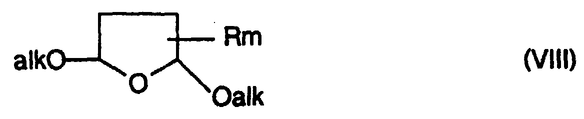 in which Rm denotes a hydrogen atom or a -COOR₁₀ radical and alk and R₁₀ have the same meanings as in claim 1, the product is isolated and is optionally converted into salt.

34. Process for the preparation of compounds of formula (I) according to claim 1, in which R denotes a C(R₄)R₅ radical in which R₅ denotes a radical which is -NH-CO-NH-alk-Ar in which Ar is optionally substituted, -NH-CO-NH-Het, -NH-CO-NH-alk-Het, -NH-CO-NH-Ar in which Ar is optionally substituted, -NH-CO-NH-Het or -NH-CO-NH₂, characterized in that a corresponding compound of formula (I) in which R denotes a C(R₄)R₅ radical, R₅ denotes an -NR₈R₉ radical and R₈ and R₉ denote hydrogen atoms is reacted with a derivative O=C=N-Rn in which Rn denotes a radical which is trimethylsilyl, alkyl, -Het, -alk-Ar in which Ar is optionally substituted by one or more substituents chosen from halogen atoms and alkyl, alkoxy, nitro, amino, hydroxyl, cyano, -alk-NH₂, -COOR₁₀ and -alk-COOR₁₀ radicals, -alk-Het or phenyl optionally substituted by one or more substituents chosen from halogen atoms and alkyl, alkoxy, nitro, amino, hydroxyl, cyano, -alk-NH₂, -COOR₁₀ and -alk-COOR₁₀ radicals in which R₁₀, alk, Ar and Het have the same meanings as in claim 1, the product is isolated and is optionally converted into salt.

35. Process for the preparation of compounds of formula (I) according to claim 1, in which R denotes a CH-R₆ radical in which R₆ denotes a hydroxyl radical, characterized in that a corresponding compound of formula (I) in which R denotes a C=R₇ radical and R₇ denotes an oxygen atom is reduced, the product is isolated and is optionally converted into salt.

36. Process for the preparation of compounds of formula (I) according to claim 1, in which R denotes a CH-R₆ radical in which R₆ denotes an alkyl (2-11 C) or phenylalkyl radical in which the phenyl nucleus is optionally substituted by one or more substituents chosen from halogen atoms and alkyl, alkoxy, nitro, amino, hydroxyl, cyano, -alk-NH₂, -COOR₁₀ and -alk-COOR₁₀ or -alk-Het radicals, characterized in that a derivative of formula: 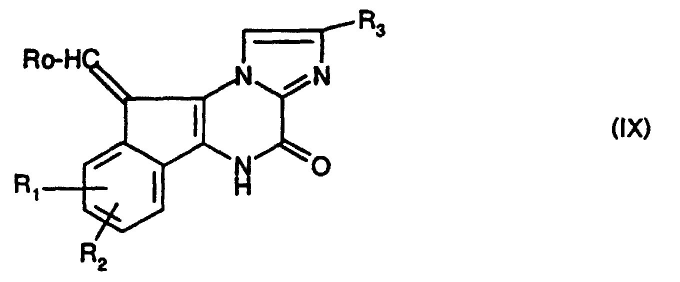 in which R₁, R₂ and R₃ have the same meanings as in claim 1, Ro denotes a radical which is alkyl as a straight or branched chain containing 1 to 10 carbon atoms, phenyl, phenyl(1-5 C)alkyl in which the phenyl nucleus is optionally substituted by one or more substituents chosen from halogen atoms and alkyl, alkoxy, nitro, amino, hydroxyl, cyano, -alk-NH₂, -COOR₁₀ and -alk-COOR₁₀ radicals, -Het or -(1-5 C)alk-Het in which alk, Het and R₁₀ have the same meanings as in claim 1 is hydrogenated, the product is isolated and is optionally converted into salt.

37. Process for the preparation of compounds of formula (I) according to claim 1, in which R denotes a CH-R₆ radical in which R₆ denotes an optionally substituted phenylalkyl or -alk-Het radical, characterized in that a corresponding compound of formula (I) in which R denotes a CH-R₆ radical and R₆ denotes a hydrogen atom is reacted with a halide of the formula Hal-Rp in which Rp denotes a phenylalkyl radical in which the phenyl nucleus is optionally substituted by one or more substituents chosen from halogen atoms and alkyl, alkoxy, nitro, amino, hydroxyl, cyano, -alk-NH₂, -COOR₁₀ and -alk-COOR₁₀ or -alk-Het radicals in which alk, Het and R₁₀ have the same meanings as in claim 1, the product is isolated and is optionally converted into salt.

38. Process for the preparation of compounds of formula (I) according to claim 1, in which R denotes a CH-R₆ radical in which R₆ denotes an alkyl (1 C) radical, characterized in that a corresponding compound of formula (I) in which R denotes a C=R₇ radical, R₇ denotes a CH-R₁₉ radical and R₁₉ denotes a hydroxyl or -NR₂₅R₂₆ radical is reduced, the product is isolated and is optionally converted into salt.

39. Process for the preparation of compounds of formula (I) according to claim 1, in which R denotes a CHR₆ radical and R₆ denotes a -(1 C)alk-OH radical, characterized in that a corresponding compound of formula (I) in which R denotes a C=R₇ radical, R₇ denotes a CH-R₁₉ radical and R₁₉ denotes a hydroxyl radical is reduced, the product is isolated and is optionally converted into salt.

40. Process for the preparation of compounds of formula (I) according to claim 1, in which R denotes a CHR₆ radical and R₆ denotes a -(2-6 C)alk-OH radical, characterized in that a derivative of formula: 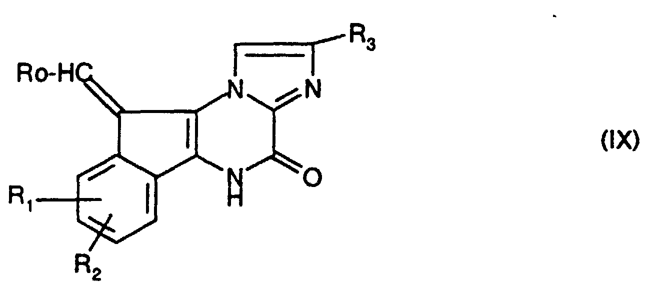 in which R₁, R₂ and R₃ have the same meanings as in claim 1 and Ro denotes a -(1-5 C)alk-O-CH₂-Ar radical, alk and Ar having the same meanings as in claim 1, is reduced, the product is isolated and is optionally converted into salt.

41. Process for the preparation of compounds of formula (I) according to claim 1, in which R denotes a CH-R₆ radical in which R₆ denotes an -NR₁₄R₁₅ radical and each of R₁₄ and R₁₅ denotes a hydrogen atom, characterized in that a corresponding compound of formula (I) in which R denotes a CH-R₆ radical in which R₆ denotes an -NR₁₄R₁₅ radical, R₁₄ denotes a hydrogen atom, R₁₅ denotes a -COR₂₂ radical and R₂₂ denotes an alkyl radical is hydrolysed, followed in the case of the compounds in which R₃ denotes an alkoxycarbonyl radical by an esterification of the corresponding acid, the product is isolated and is optionally converted into salt.

42. Process for the preparation of compounds of formula (I) according to claim 1, in which R denotes a CH-R₆ radical in which R₆ denotes an -NR₁₄R₁₅ radical, R₁₄ denotes a hydrogen atom, R₁₅ denotes a -COR₂₂ radical and R₂₂ denotes an alkyl radical, characterized in that a reducing agent, and then an acid anhydride (alkCO)₂O in which alk denotes an alkyl radical, are reacted with a corresponding compound of formula (I) in which R denotes a C=R₇ radical and R₇ denotes an NOH radical, the product is isolated and is optionally converted into salt.

43. Process for the preparation of compounds of formula (I) according to claim 1, in which R denotes a CH-R₆ radical and R₆ denotes an -NR₁₄R₁₅ or -alk-NR₁₄R₁₅ radical, in which each of R₁₄ and R₁₅, which are identical or different, denotes an alkyl radical or else R₁₄ denotes a hydrogen atom and R₁₅ denotes an alkyl, -COR₂₂ or -SO₂R₂₄ radical, R₂₂ denotes a radical which is alkyl, cycloalkyl, -COOalk, -alk-COOR₁₀, phenyl optionally substituted by one or more substituents chosen from halogen atoms and alkyl, alkoxy, nitro, amino, hydroxyl, -alk-NH₂, -COOR₁₀, cyano and -alk-COOR₁₀ radicals, phenylalkyl in which the phenyl nucleus is optionally substituted by one or more substituents chosen from halogen atoms and alkyl, alkoxy, nitro, amino, hydroxyl, -alk-NH₂, -COOR₁₀, cyano and -alk-COOR₁₀ radicals, -OR₁₇, -Het, -alk-Het, -alk-NR₁₀R₁₂ and R₂₄ denotes an alkyl or phenyl radical, characterized in that a corresponding compound of formula (I) in which each of R₁₄ and R₁₅ denotes a hydrogen atom is reacted with a halide of formula Hal-Rr in which Rr denotes an alkyl, -COR₂₂ or -SO₂R₂₄ radical, R₂₂ denotes a radical which is alkyl, cycloalkyl, -COOalk, -alk-COOR₁₀, phenyl optionally substituted by one or more substituents chosen from halogen atoms and alkyl, alkoxy, nitro, amino, hydroxyl, -alk-NH₂, -COOR₁₀, cyano and -alk-COOR₁₀ radicals, phenylalkyl in which the phenyl nucleus is optionally substituted by one or more substituents chosen from halogen atoms and alkyl, alkoxy, nitro, amino, hydroxyl, -alk-NH₂, -COOR₁₀, cyano and -alk-COOR₁₀ radicals, -OR₁₇, -Het or -alk-Het, -alk-NR₁₀R₁₂ and R₂₄ denotes an alkyl or phenyl radical, alk, Het, R₁₂, R₁₇ and R₁₀ having the same meanings as in claim 1, the product is isolated and is optionally converted into salt.

44. Process for the preparation of compounds of formula (I) according to claim 1 in which R denotes a CH-R₆ radical and R₆ denotes an -NR₁₄R₁₅ or -alk-NR₁₄R₁₅ radical in which R₁₄ denotes a hydrogen atom, R₁₅ denotes a -COR₂₂ or -CSR₂₃ radical, R₂₂ denotes a radical which is -NH-alk, -NH₂, -NH-Ar in which Ar is optionally substituted -NH-alk-Ar in which Ar is optionally substituted, -NH-alk-Het or -NH-Het and R₂₃ denotes an -NH-alk, -NH₂, -NH-Ar or -NH-Het radical, characterized in that a corresponding compound of formula (I) in which R denotes a CH-R₆ radical, R₆ denotes an -NR₁₄R₁₅ radical and each of R₁₄ and R₁₅ denotes a hydrogen atom is reacted with a derivative Rs-N=C=Rt in which Rs denotes a radical which is trimethylsilyl, alkyl, phenyl optionally substituted by one or more substituents chosen from halogen atoms and alkyl, alkoxy, nitro, amino, hydroxyl, cyano, -alk-NH₂, -COOR₁₀ and -alk-COOR₁₀ radicals, -alk-Ar in which Ar is optionally substituted by one or more substituents chosen from halogen atoms and alkyl, alkoxy, nitro, amino, hydroxyl, cyano, -alk-NH₂, -COOR₁₀ and -alk-COOR₁₀ radicals, -alk-Het or -Het in which Het, alk, R₁₀ and Ar have the same meanings as in claim 1 and Rt denotes an oxygen or sulphur atom, optionally followed by a hydrolysis, the product is isolated and is optionally converted into salt.

45. Process for the preparation of compounds of formula (I) according to claim 1, in which R denotes a CH-R₆ radical and R₆ denotes an -NR₁₄R₁₅ or -alk-NR₁₄R₁₅ radical in which R₁₄ denotes a hydrogen atom, R₁₅ denotes a -COR₂₂ radical and R₂₂ denotes a -(1 C)alk-NR₁₀R₁₂ radical in which R₁₀ and R₁₂ are hydrogen atoms, characterized in that a corresponding compound of formula (I) in which each of R₁₄ and R₁₅ denotes a hydrogen atom is reacted with an acid HOOC-CH₂-NH-Ru in which Ru denotes a protecting group for the amine functional group, followed by a hydrolysis, the product is isolated and is optionally converted into salt.

46. Process for the preparation of compounds of formula (I) according to claim 1, in which R denotes a CH-R₆ radical, R₆ denotes an -alk-NR₁₄R₁₅ radical and each of R₁₄ and R₁₅ denotes a hydrogen atom with the exception of those in which R₃ denotes a carboxamido radical, characterized in that bromine and sodium hydroxide are reacted with a corresponding compound of formula (I) in which R denotes a CH-R₆ radical, R₆ denotes an -alk-CONR₁₀R₂₁ radical and R₁₀ and R₂₁ denote hydrogen atoms, the product is isolated and is optionally converted into salt.

47. Process for the preparation of compounds of formula (I) according to claim 1, in which R denotes a CH-R₆ radical, R₆ denotes an -alk-NR₁₄R₁₅ radical, each of R₁₄ and R₁₅ denotes a hydrogen atom and R₃ denotes a carboxamido radical, characterized in that ammonia is reacted with a corresponding compound of formula (I) in which R denotes a CH-R₆ radical, R₆ denotes an -alk-NR₁₄R₁₅ radical, each of R₁₄ and R₁₅ denotes a hydrogen atom and R₃ denotes an alkoxycarbonyl radical, the product is isolated and is optionally converted into salt.

48. Process for the preparation of compounds of formual (I) according to claim 1, in which R denotes a CH-R₆ radical, R₆ denotes an -NR₁₄R₁₅ or -alk-NR₁₄R₁₅ radical in which R₁₄ denotes a hydrogen atom and R₁₅ denotes a -COR₂₂ radical, R₂₂ denotes a radical which is alkyl, cycloalkyl, -alk-COOR₁₀, optionally substituted phenyl, phenylalkyl in which the phenyl is optionally substituted, Het, -alk-Het or -alk-NR₁₀R₁₂, characterized in that a corresponding compound of formula (I) in which R denotes a CH-R₆ radical, R₆ denotes an -NR₁₄R₁₅ radical, R₁₄ denotes a hydrogen atom and R₁₅ denotes a hydrogen atom is reacted with an acid HOOC-R₂₂ in which R₂₂ denotes a radical which is alkyl, cycloalkyl, -Het, -alk-COOR₁₀, -alk-NR₁₀R₁₂, phenylalkyl in which the phenyl is optionally substituted by one or more substituents chosen from halogen atoms and alkyl, alkoxy, nitro, amino, hydroxyl, cyano, -alk-NH₂, -COOR₁₀ and -alk-COOR₁₀ radicals, -alk-Het, phenyl optionally substituted by one or more substituents chosen from halogen atoms and alkyl, alkoxy, nitro, amino, hydroxyl, cyano, -alk-NH₂, -COOR₁₀ and -alk-COOR₁₀, alk, Het, R₁₀ and R₁₂ having the same meanings as in claim 1 or the corresponding anhydride, the product is isolated and is optionally converted into salt.

49. Process for the preparation of compounds of formula (I) according to claim 1, in which R denotes a CH-R₆ radical and R₆ denotes an -alk-COOR₁₀ radical, characterized in that a corresponding derivative of formula: 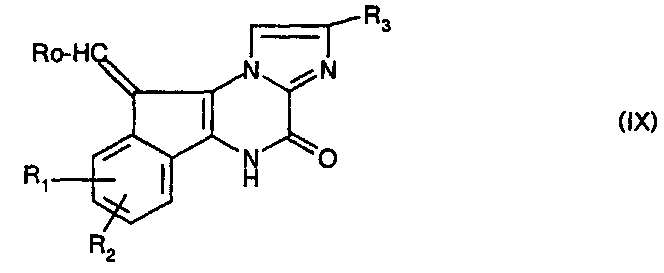 in which R₁, R₂ and R₃ have the same meanings as in claim 1 and Ro denotes a -(1-5 C)alk-COOR₁₀ or -COOR₁₀ radical, in which alk has the same meanings as in claim 1, R₁₀ denotes an alkyl or phenylalkyl radical is hydrogenated, followed optionally by a saponification of the ester thus obtained, the product is isolated and is optionally converted into salt.

50. Process for the preparation of compounds of formula (I) according to claim 1, in which R denotes a CH-R₆ radical and R₆ denotes an -alk-CO-NR₁₀R₂₁ radical, characterized in that a corresponding compound of formula (I) in which R denotes a CH-R₆ radical and R₆ denotes an -alk-COOR₁₀ radical, alk having the same meaning as in claim 1, and R₁₀ denotes a hydrogen atom or an alkyl radical is reacted with an amine HNR₁₀R₂₁ in which R₁₀ and R₂₁ have the same meanings as in claim 1, the product is isolated and is optionally converted into salt.

51. Process for the preparation of compounds of formula (I) according to claim 1, in which R denotes a CH-R₆ radical, R₆ denotes a -(C 1)alk-CO-NR₁₀R₂₁ radical and R₁₀ and R₂₁ denote hydrogen atoms, characterized in that a derivative of formula: 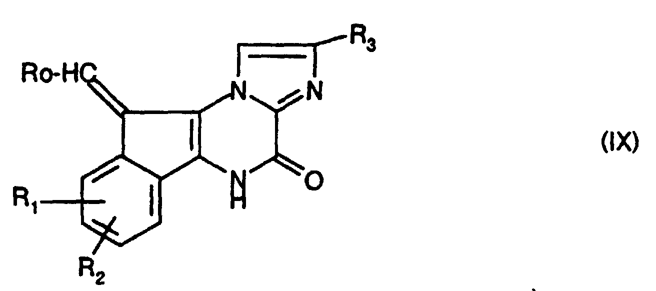 in which R₁, R₂ and R₃ have the same meanings as in formula (I) and Ro denotes a -CONH₂ radical is hydrogenated, the product is isolated and is optionally converted into salt.

52. Process for the preparation of compounds of formula (I) according to claim 1, in which R denotes a CH-R₆ radical and R₆ denotes an -R₁₆-COOR₁₀ radical, characterized in that a corresponding compound of formula (I) in which R denotes a CH-R₆ radical and R₆ denotes a hydrogen atom is reacted with a derivative of formula OHC-(0-5 C)alk-COOR₁₀ in which alk and R₁₀ have the same meanings as in claim 1, the product is isolated and is optionally converted into salt.

53. Process for the preparation of compounds of formula (I) according to claim 1, in which R denotes a CH-R₆ radical and R₆ denotes an -NH-CHO radical, characterized in that a corresponding compound of formula (I) in which R denotes a CH-R₆ radical, R₆ denotes an -NR₁₄R₁₅ radical and R₁₄ and R₁₅ denote hydrogen atoms is reacted with CH₃COOCHO, the product is isolated and is optionally converted into salt.

54. Process for the preparation of the compounds of formula (I) according to claim 1, in which R denotes a CH-R₆ radical and R₆ denotes a -CO-COOR₁₀ radical, characterized in that a corresponding compound of formula (I) in which R denotes a CH-R₆ radical, R₆ denotes a -R₁₆-COOR₁₀ radical and R₁₆ denotes a -CHOH- radical is oxidized, followed optionally by an esterification, the product is isolated and is optionally converted into salt.

55. Process for the preparation of compounds of formula (I) according to claim 1, in which R denotes a CH-R₆ radical and R₆ denotes a 1-pyrrolyl radical optionally substituted by a -COOR₁₀ radical, characterized in that a corresponding compound of formula (I) in which R denotes a CH-R₆ radical and R₆ denotes an -NR₁₄R₁₅ radical and R₁₄ and R₁₅ denote hydrogen atoms is reacted with a derivative of formula: 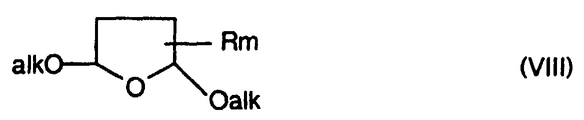 in which Rm denotes a hydrogen atom or a -COOR₁₀ radical, R₁₀ having the same meanings as in claim 1, the product is isolated and is optionally converted into salt.

56. Process for the preparation of compounds of formula (I) according to claim 1, in which R denotes a CH-R₆ radical and R₆ denotes a -COOalk radical, characterized in that an inorganic acid is reacted with a derivative of formula: 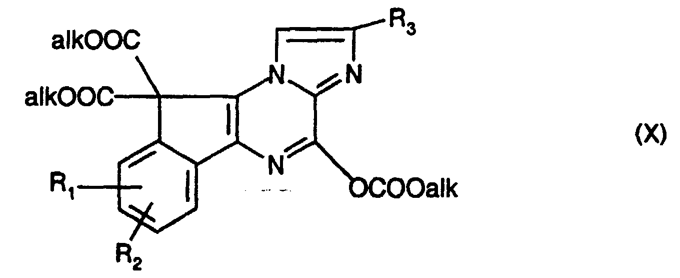 in which R₁, R₂ and R₃ have the same meanings as in claim 1 and alk denotes an alkyl radical, the product is isolated and is optionally converted into salt.

57. Process for the preparation of compounds of formula (I) according to claim 1, in which R₃ denotes a carboxyl radical, characterized in that a corresponding compound of formula (I) in which R₃ denotes an alkoxycarbonyl radical is hydrolysed, the product is isolated and is optionally converted into salt.

58. Process for the preparation of compounds of formula (I) according to claim 1, in which R₁ and/or R₂ denote an -NH-CO-NR₁₁R₁₂ radical in which R₁₁ denotes a hydrogen atom or a radical which is alkyl (1-9 C as a straight or branched chain), -alk-COOR₁₀, -alk-Het, -alk-NR₁₂R₁₀, phenylalkyl in which the phenyl nucleus is optionally substituted by one or more substituents chosen from halogen atoms and alkyl, alkoxy, nitro, amino, hydroxyl, -alk-NH₂, carboxyl, alkoxycarbonyl, cyano and -alk-COOR₁₀ radicals, phenyl optionally substituted by one or more substituents chosen from halogen atoms and alkyl, alkoxy, nitro, amino, hydroxyl, -alk-NH₂, carboxyl, alkoxycarbonyl, cyano and -alk-COOR₁₀ radicals or -Het and R₁₂ denotes a hydrogen atom, characterized in that a corresponding compound of formula (I) in which R₁ and/or R₂ denotes an amino radical is reacted with an isocyanate O=C=NR₁₁ in which R₁₁ has the same meanings as above or denotes a trimethylsilyl radical, the product is isolated and is optionally converted into salt.

59. Process for the preparation of compounds of formula (I) according to claim 1, in which R₁ and/or R₂ denote an amino radical, characterized in that a corresponding compound of formula (I) in which R₁ and/or R₂ denote a nitro radical is reduced, the product is isolated and is optionally converted into salt.

60. Process for the preparation of compounds of formula (I) according to claim 1, in which R denotes a CH-R₆ radical, R₆ denotes an -NR₁₄R₁₅ radical and R₁₄ and R₁₅ denote hydrogen atoms, characterized in that a corresponding compound of formula (I) in which R denotes a C=R₇ radical and R₇ denotes an NOH radical is reduced in the presence of a reducing agent, the product is isolated and is optionally converted into salt.

61. Process for the preparation of compounds of formula (I) according to claim 1, in which R denotes a C=R₇ radical, R₇ denotes a CH-R₁₉ radical, R₁₉ denotes a phenyl radical substituted by a -COOR₁₀ radical and R₁₀ denotes a hydrogen atom, characterized in that a corresponding compound of formula (I) in which R denotes a C=R₇ radical, R₇ denotes a CH-R₁₉ radical and R₁₉ denotes a phenyl radical substituted by a cyano radical is hydrolysed, the product is isolated and is optionally converted into salt.

62. Process for the preparation of compounds of formula (I) according to claim 1, in which R denotes a CH-R₆ radical and R₆ denotes a 2-oxo-2,5-dihydro-pyrrol-1-yl radical, characterized in that a corresponding compound of formula (I) in which R denotes a CH-R₆ radical, R₆ denotes an -NR₁₄R₁₅ radical and R₁₄ and R₁₅ denote hydrogen atoms is reacted with 2,5-dimethoxy-2,5-dihydrofuran, the product is isolated and is optionally converted into salt.

63. Compounds of formula (I) according to claim 1, in which R denotes a CH-R₆, C=R₇ or C(R₄)R₅ radical, R₁ denotes a hydrogen or halogen atom or an -NH-CO-NR₁₁R₁₂ radical in which R₁₁ is a hydrogen atom and R₁₂ is an alkyl radical, R₂ denotes a hydrogen atom, R₃ denotes a carboxyl, alkoxycarbonyl or carboxamido radical, R₄ denotes an alkyl radical, R₅ denotes an -alk-COOR₁₀ radical, R₆ denotes a hydrogen atom or a radical which is alk-Het, 2-oxo-2,5-dihydropyrrol-l-yl, 1-pyrrolyl substituted by -COOR₁₀ or -NR₁₄R₁₅, R₇ denotes a CH-R₁₉, NO-alk-COOR₁₀ or NOH radical, R₁₉ denotes a Het or phenyl radical substituted by -COOR₁₀, and either R₁₄ and R₁₅ are hydrogen atoms or R₁₄ is a hydrogen atom and R₁₅ is a -COR₂₂ radical in whi-ch R₂₂ is an -alk-COOR₁₀ or -NH-Ar radical and their salts.

64. Medications containing, as active principle, at least one compound of formula (I) according to one of claims 1, 2 and 63 or a salt of such a compound.
